# EUROPEAN PATENT SPECIFICATION

(11) **EP 0 249 613 B1**
(45) Date of publication and mention of the grant of the patent: **03.07.1996**
(21) Application number: 86907184.5
(22) Date of filing: 19.11.1986
(51) Int. Cl.: C07K 14/54, C12N 15/70, A61K 38/20

(54) **INTERLEUKIN-4 PROTEIN HAVING BCGF AND TCGF ACTIVITY ON HUMAN CELLS (HUMAN INTERLEUKIN-4)**
INTERLEUKIN-4 PROTEIN MIT BCGF- UND TCGF-AKTIVITÄT GEGENÜBER MENSCHLICHEN ZELLEN (MENSCHLICHES INTERLEUKIN-4)
INTERLEUKINE-4 PROTEINE AYANT UNE ACTIVITE BCGF AND TCGF VERS CELLULES HUMAINES (INTERLEUKINE-4 HUMAINE)

(30) Priority: 19.11.1985 US 799668; 19.11.1985 US 799669; 25.03.1986 US 843958; 03.07.1986 US 881553; 17.09.1986 US 908215
(43) Date of publication of application: 23.12.1987
(60) Divisional application: 95108150.4
(73) Proprietor: Schering Biotech Corporation, Palo Alto California 94304-1104 (US)
(72) Inventor: LEE, Frank, Palo Alto, CA 94301 (US); YOKOTA, Takashi, Palo Alto, CA 94303 (US); ARAI, Ken-ichi, Palo Alto, CA 94306 (US); MOSMANN, Timothy, Atherton, CA 94025 (US); RENNICK, Donna, Los Altos, CA 94022 (US); SMITH, Craig, Mountain View, CA 94041 (US)
(74) Representative: Ritter, Stephen David
(86) International application number: US8602464
(87) International publication number: WO8702990

(56) References cited:
- EP-A- 0 138 133
- WO-A-84/01504
- Proc. Natl. Acad. Sci. USA 83 (1986), 437 - 440 Science 231 (1986), 1118 - 1122 J. Exp. Med. 155 (1982), 914 - 923

## Description

The present invention relates generally to protein and mutein factors of the mammalian immune system and to nucleic acids coding therefor. More particularly, the invention relates to protein and mutein factors (along with their encoding nucleic acids) which exhibit both T cell growth factor activity and B cell growth factor activity.

Recombinant DNA technology refers generally to the technique of integrating genetic information from a donor source into vectors for subsequent processing, such as through introduction into a host, whereby the transferred genetic information is copied and/or expressed in the new environment. Commonly, the genetic information exists in the form of complementary DNA (cDNA) derived from messenger RNA (mRNA) coding for a desired protein product. The carrier is frequently a plasmid having the capacity to incorporate cDNA for later replication in a host and, in some cases, actually to control expression of the cDNA and thereby direct synthesis of the encoded product in the host.

For some time, it has been known that the mammalian immune response is based on a series of complex cellular interactions, called the "immune network." Recent research has provided new insights into the inner workings of this network. While it remains clear that much of the response does, in fact, revolve around the network-like interactions of lymphocytes, macrophages, granulocytes and other cells, immunologists now generally hold the opinion that soluble proteins (e.g., the so-called "lymphokines" or "monokines") play a critical role in controlling these cellular interactions. Thus, there is considerable interest in the isolation, characterization, and mechanisms of action of cell modulatory factors, an understanding of which should yield significant breakthroughs in the diagnosis and therapy of numerous disease states.

Lymphokines apparently mediate cellular activities in a variety of ways. They have been shown to support the proliferation, growth and differentiation of the pluripotential hematopoietic stem cells into the vast number of progenitors composing the diverse cellular lineages responsible for the immune response. These lineages often respond in a different manner when lymphokines are used in conjunction with other agents.

Cell lineages that are especially important to the immune response include two classes of lymphocytes: B-cells, which can produce and secrete immunoglobulins (proteins with the capability of recognizing and binding to foreign matter to effect its removal), and T-cells of various subsets that secrete lymphokines and induce or suppress the B-cells and some of the other cells (including other T-cells) making up the immune network.

Another important cell lineage is the mast cell (which has not been positively identified in all mammalian species)--a granule-containing connective tissue cell located proximal to capillaries throughout the body, with especially high concentrations in the lungs, skin, and gastrointestinal and genitourinary tracts. Mast cells play a central role in allergy-related disorders, particularly anaphylaxis, as follows: when selected antigens crosslink one class of immunoglobulins bound to receptors on the mast cell surface, the mast cell degranulates and releases the mediators (e.g., histamine, serotonin, heparin, prostaglandins, etc.) which cause allergic reactions, e.g., anaphylaxis.

Research to better understand (and thus potentially treat therapeutically) various immune disorders has been hampered by the general inability to maintain cells of the immune system in vitro. Immunologists have discovered that culturing these cells can be accomplished through the use of T-cell and other cell supernatants, which contain various growth factors, such as some of the lymphokines.

The detection, isolation and purification of factors such as lymphokines and other soluble mediators of immune reactions is extremely difficult, and is frequently complicated by the very nature of the supernatants they are typically located in, the divergencies and cross-overs of activities of the various components in the mixtures, the sensitivity (or lack thereof) of the assays utilized to ascertain the factors' properties, the frequent similarity in the range of molecular weights and other characteristics of the factors, and the very low concentration of the factors in their natural setting.

As more lymphokines become available, primarily through molecular cloning, interest has heightened in finding clinical applications for them. Because of physiological similarities to hormones (e.g., soluble factors, growth mediators, action via cell receptors), potential uses of lymphokines have been analogized to the current uses of hormones, e.g. Dexter, Nature, Vol. 321, pg. 198 (1986). One hope is that the levels of lymphokines in a patient can be manipulated directly or indirectly to bring about a beneficial immune response, e.g. suppression of inflammation, allergy, or tissue rejection, or stimulation or potentiation against infection or malignant growth. Other potential clinical uses of lymphokines include maintaining and expanding in vitro populations of certain immune system cells of one person for eventual reintroduction into the same or another person for a beneficial effect. For example, investigations are currently underway to determine whether populations of lymphokine-activated killer T cells of a patient can be expanded outside his or her body and then reinjected to bring about an enhanced antitumor response. Another potential clinical use of lymphokines, particularly colony stimulating factors, such as granulocyte-macrophage colony stimulating factor (GM-CSF), and factors which enhance their activities, is stimulating blood cell generation, for example in pre- or post-chemotherapy or radiation therapy against tumors, in treatment of myeloid hypoplasias, or in treatment of neutrophil deficiency syndromes: Dexter, Nature, Vol. 321, pg. 198 (1986). Such factors would also be useful in bone marrow transplant therapy, which is being used increasingly to treat aplastic anemia and certain leukemias.

There are two properties of lymphokines that have important consequences for such clinical applications: individual lymphokines are frequently pleiotropic; and the biological effects of one lymphokine can usually be modulated by at least one other lymphokine, either by inhibition or by potentiation. For example, tumor necrosis factor, which synergizes with gamma-interferon, stimulates interleukin-1 (IL-1) production and can activate the phagocytic activity of neutrophils. IL-1, a protein produced by activated macrophages, mediates a wide range of biological activities, including stimulation of thymocyte proliferation via induction of interleukin-2 (IL-2) release, stimulation of B-lymphocyte maturation and proliferation, fibroblast growth factor activity and induction of acute-phase protein synthesis by hepatocytes. IL-1 has also been reported to stimulate prostaglandin and collagenase release from synovial cells, and to be identical to endogenous pyrogen: Krampschmidt, J. Leuk. Biol., Vol. 36, pgs. 341-355 (1984).

Interleukin-2, formerly referred to as T-cell growth factor, is a lymphokine which is produced by lectin- or antigen-activated T cells. The reported biological activities of IL-2 include stimulation of the long-term in vitro growth of activated T-cell clones, enhancement of thymocyte mitogenesis, and induction of cytotoxic T-cell reactivity and plaque-forming cell responses in cultures of nude mouse-spleen cells. In addition, like interferons (IFNs), IL-2 has been shown to augment natural killer cell activity, suggesting a potential use in the treatment of neoplastic diseases: Henney et al., Nature, Vol, 291, pgs. 335-338 (1981). Some success has been reported in such therapy, e.g. Lotze and Rosenberg, "Treatment of Tumor Patients with Purified Human Interleukin-2," pgs. 711-719, in Sorg et al., Eds. Cellular and Molecular Biology of Lymphokines (Academic Press, Inc., New York, 1985); and Rosenberg and Lotze, "Cancer Immunotherapy Using Interleukin-2 and Interleukin-2-Activated Lymphocytes," Ann. Rev. Immunol., Vol. 4, pgs. 681-709 (1986). However, IL-2 toxicity has limited the dosages which can be delivered to cancer patients for taking advantage of these properties: Lotze and Rosenberg, pgs. 711-719; and Welte et al., pgs. 755-759, in Sorg et al. Eds. (cited above).

Metcalf, D., The Hematopoietic Colony Stimulating Factors, (Elsevier, Amsterdam, 1984), provides an overview of research concerning lymphokines and various growth factors involved in the mammalian immune response. Yung, Y.-P., et al., J. Immunol. Vol. 127 pg. 794 (1981), describe the partial purification of the protein of approximately 35 kd exhibiting mast cell growth factor (MCGF) activity and its separation from interleukin-2 (IL-2), also known as T-cell growth factor (TCGF). Nabel, G., et al., Nature, Vol. 291, pg. 332 (1981) report an MCGF exhibiting a molecular weight of about 45 kd and a pI of about 6.0. Clark-Lewis, I. and Schrader, J., J. Immunol., Vol. 127, pg. 1941 (1981), describe a protein having mast cell like growth factor activity that exhibits a molecular weight of about 29 kd in phosphate-buffered saline and about 23 kd in 6M guanidine hydrochloride, with a pI of about 4-8 but of about 6-8 after neuraminidase treatment. Murine IL-2 and interleukin-3 (IL-3) have been partially characterized biochemically by Gillis, S., et al., J. Immunol., Vol. 124, pgs. 1954-1962 (1980), and Ihle, J., et al., J. Immunol., Vol. 129, pgs. 2431-2436 (1982), respectively, with IL-2 having an apparent molecular weight (probably as a dimer) of about 30-35 kd and IL-3 having a molecular weight of about 28 kd. Human IL-2 apparently has a molecular weight of about 15 kd and is described by Gillis, S., et al., Immu. Rev., Vol. 63, pgs. 167-209 (1982). Comparison between IL-3 and MCGF activities of T-cell supernatants have been reported by Yung Y. and Moore, M., Contemp. Top. Mol. Immunol., Vol. 10, pgs. 147-179 (1985), and Rennick, D., et al., J. Immunol., Vol. 134, pgs. 910-919 (1985).

An extensive literature exists concerning the regulation of B-cell growth and differentiation by soluble factors: e.g. for reviews see Howard and Paul, Ann. Rev. Immunol., Vol. 1, pgs. 307-333 (1983); Howard et al., Immunol. Rev., 1984, No. 78, pgs. 185-210; Kishimoto et al., Immunol. Rev., 1984, No. 78 pgs. 97-118; and Kishimoto, Ann. Rev. Immunol., Vol. 3, pgs. 133-157 (1985). Some confusion has existed over the nomenclature used for labeling the various factors because of differences in source materials, difficulties in purification, and differences in the assays used to define their biological activities. Consensus in regard to nomenclature apparently has been reached in some cases: Paul, Immunology Today, Vol. 4, pg. 322 (1983); and Paul, Molecular Immunol., Vol. 21, pg. 343 (1984). B-cell growth factor (BCGF) activity is characterized by a capacity to cause DNA synthesis in B cells co-stimulated by exposure to anti-IgM, or like antigens. It is believed that interleukin-1 (IL-1) is also required for BCGF activity to be manifested, at least when the assay is conducted with low densities of B cells. Alternative assays for human BCGF have been described: e.g. Maizel et al, Proc. Natl. Acad. Sci., Vol. 80, pgs. 5047-5051 (1983) (support of long-term growth of human B cells in culture). The activity associated with the former assay has also been labelled B cell stimulatory factor-1 (BSF-1) activity and BCGF I, to distinguish it from similar and/or related activities. In particular, an activity designated BCGF II has been described. It is characterized by a capacity to cause DNA synthesis in mitogen-stimulated B cells or in transformed B cell lines. Mitogens associated with BCGF II activity include dextran sulfate, lipopolysaccharide, and Staphylococcus extracts. BCGF I registers no response in these assays. In humans it is believed that BCGF II is a molecule having a molecular weight of about 50 kilodaltons (kD), and that it acts synergistically with BCGF I (i.e. BSF-1) in promoting B cell proliferation in an immune response: Yoshizaka et al., J. Immunol., Vol. 130, pgs. 1241-1246 (1983). Howard et al., J. Exp. Med., Vol. 155, pgs. 914-923 (1982) were the first to show the existence of a murine BCGF (later to be called variously BCGF I, BSF-1, or IgG₁ induction factor) distinct from interleukin-2. Similar observations were reported almost simultaneously for a human system by Yoshizaki et al., J. Immunol., Vol. 128, pgs. 1296-1301 (1982), and later by Okada et al., J. Exp. Med., Vol. 157, pgs. 583-590 (1983).

Biochemical and biological characterization of molecules exhibiting BCGF, or BSF-1, activity has progressed steadily since these initial discoveries. Maizel et al., Proc. Natl. Acad. Sci., Vol. 79, pgs. 5998-6002 (1982), have reported a trypsin-sensitive human BCGF having a molecular weight of 12-13 kD and an isoelectric point (pI) of about 6.3-6.6. Farrar et al., J. Immunol., vol. 131, pgs. 1838-1842 (1983) reported partial purification of a heterogeneous murine BCGF having molecular weights of 11 and 15 kD by SDS-PAGE and pIs of 6.4-8.7. Ohara and Paul, in Nature, Vol. 315, pgs. 333-336 (1985) describe a monoclonal antibody specific for murine BSF-1, and give molecular weights for BSF-1 of 14 kD and 19-20 kD with pI of 6.7. Butler et al., J. Immunol., Vol. 133, pgs. 251-255 (1984), report a human BCGF having a molecular weight of 18-20 kD and a pI of 6.3-6.6. Rubin et al., Proc. Natl. Acad. Sci., vol. 82, pgs. 2935-2939 (1985), report that pre-incubation of resting B cells with BSF-1 prior to exposure to anti-IgM antibodies increases cell volume, and later speeds entry to S phase upon exposure to anti-IgM antibodies. Vitetta et al, J. Exp. Med., Vol. 162, pgs. 1726-1731 (1985), describe partial purification of murine BSF-1 by reverse phase HPLC of serum-free supernatants of EL-4 cells. SDS-PAGE indicated a protein of about 20-22kD. Ohara et al., J. Immunol., Vol. 135, pgs. 2518-2523 (1985) also report partial purification of murine BSF-1 by a similar procedure, and report the factor to be a protein of about 13-21.7 kD. Sideras et al., in Eur. J. Immunol., Vol. 15, pgs. 586-593, and 593-598 (1985), report partial purification of a murine IgG₁-inducing factor, that is a BSF-1, and report the factor to be a protein of about 20 kD having pIs of 7.2-7.4 and 6.2-6.4; and Smith and Rennick, in Proc. Natl. Acad. Sci., Vol. 83, pgs. 1857-1861 (1986), report the separation of a factor from IL-2 and IL-3 which exhibits T cell growth factor activity and mast cell growth factor activity. Later, Noma et al., Nature, Vol. 319, pgs. 640-646 (1986), cloned and sequenced a nucleic acid coding for the Sideras et al. factor, and Lee et al., Proc. Natl. Acad. Sci., Vol. 83, pgs. 2061-2065 (1986) cloned and sequenced a nucleic acid coding for the Smith and Rennick factor. More recently, Grabstein et al., J. Exp. Med., vol. 163, pgs. 1405-1414 (1986), report purifying and sequencing murine BSF-1.

Milanese, et al, in Science, Vol. 231, pgs. 1118-1122 (1986), report a lymphokine unrelated to BSF-1 which they provisionally designate IL-4A. Their IL-4A is a 10-12 kD protein secreted from helper T cells after cross-linking of T3-Ti receptors. It stimulates resting lymphocytes via interaction with T11 receptors and subsequent induction of interleukin-2 (IL-2) receptors.

Sanderson et al., in Proc. Natl. Acad. Sci., Vol. 83, pgs. 437-440 (1986), proposed that the name interleukin 4 be given to eosinophil differentiation factor based on evidence that it is apparently the same as B cell growth factor II.

Other publications describing growth factors include: EP-A-0 138 133 entitled "cDNA Clones Coding for Polypeptides Exhibiting Multi-lineage Cellular Growth Factor Activity and/or Mast Cell Growth Factor Activity"; WO 84/01504 entitled "Lymphocyte Growth Factor" and an article entitled "T Cell-Derived B Cell Growth Factor(s) Can Induce Stimulation of Both Resting and Activated B Cells", by Thompson, C.B., et al in The Journal of Immunology, Vol. 134, No. 1, January 1985, pages 369-374.

From the foregoing it is evident that the discovery and development of new lymphokines could contribute to the development of therapies for a wide range of degenerative conditions which directly or indirectly involve the immune system and/or hematopoietic cells. In particular, the discovery and development of lymphokines which enhance or potentiate the beneficial activities of known lymphokines would be highly advantageous. For example, the dose-limiting toxicity of IL-2 in tumor therapy could be reduced by the availability of a lymphokine or cofactor with potentiating effects; or the efficacy of bone marrow transplants could be increased by the availability of factors which potentiate the activities of the colony stimulating factors.

The present invention is directed to mammalian interleukin-4 (IL-4). It includes nucleic acids coding for polypeptides exhibiting IL-4 activity, as well as the polypeptides themselves and methods for their production. The nucleic acids of the invention are defined (1) by their homology to cloned complementary DNA (cDNA) sequences disclosed herein, and (2) by functional assays for IL-4 activity applied to the polypeptides encoded by the nucleic acids. As used herein, the term "IL-4 activity" in reference to a protein or a polypeptide means that the protein or polypeptide exhibits both B-cell growth factor (BCGF) activity and T cell growth factor (TCGF) activity. For a given mammal, IL-4 activity is determined by species-specific TCGF and BCGF assays. As explained more fully below, specific embodiments of IL-4 can be further characterized by additional assays. For example, some forms of murine IL-4 exhibit mast cell growth factor (MCGF) activity; some forms of both human and murine IL-4 potentiate the TCGF activity of IL-2; some forms of both murine and human IL-4 potentiate GM-CSF stimulated proliferation in certain cell types; some forms of both human and murine IL-4 can induce Fc-epsilon receptor expression on B cells; and some forms of both human and murine IL-4 can induce the expression of major histocompatibility complex (MHC) antigens on B cells: the class II DR antigen on human B cells, and the Ia antigen on mouse B cells.

The invention is based in part on the discovery and cloning of cDNAs which are capable of expressing proteins having IL-4 activity. cDNA clones of the invention include human cDNA inserts of plasmid vectors "clone 46" (also referred to herein as pcD-2F1-13 or pcD-46) and "clone 125" (also referred to herein as pcD-125); and mouse cDNA insert of plasmid vector pcD-2A-E3. The three vectors are deposited with the American Type Culture Collection (ATCC), Rockville, MD, under ATCC accession numbers 53337, 67029, and 53330, respectively.

The invention includes nucleic acids having nucleotide sequences which are effectively homologous to the cDNA clones of the invention and which express IL-4 activity. Nucleic acids and proteins of the invention can be derived from the above-mentioned cDNas by standard techniques for mutating nucleic acid sequences. They can be prepared de novo from immune system-derived cell lines, such as T cell hybridomas, which contain or can be induced to contain messenger RNA (mRNA) sequences coding for IL-4; and they can be obtained by probing DNA or RNA extracts or libraries with probes derived from the cDNA clones of the invention.

The term "effectively homologous" as used herein means that the nucleotide sequence is capable of being detected by a hybridization probe derived from a cDNA clone of the invention. The exact numerical measure of homology necessary to detect nucleic acids coding for IL-4 activity depends on several factors including (1) the homology of the probe to non-IL-4 coding sequences associated with the target nucleic acids, (2) the stringency of the hybridization conditions, (3) whether single stranded or double stranded probes are employed, (4) whether RNA or DNA probes are employed, (5) the measures taken to reduce nonspecific binding of the probe, (6) the nature of the label used to detect the probe, (7) the fraction of guanidine and cytosine bases in the probe, (8) the distribution of mismatches between probe and target, (9) the size of the probe, and the like.

Preferably, an effectively homologous probe derived from the cDNA of the invention is at least fifty percent (50%) homologous to the sequence to be isolated. More preferably, the effectively homologous probe is at least seventy-five to eighty percent (75-80%) homologous to the sequence to be isolated. Most preferably, the effectively homologous probe is at least ninety percent (90%) homologous to the sequence to be isolated.

Homology as the term is used herein is a measure of similarity between two nucleotide (or amino acid) sequences. Homology is expressed as the fraction or percentage of matching bases (or amino acids) after two sequences (possibly of unequal length) have been aligned. The term alignment is used in the sense defined by Sankoff and Kruskal in chapter one of Time Warps, String Edits, and Macromolecules: The Theory and Practice of Sequence Comparison (Addison-Wesley, Reading, MA, 1983). Roughly, two sequences are aligned by maximizing the number of matching bases (or amino acids) between the two sequences with the insertion of a minimal number of "blank" or "null" bases into either sequence to bring about the maximum overlap. Given two sequences, algorithms are available for computing their homology: e.g. Needleham and Wunsch, J. Mol. Biol., Vol. 48, pgs. 443-453 (1970); and Sankoff and Kruskal (cited above) pgs. 23-29. Also, commercial services are available for performing such comparisons, e.g. Intelligenetics, Inc. (Palo Alto, CA).

The present invention also relates to the natural mammalian growth factors (polypeptides) exhibiting a broad activity spectrum for a variety of cells integral to the immune response. Methods of producing such factors and their use for in vivo treatment of mammals are provided. Such polypeptide compositions include a mammalian factor in substantially pure form capable of exhibiting B-cell, T-cell and mast cell stimulatory activity.

These factors, which were originally isolated from the supernatant fluid of various T cells, include natural polypeptides exhibiting, under non-reducing conditions, a molecular weight of about 20 kd or 15 kd, or active fragments of such polypeptides. The factors may be used alone or in conjunction with other compounds for various diagnostic and therapeutic purposes, including the production of specific antibodies.

A preferred embodiment of the invention is the set of glycosylated or unglycosylated human IL-4 proteins and muteins defined by the following formula: 
X(His) - X(Lys) - X(Cys) - X(Asp) - X(Ile) - X(Thr) -
X(Leu) - X(Gln) - X(Glu) - X(Ile) - X(Ile) - X(Lys) -
X(Thr) - X(Leu) - X(Asn) - X(Ser) - X(Leu) - X(Thr) -
X(Glu) - X(Gln) - X(Lys) - X(Thr) - X(Leu) - X(Cys) -
X(Thr) - X(Glu) - X(Leu) - X(Thr) - X(Val) - X(Thr) -
X(Asp) - X(Ile) - X(Phe) - X(Ala) - X(Ala) - X(Ser) -
X(Lys) - X(Asn) - X(Thr) - X(Thr) - X(Glu) - X(Lys) -
X(Glu) - X(Thr) - X(Phe) - X(Cys) - X(Arg) - X(Ala) -
X(Ala) - X(Thr) - X(Val) - X(Leu) - X(Arg) - X(Gln) -
X(Phe) - X(Tyr) - X(Ser) - X(His) - X(His) - X(Glu) -
X(Lys) - X(Asp) - X(Thr) - X(Arg) - X(Cys) - X(Leu) -
X(Gly) - X(Ala) - X(Thr) - X(Ala) - X(Gln) - X(Gln) -
X(Phe) - X(His) - X(Arg) - X(His) - X(Lys) - X(Gln) -
X(Leu) - X(Ile) - X(Arg) - X(Phe) - X(Leu) - X(Lys) -
X(Arg) - X(Leu) - X(Asp) - X(Arg) - X(Asn) - X(Leu) -
X(Trp) - X(Gly) - X(Leu) - X(Ala) - X(Gly) - X(Leu) -
X(Asn) - X(Ser) - X(Cys) - X(Pro) - X(Val) - X(Lys) -
X(Glu) - X(Ala) - X(Asn) - X(Gln) - X(Ser) - X(Thr) -
X(Leu) - X(Glu) - X(Asn) - X(Phe) - X(Leu) - X(Glu) -
X(Arg) - X(Leu) - X(Lys) - X(Thr) - X(Ile) - X(Met) -
X(Arg) - X(Glu) - X(Lys) - X(Tyr) - X(Ser) - X(Lys) -
X(Cys) - X(Ser) - X(Ser)

Formula I
 wherein the term X(Xaa) represents the group of synonymous amino acids to the amino acid Xaa. Synonymous amino acids within a group have sufficiently similar physicochemical properties for substitution between members of the group to preserve the biological function of the molecule: Grantham, Science, vol. 185, pgs. 862-864 (1974). It is clear that insertions and deletions of amino acids may also be made in the above-defined sequence without altering biological function, particularly if the insertions or deletions only involve a few amino acids, e.g. under ten, and do not remove or displace amino acids which are critical to a functional conformation, e.g. cysteine residues, Anfinsen, "Principles That Govern The Folding of Protein Chains", Science, Vol. 181, pgs. 223-230 (1973). Proteins and muteins produced by such deletions and/or insertions come within the purview of the present invention. Whenever amino acid residues of the protein of Formula I are referred to herein by number, such number or numbers are in reference to the N-terminus of the protein.

Preferably the synonymous amino acid groups are those defined in Table I. More preferably, the synonymous amino acid groups are those listed before the second slash in each line in Tahle I; and most preferably the synonymous amino acid groups are those listed before the first slash in each line in Table II.

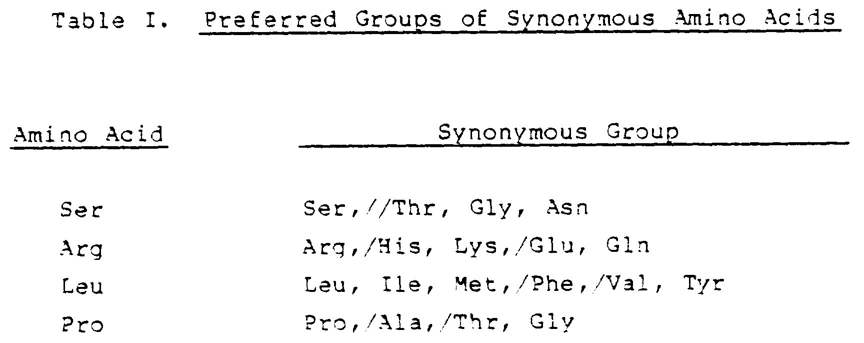

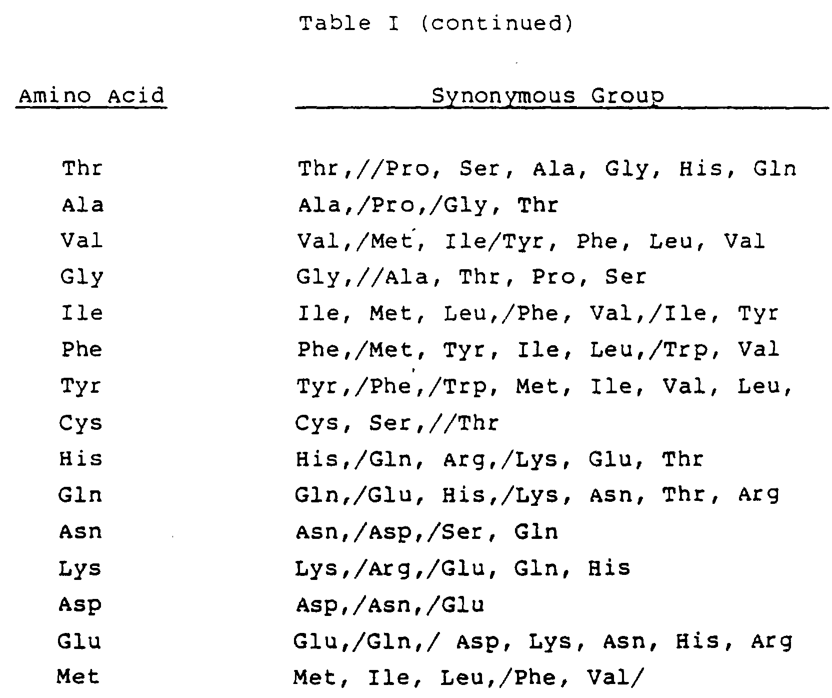

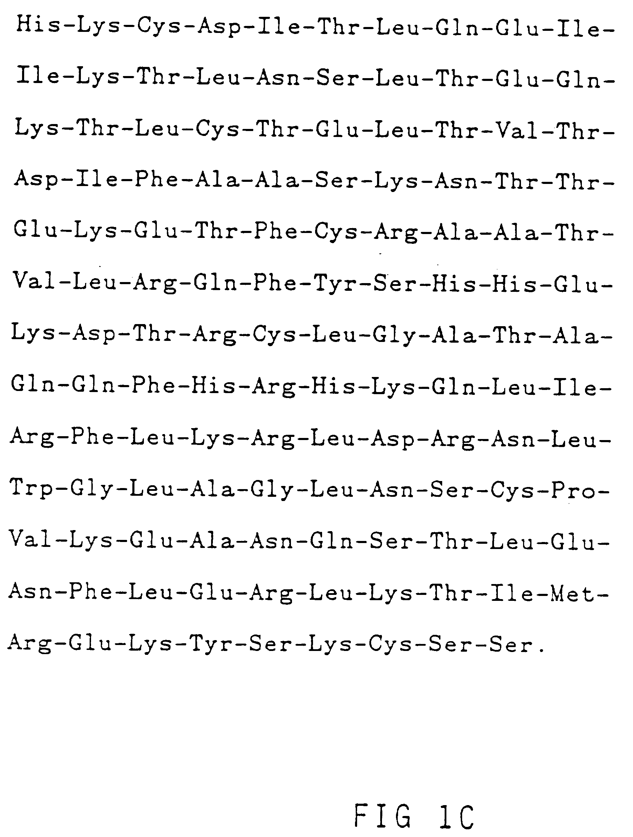

The invention includes the polypeptides of Formula I with amino acid substitutions (between an amino acid of the native human IL-4 and a synonymous amino acid) at a single position or at multiple positions. The term "N-fold substituted" is used to describe a subset of polypeptides defined by Formula I wherein the native amino acids have been substituted by synonymous amino acids at no more than N positions. Thus, for example, the group of 1-fold substituted polypeptides of Formula I consists of 559 polypeptides for the preferred groups of synonymous amino acids, 189 for the more preferred groups of synonymous amino acids, and 56 for the most preferred groups of synonymous amino acids. These numbers were arrived at by summing the number of amino acids of each kind in the native chain times the size of the synonymous amino acid group for that amino acid. Preferably the group of human IL-4 polypeptides consists of the 10-fold substituted polypeptides of Formula I; more preferably it consists of 3-fold substituted polypeptides of Formula I; and most preferably it consists of 1-fold substituted polypeptides of Formula I, which in particular includes the native human IL-4 polypeptide whose sequence is illustrated in Figure 1C of the drawings.

Likewise, the term "N-fold inserted" in reference to the peptides of Formula I is used to describe a set of polypeptides wherein from 1 to N amino acids have been inserted into the sequence defined by Formula I. Preferably, the inserted amino acids are selected from the preferred groups of synonymous amino acids (Table I) of the amino acids flanking the insertion; more preferably they are selected from the more preferred groups of synonymous amino acids (Table II) of the amino acids flanking the insertion, and most preferably they are selected from the most preferred groups of synonymous amino acids (Table III) of the amino acids flanking the insertion. Thus, for example, one subgroup of the group of 1-fold inserted peptides comprises an amino acid inserted between the N-terminal X(His) and the adjacent X(Gly). The insertions defining the members of this subgroup are preferably selected from the group consisting of Pro, Ala, Gly, Thr, Ser, Gln, Glu, Arg, His, and Lys; more preferably they are selected from the group consisting of Gly, His, Gln and Arg, and most preferably they are selected from the group consisting of His and Gly. Insertions can be made between any adjacent amino acids of Formula I. Since there are 128 possible insertion locations, and since multiple insertions can be made at the same location, a 2-fold inserted peptide of Formula I gives rise to 16,384 subgroups of peptides, and the size of each subgroup depends on the sizes of the synonymous amino acid groups of the amino acids flanking the insertions.

The term "N-fold deleted" in reference to the polypeptides of Formula I is used to describe a set of peptides having from 1 to N amino acids deleted from the sequence defined by Formula I. Thus, the set of 1-fold deleted polypeptides of Formula I consists of 129 subgroups of polypeptides each 128 amino acids in length (128-mers). Each of the subgroups in turn consists of all the 128-mers defined by the preferred, more preferred, and most preferred synonymous amino acid groups.

The above preferred embodiment of the invention further includes nucleotide sequences effectively homologous to or capable of encoding the polypeptides of Formula I for the preferred, more preferred, and most preferred groups of synonymous amino acids. More preferably said nucleotide sequences are capable of encoding the polypeptides of Formula I for the preferred, more preferred, and most preferred groups of synonymous amino acids.

In particular, the invention includes native human IL-4, the amino acid sequence of which is illustrated in Figure 1C, and all nucleotide sequences capable of encoding it.

Throughout, standard abbreviations are used to designate amino acids, nucleotides, restriction endonucleases, and the like: e.g. Cohn, "Nomenclature and Symbolism of α-Amino Acids, "Methods in Enzymology, Vol. 106, pgs. 3-17 (1984); Wood et al. Biochemistry: A Problems Approach, 2nd ed. (Benjamin, Menlo Park, 1981); and Roberts, "Directory of Restriction Endonucleases", Methods in Enzymology, Vol. 68, pgs. 27-40 (1979).

The present invention is addressed to problems associated with the application of immunoregulatory agents to treat medical and/or veterinary disorders. In particular, it provides compounds which have beneficial effects when used alone or which can act in concert with other lymphokines and immune-system mediators to produce beneficial effects.

Preferred embodiments of the invention will now be described, partly in conjunction with the accompanying drawings (Figs. 1 to 19).

Figure 1A illustrates the nucleotide sequence and deduced amino acid sequence of the insert of vector pcD-2A-E3, which expresses murine IL-4.

Figure 1B illustrates the nucleotide sequence and deduced amino acid sequence of the insert of vector pcD-125, which expresses human IL-4.

Figure 1C illustrates the amino acid sequence of purified native human IL-4 expressed and secreted by COS 7 monkey cells transfected with pcD-125.

Figure 2A is a map of vector pcD-2A-E3, the insert of which codes murine IL-4.

Figure 2B is a restriction endonuclease cleavage map of the insert of vector pcD-2A-E3.

Figure 2C is a map of vector pcD-46, the insert of which codes human IL-4.

Figure 2D is a restriction endonuclease cleavage map of the insert of vector pcD-46.

Figure 3A illustrates relative TCGF activities (over a range of dilutions) of various culture supernatants including one (curve 1) from pcD-2a-E3-transfected COS 7 cells.

Figure 3B illustrates relative MCGF activities (over a range of dilutions) of various culture supernatants including one (curve 1) from pcD-2A-E3-transfected COS 7 cells.

Figure 3C illustrates the relative degrees of Ia induction produced by the indicated amounts of supernatant from pcD-2A-E3-transfected COS 7 cells (curve 1), Cl.Ly1⁺2⁻/9 cells (curve 2), and mock transfected COS 7 cells (curve 3) .

Figure 3D graphically illustrates the extent of IgE and IgG₁ induction by supernatants from pcD-2A-E3-transfected COS 7 cells and various controls in T cell-depleted mouse spleen cells.

Figure 4A illustrates the TCGF activities of several pcD-125 transfection supernatants and controls as measured by a colorimetric proliferation assay on the factor-dependent human helper T cell line, JL-EBV.

Figure 4B illustrates the TCGF activities of a pcD-125 transfection supernatant and controls as measured by a colorimetric proliferation assay on PHA-stimulated peripheral blood lymphocytes.

Figure 4C illustrates the TCGF activities of a pcD-125 transfection supernatant and controls as measured by tritiated thymidine incorporation by PHA-stimulated peripheral blood lymphocytes.

Figure 5A is a histogram of cell frequency versus fluorescence intensity for a control population of stimulated human tonsilar B cells whose Fc-epsilon receptors have been fluorescently labeled.

Figure 5B is a histogram of cell frequency versus fluorescence intensity for a population of stimulated human tonsilar B cells which had been exposed to medium consisting of 0.1% supernatant from pcD-125-transfected COS 7 cells and whose Fc-epsilon receptors have been fluorescently labeled.

Figure 5C is a histogram of cell frequency versus fluorescence intensity for a population of stimulated human tonsilar B cells which had been exposed to medium consisting of 1% supernatant from pcD-125-transfected COS 7 cells and whose Fc-epsilon receptors have been fluorescently labeled.

Figure 5D is a histogram of cell frequency versus fluorescence intensity for a population of stimulated human tonsilar B cells which had been exposed to medium consisting of 10% supernatant from pcD-125-transfected COS 7 cells and whose Fc-epsilon receptors have been fluorescently labeled.

Figure 6a illustrates the nucleotide sequence of a synthetic human IL-4 gene useful for expressing native or mutant IL-4s in E. coli.

Figure 6B is a restriction endonuclease cleavage map of a synthetic human IL-4 gene inserted in plasmid pUC18.

Figures 7A-7F respectively illustrate the double stranded DNA fragments 1A/B through 6A/B used to construct a synthetic human IL-4 gene.

Figure 8 illustrates nucleotide sequences adjacent to the initiator ATG codon in the E. coli expression vector TAC-RBS. The sequences commence at an EcoRI restriction site and end with a HindIII site. The ribosome binding sequence (RBS) showing complementarity to the 3' end of 16S ribosomal RNA is underlined, and the ATG initiator codon is underlined.

Figure 9 illustrates histograms of cell frequency versus fluorescence intensity for populations of cells derived from a patient with bare lymphocyte syndrome. The cells were stained with fluorescently-labeled anti-DR monoclonal antibodies.

Figure 10 illustrates the 215 nm absorption profile in the final human IL-4 purification step, which consisted of reversed-phase HPLC on a C-4 column.

Figure 11 is a construction map of plasmid pEBV-178 containing human IL-4 cDNA.

Figure 12 is a construction map of plasmid TRPC11.

Figure 13A is a construction map of plasmid pMF-alpha8.

Figure 13B illustrates the TCGF activities of several transfection supernatants from yeast cultures expressing native human IL-4 and various muteins thereof.

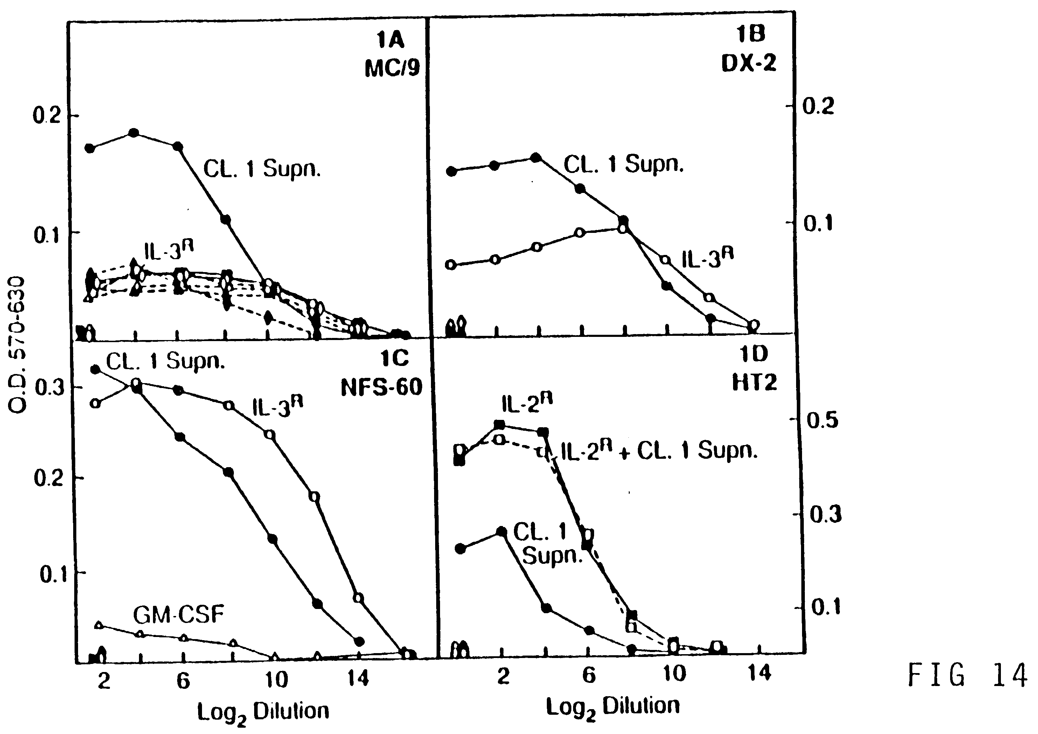

Figure 14 depicts growth curves of factor-dependent cell lines; MC/9 mast cells (1A), DX-2 mast cells (1B), NFS-60 cells (1C) and HT2 T cells (1D). 5 x 10³ cells were cultured with varying concentrations of C1.1 supernatant (closed circles), IL-2^{R} (closed squares), IL-3^{R} (open circles), GM-CSF^{R} (open triangles), IFN-γ^{R} (closed triangles), or P388D1 supernatant (open diamonds). MC/9 cells (1A) were also cultured with varying concentrations of purified IL-3 (closed circles) or IL-3^{R} mixed with 200 units of IL-2^{R} (closed squares), GM-CSF^{R} (open triangles), IFN-γ^{R} (closed triangles), or P388D1 supernatant (open diamonds). Growth factor activity was measured after 24 hours by colorimetric assay. The absorbance at 570 nm (reference 630 nm) was measured on a Dynatek Micro Elisa reader.

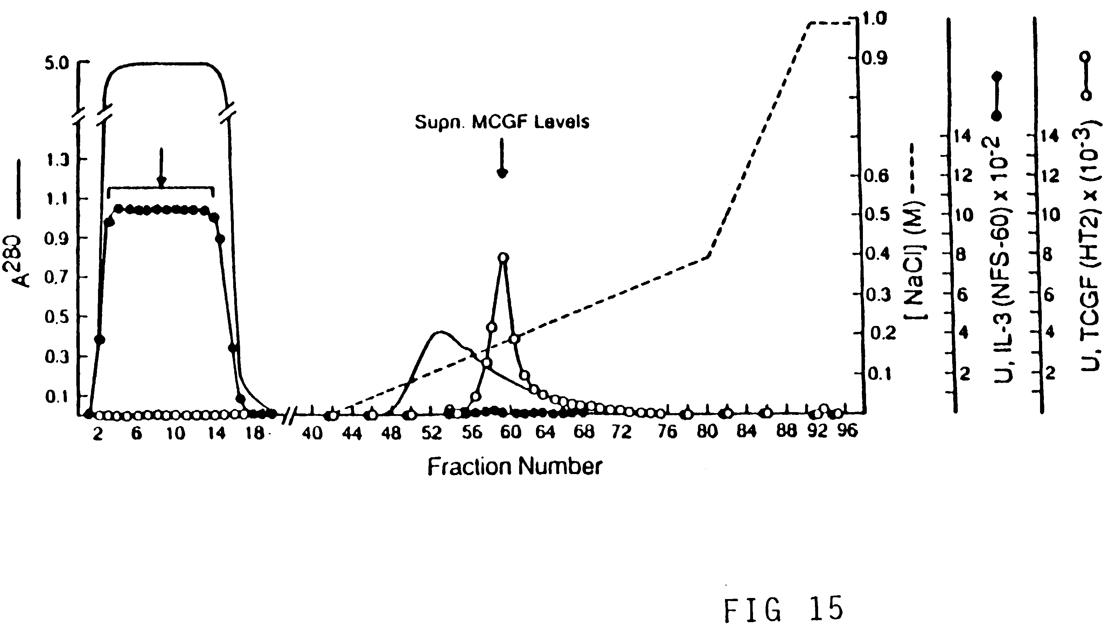

Figure 15 illustrates the FPLC cation exchange chromatography of C1.1 supernatant. 7 ml (35 mg protein) of concentrated supernatant was dialyzed into 50 nM Na phosphate, 1 mm EDTA, pH 7.0 (7.8 mS/cm) and applied to Pharmacia Mono S column (0.5 x 5 cm) equilibrated with the same buffer (Buffer A). Buffer B = A + 1M NaCl. Elution conditions: 0.5 ml/min flow rate; 0.5 ml/ fraction; 0-40% B in 40 min, 40-100% B in 10 minutes. Aliquots of each fraction were assayed for proliferation activity on NFS-60 (IL-3, closed circles), HT2 (TCGF, open circles) and MC/9 (MCGF) cells. The MC/9 response is not shown; arrows denote positions were MC/9 proliferation levels reached those of C1.1 supernatant.

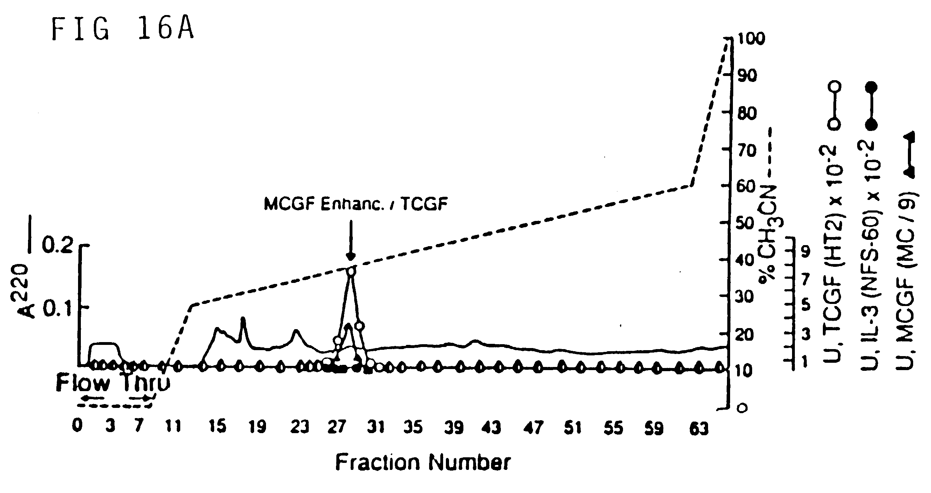

Figure 16A illustrates reverse phase C8 chromatography of IL-3 depleted C1.1 supernatant. 100 µl of 3X mono-S passed supernatant (fractions 59-61, Fig. 15) in 0.1% TFA was loaded onto Pharmacia C8 reversed phase column (0.5 x 2 cm). Buffer A = 0.1% TFA in H₂O, Buffer B = 0.1% TFA in acetonitrile. Elution conditions: 0.5 ml/min, 0.5 ml/fraction, 0-25% B in 4 minutes, 25-60% B in 50 minutes, 60-100% B in 4 minutes. Aliquots of each fraction were assayed for proliferation activity on NFS-60 (IL-3, closed circles), HT2, (TCGF, open circles) and MC/9 cells (MCGF, closed triangles). Arrow denotes fraction containing peak TCGF activity, and which produced MC/9 proliferation levels similar to those of C1.1 supernatant after addition of saturating IL-3^{R} levels to each fraction.

Figure 16B illustrates reverse phase C8 chromatography of GK15-1 murine T cell supernatant. 2 mg (0.5 ml) of concentrated GK15-1 supernatant in 0.1% TFA was applied to a Pharmacia C8 reverse phase column and eluted as above. Aliquots of each fraction were assayed for TCGF activity on HT2 cells (open circles). Arrow marks the position where murine IL-2^{R} eluted under identical conditions. INSERT: titrations of C1.1 (closed circles) and IL-2^{R} (closed squares) and GK15-1 supernatant (open circles) TCGF activities directly compared on the same plate.

Figure 17 depicts the MCGF and TCGF activity of partially purified factor from reverse phase column (RP factor). Proliferation was measured by [³H]-thymidine incorporation after a 24 hour culture period. MC/9 mast cells and HT2 T cells were cultured with varying concentration of C1.1 supernatant (closed circles), IL-3^{R} (open circles), IL-2^{R} (open triangles), RP factor (open squares) and dilutions of RP factor in the presence of 200 units of IL-3^{R} (closed squares) or dilutions of IL-2 in the presence of saturating levels of RP factor (closed triangles).

Figure 18 illustrates chromatofocussing of C1.1 supernatant. 1.5 ml (3.5 mg) of supernatant in 25 nM bis-tris, pH 7.1 was loaded onto a Pharmacia Mono P column (0.5 x 20 cm) and eluted with Polybuffer 74 (1:10), pH 4.0, flow rate 0.5 ml/min, 1 ml/fraction. Aliquots of each fraction were assayed by proliferation on NFS-60 (IL-3, open circles) HT2 (TCGF, closed circles) and MC/9 (MCGF, not shown, except that peak MCGF activity is indicated by arrow). The TCGF peak (fraction 12, pI=6.2) coincides with a minimum IL-3 activity; MCGF proliferation levels are highest at the arrow designation.

Figure 19 depicts an SDS PAGE of C1.1 supernatant and partially purified MCGF/TCGF.
A) Non-reducing SDS PAGE of unfractionated supernatant. Prior reduction with 50 mM DTT (60° x 5 minutes) shifts TCGF peaks to slightly higher molecular weights (21 Kd and 16 Kd), and is accompanied by a drastic loss in activity.
B) Non-reducing SDS PAGE of peak MCGF/TCGF fractions from cation exchange chromatography (Fractions 59-61, Fig. 15). Prior reduction with 50 mM DTT (at 60°C for 5 minutes) shifts MCGF and TCGF peaks to slightly higher molecular weights (21 Kd and 16 Kd), and is accompanied by a drastic loss in activity. Arrows mark the only fractions to which addition of saturating amounts of IL-3 raises MCGF activity to supernatant levels: IL-3 (open circles), TCGF (closed circles), MCGF (open triangles).

The present invention includes glycosylated or unglycosylated mammalian polypeptides which exhibit IL-4 activity, and which are derivable from the IL-4 polypeptides disclosed herein using standard protein engineering techniques. The invention also includes nucleic acids having sequences capable of coding for the polypeptides of the invention, and nucleic acids whose sequences are effectively homologous to the cDNA clones of the invention. Finally, the invention includes methods of making the glycosylated or unglycosylated polypeptides of the invention which utilize the nucleotides sequences disclosed herein, and methods of using the polypeptides of the invention.

Techniques for making, using, and identifying the polypeptides and nucleic acids of the invention are discussed below in general terms. Afterwards several specific examples are provided wherein the general techniques are applied using specific cell types, vectors, reagents, and the like. Their isolation from natural sources will first be briefly described.

These polypeptides can be purified to apparent homogenity from supernatants of readily available cellular sources, and can thus be economically manufactured in pure form, enabling various therapeutic and other utilities.

These polypeptides are characterized by including a polypeptide exhibiting B-cell-, T-cell- and mast-cell-stimulatory activity. In natural form in one species, the polypeptides exhibit a molecular weight on SDS PAGE of about 20 kd and 15 kd under non-reducing conditions and 21 kd and 16 kd under reducing conditions. A composition containing the subject polypeptides in substantially pure form exhibits an isoelectric point (pI) of about 6.2 by chromato-focussing. The growth factors are extremely potent agents, exhibiting significant activity at extremely low concentrations (e.g., less than 1 nanogram per ml). The polypeptides can be distinguished from hormones and other proteins produced naturally by T cells (e.g., IL-2, IL-3, GM-CSF and IFN-γ) through cation exchange chromatography or unique elution conditions from reverse-phase columns.

In murine species, some polypeptides of the present invention support only low levels of proliferation of certain cells, such as IL-3-dependent mast cell lines, but synergistically enhance the growth of such cells (e.g., mast cells) in the presence of a second factor (e.g., IL-3). The polypeptides can stimulate the proliferation of several T-cell lines, but generally to a lesser extent than purified IL-2. Also, such polypeptides can induce Ia expression on resting B-cells and enhance IgG₁ and IgE secretion by B-cells, similar to BSF-1. These activities are not separable despite multiple biochemical fractionations. However, most of the activities are destroyed after reduction, e.g., in the presence of SDS.

These factors can be obtained from a number of natural sources in a variety of ways. One suitable source is any of a number of T-cell lines that produce the subject polypeptides. One such T-cell line was derived from C57GL6 mice (Nable, G. et al., Proc. Natl. Acad. Sci. U.S.A., 78: 1157-1161 (1981), which line can be maintained in modified supplemental DME (Nable, G. et al., Cell, 23: 19-28 (1982). This cell line, originally designated C1.Ly1⁺2⁻/9, was deposited at the American Type Culture Collection and designated accession number CRL 8179. Other suitable cellular sources of these polypeptides include almost any cells that secrete the various antibodies ascribed to the polypeptides, such as human peripheral blood lymphocytes as well as any of the well known T-cell sources, such as tonsils, spleens, etc.

The supernatant from such cellular sources or, in some cases, fluid from the disrupted cells, may be subjected to a variety of well-known purification procedures in order to separate the proteins of the present invention. Preferably, the purification techniques are used in combination to ensure high purity levels. Typically, the purifications will utilize gel filtration chromatography, SP cation exchange chromatography, reverse phase chromatography, group-specific chromatography (e.g., on Heparin Sepharose), or other common separation methods providing effective protein fractionation. High pressure liquid chromatography (HPLC), isoelectric focussing, and SDS polyacrylamide gel electrophoresis may also be utilized.

For example, a supernatant from an appropriate cell line may be first fractionated by strong cation exchange chromatography. Typically, the polypeptides of the present invention remain bound to the column, while about 98% of the loaded protein passes through. The remaining bound protein may be eluted with a salt gradient. In one embodiment of the present invention, a sodium chloride gradient released the subject polypeptide at about 0.19 M. This fractionation was repeated twice more (primarily to remove residual IL-3), providing greater than about 95% purity. Thereafter, the material was successively fractionated by Heparin-Sepharose and reverse phase (c4 or c18) chromatography. In the latter case, these polypeptides eluted at 42% acetonitrile. These pooled fractions contain the subject polypeptide in extremely high purity, essentially to homogeneity (a single band at about 20 kd on silver-stained SDS-PAGE). This purified material is at least 65,000 times more pure than the material as it exists in natural form.

These polypeptides may be used as antigens for the production of antibodies, which in turn may be used as antigens for the production of anti-idiotypic antibodies. Either polyclonal or monoclonal antibodies may be prepared in conventional ways. These polypeptides or fragments thereof, generally fragments having at least about 15 amino acids, may be used by themselves, but are preferably bound or linked to an adjuvant or antigen which activates the immune system. Various antigens may be used, such as serum albumins, keyhole limpet hemocyanin, globulins, or the like. A wide variety of techniques are available for linking adjuvants to polypeptides, such as glutaraldehyde, maleimidobenzoic acid, diazobenzoic acid, or the like. Adjuvants include Freund's adjuvant, aluminum hydroxide, or the like. The antigen is injected into an appropriate host in conventional amounts, and one or more booster injections may be made at from 2 to 4 week intervals. Where monoclonal antibodies are employed, normally a mouse is injected with the original and booster injections and the spleen is isolated, and then the splenocytes are fused with an appropriate fusion partner in accordance with conventional techniques. See, for example, Galfre et al., Nature (1977) 266:550; Kennett et al., Current Topics in Microbiology and Immunology (1978) 81:77; and U.S. Patent Nos. 4,381,292 and 4,363,799. However, for special purposes, other mammals may be employed, such as primates, e.g., humans, for production of antibodies having human F_{c} chains.

The polypeptides may be used by themselves or in combination with the antibodies in diagnosis for the polypeptides. Either or both may be labeled or unlabeled for use in diagnostic assays. A large number of diagnostic assays are described in the literature and including binding, either directly or indirectly, these polypeptides or antibodies to a variety of labels, such as enzymes, radionuclides, fluorescers, substrates, coenzymes, particles, e.g., magnetic particles, or the like. As illustrative of these assays see for example U.S. Patents Nos. 3,817,837; 3,850,752; 4,174,384; 4,177,437 and 4,374,925.

Various assays are divided arbitrarily into homogeneous and heterogeneous immunoassays, where the distinction is based on whether the complex between the polypeptide and its antibody must be separated from the uncomplexed members of the specific binding pair. Various assays are referred to as EI, ELISA, RIA, homogeneous EIA, dot-blot, Westerns, or the like.

Antibodies to these polypeptides may be used in themselves as antigens to produce anti-idiotypes, which may serve as competitive antigens, having epitopic sites competitive with epitopic sites of these polypeptides. These anti-idiotypes may therefore serve as tumor inhibitors, as substitutes for the subject polypeptides or as antagonists for the subject polypeptides.

These polypeptides form a family of naturally-occurring polypeptides which may be derived from natural sources, as well as non-naturally occurring polypeptides which share physiological properties, such as binding specificity and B-cell or mast cell stimulatory activity. Minor differences between species or origin may require modification in the purification protocols, as known to those skilled in the art. Alternatively, these polypeptides may be sequenced and then fragments synthesized in accordance with well-known techniques. See, for example, Barany and Merrifield, Solid-Phase Peptide Synthesis, "The Peptides, Analysis Synthesis Biology," Special Methods in Peptide Synthesis, Part A, Vol. 2, Gross and Merenhofer, eds., Academic Press, N.Y. 1980, pp. 1-284.

The polypeptide compositions of the present invention will find utility in a variety of ways. For example, these polypeptides can indue the growth of T-cells and mast cells, either in culture or in vivo. Mast cells, as noted previously, are important sources of heparin, histamines, prostaglandins, and other physiologically important materials. Similarly, the growth factors may be utilized to stimulate B- and T-cells, especially those known to secrete proteins (e.g., lymphokines and immunoglobulins) useful in the immune system. Typically, the factors will be incorporated into culture medium added to cell cultures. A suitable concentration of the factors will vary depending upon the type of cell line, but will generally be present in about 0.1 µg/ml to about 1 mg/ml, more preferably about 1 µg/ml to about 10 µg/ml, in cultures. The use of the growth factors of the present invention can eliminate the requirement of utilizing feeder cells to maintain the desired cell lines, resulting in substantial increases in the purity of the products from the cell line.

The polypeptide compositions of the present invention will also find use in vivo in the treatment of various immune deficiencies, or to increase the natural immune response. For example, such polypeptide compositions may aid in the treatment or prevention of infection by stimulating the maturation and/or growth of B-cells, T-cells and mast cells, thus decreasing the animal's susceptibility to infection.

### I. De Novo Preparation of IL-4 cDNA

A variety of methods are now available for de novo preparation and cloning of cDNAs, and for the construction of cDNA libraries: e.g. recent reviews are given by Doherty, "Cloning and Expression of cDNA", Chapter 10 in Gottesman, Ed. Molecular Cell Genetics (John Wiley & Sons, New York, 1985); and Brandis et al., "Preparation of cDNA Libraries and the Detection of Specific Gene Sequences", in Setlow et al., Eds. Genetic Engineering, Vol. 8, pgs. 299-316 (Plenum Press, New York, 1986).

By way of example, total mRNA is extracted (e.g., as reported by Berger, S. et al., Biochemistry 18: 5143-5149 [1979]) from cells (e.g., a nontransformed human T-cell source) producing polypeptides exhibiting the desired activity. The double-stranded cDNAs from this total mRNA can be constructed by using primerinitiated reverse transcription (Verme, I., Biochem. Biophys. Acta, Vol. 473, pgs. 1-38 [1977]) to make first the complement of each mRNA sequence, and then by priming for second strand synthesis (Land, H. et al., Nucleic Acids Res., 9: 2251-2266 [1981]). Subsequently, the cDNAs can be cloned by joining them to suitable plasmid or bacteriophage vectors (Rougeon, F. et al., Nucleic Acids Res., 2, 2365-2378 [1975]) or Scherer, G. et al., Dev. Biol. 86, 438-447 [1981]) through complementary homopolymeric tails (Efstratiadis, A. et al., Cell, 10, 571-585 [1977]) or cohesive ends created with linker segments containing appropriate restriction sites (Maniatis et al., Molecular Cloning: A Laboratory Manual, Cold Spring Harbor Laboratory, N.Y. 1982), and then transforming a suitable host. (See generally, Efstratiadis, A., and Villa-Kormaroff, L., "Cloning of double stranded cDNA" in Setlow, J. and Hollaender, A. (eds.) Genetic Engineering, Vol, 1, Plenum Publishing Corp., N.Y., U.S.A. [1982].)

A preferred source of mRNA encoding the desired polypeptides is cells whose supernatants contain the stimulating activities for the B-cell, T-cell and/or mast cells, or other activities associated with the polypeptides of the present invention. One such line is the mouse T-cell line C1.Ly1⁺2⁻/9 (A.T.C.C. Accession No. CRL8179) (Nabel, G. et al., Nature 291: 332-334 (1981)). In general, suitable T-cells can be obtained from a variety of sources, such as mammalian (e.g. human) spleen, tonsils and peripheral blood. T-cell clones, such as those isolated from peripheral blood T-lymphocytes, may also be used (see Research Monographs in Immunology, eds. von Doehmer, H. and Haaf, V.; Section D: "Human T-Cell Clones", vol.8, pgs. 243-333; Elsevier Science Publishers, N.Y. [1985]).

Production of mRNAS capable of coding for IL-4 by such cells can be confirmed by microinjection of the extracted mRNA into oocytes of Xenopus laevis. This microinjection technique is described more fully below, and is disclosed generally in Colman et al., "Export of Proteins from Oocytes of Xenopus Laevis", Cell, Vol. 17, pgs. 517-526 (1979); and Maniatis et al. Molecular Cloning: A Laboratory Manual, pgs. 350-352 (Cold Spring Harbor Laboratory, New York, 1982).

If the mRNAs coding for a desired IL-4 make up a very small fraction of the total mRNA steps may be necessary to enrich the fractional concentration in order to make practical the screening procedure for detecting cDNA clones of interest. Such procedures are standard in the art and are disclosed in the examples below and in several papers and references, such as Maniatis et al., pgs. 225-228, cited above; Suggs et al., Proc. Natl. Acad. Sci., Vol. 78, pgs. 6613-6617 (1981); Parnes et al., Proc. Natl. Acad. Sci., Vol. 78, pgs. 2253-2257 (1981), Davis et al., Proc. Natl. Acad. Sci., Vol. 81, pgs. 2194-2198 (1984).

A preferred method of de novo preparation of IL-4 cDNAs relies on functional expression of the cDNas in pcD expression system developed by Okayama and Berg, disclosed in Mol. Cell. Biol., Vol. 2, pgs. 161-170 (1982); and Vol. 3. pgs. 280-289 (1983), and available from Pharmacia (Piscataway, N.J.). The pcD expression vector contains the SV40 early promoter, late splicing junction, and the replication origin. This vector permits expression of cDNA inserts in COS 7 monkey cells which provide T antigen for replication of the pcD plasmid. Screening of cDNA libraries includes transfection of pools of plasmid DNA into COS 7 cells using DEAE-Dextran. Since lymphokines, and in particular IL-4s, are secreted proteins, the supernatants from the transfected cells can be assayed for biological activity after incubation for several days. Positive pools are further divided to identify single cDNA clones which give biological activity after transfection.

Briefly, the Okayama and Berg expression vector is constructed as follows. Polyadenylated mRNA is annealed to a polydeoxythymidylic acid (oligo dT) tail attached to the protruding strand of a KpnI-digested pBR322 plasmid containing the SV40 early promoter region. That is, the entire vector serves as a primer for cDNA synthesis. After cDNA synthesis, 3' polydeoxycytidylate (oligo dC) tails are attached, followed by HindIII digestion, which lops off (at a unique HindIII site) a fragment of the SV40 DNA to which one of the oligo dC tails is attached. The SV40 early promoter remains intact, and fortuitously occurring HindIII sites of the insert are affected minimally because the hybrid cDNA/RNA is resistant to HindIII digestion. A separately constructed HindIII fragment having a 3' polyquanidylated (oligo dG) tail is annealed to the sticky end left by the Hind III digestion. The vector is circularized and treated with E. coli RNase H, DNA polymerase I, and DNA ligase to replace the RNA strand with DNA. The vectors are cloned in E. coli to form the cDNA library. The SV40 elements permit the vectors to be expressed in eucaryotic cells as well as procaryotic cells, and particularly in mammalian cells, such as COS7 monkey cells or Chinese hamster ovary (CHO) cells.

Once the cDNA library in the Okayama/Berg plasmid vector has been completed, the cDNA clones are collected, and random pools checked for the presence of the desired cDNAs by standard procedures, e.g. hybrid selection, detection of antigenic determinants on expressed products, and/or functional assays. Positive pools can then be probed with a cDNA from an induced T cell line. Thereafter, the positive, probed pools are divided into individual clones which are further tested by transfection into a suitable host (such as a mammalian cell culture), and the host supernatant is assayed for activity.

### II. Preparation of IL-4 cDNAs Via Hybridization Probes Derived from Disclosed cDNAs

The cDNAs disclosed herein can be used as probes to identify homologous sequences in different cell types, as an alternative to de novo isolation and cloning of the IL-4 coding nucleotides. Standard techniques are employed, e.g. Beltz et al., "Isolation of Multigene Families and Determination of Homologies by Filter Hybridization Methods," Methods in Enzymology, Vol. 100, pgs. 266-285 (1983); and Callahan et al., "Detection and Cloning of Human DNA Sequences Related to the Mouse Mammary Tumor Virus Genome," Proc. Natl. Acad. Sci., Vol. 79, pgs. 5503-5507 (1982). Basically, the cDNAs of the invention are used to construct probes (using standard techniques, e.g. see Maniatis et al., cited above) for screening, at low hybridization stringencies, genomic or cDNA libraries (again, constructed by standard techniques) of a cell type suspected of producing IL-4. Standard screening procedures are employed, e.g. Grunstein et al., Proc. Natl. Acad. Sci., Vol. 72, pgs. 3961-3965 (1975); or Benton et al., Science, Vol. 196, pgs. 180-183 (1977).

As described more fully below, human IL-4 was isolated by a murine IL-4 probe. Subsequent analysis indicated about 70% homology between selected regions of the human and mouse cDNAs. Given the evolutionary distance between mice and humans it is believed that most, if not all, mammalian IL-4 genes are detectable by probes constructed from one or more cDNAs of the invention: Wilson et. al. "Biochemical Evolution", Ann. Rev. Biochem., Vol. 46, pgs. 573-639 (1977); Kimura, "The Neutral Theory of Molecular Evolution," Chapter 11 in Nei and Koehn, Eds. Evolution of Genes and Proteins (Sinauer Associates, Sunderland, MA, 1983).

### III. Preparation of Mutant IL-4s by Protein Engineering

Once nucleic acid sequence and/or amino acid sequence information is available for a native protein a variety of techniques become available for producing virtually any mutation in the native sequence. Shortle, in Science, Vol. 229, pgs. 1193-1201 (1985), reviews techniques for mutating nucleic acids which are applicable to the present invention. Preferably, mutants of the native IL-4s, i.e. IL-4 muteins, are produced by site-specific oligonucleotide-directed mutagenesis, e.g. Zoller and Smith, Methods in Enzymology, Vol. 100, pgs. 468-500 (1983); Mark et al., U.S. Patent 4,518,584 entitled "Human Recombinant Interleukin-2 Muteins"; or by so-called "cassette" mutagenesis described by Wells et al., in Gene, Vol. 34, pgs. 315-323 (1985); and Estell et al., Science, Vol. 233, pgs. 659-663 (1986). In sections below, the notation used by Estell et al. (cited above) to identify muteins is followed and generalized. For example, "human IL-4 mutein Leu⁸²" (or simply "Leu⁸²" if the native protein is understood from the context) indicates a polypeptide whose amino acid sequence is identical to that of the native protein except for position 82 with respect to the N-terminus. At that position Leu has been substituted for Phe. More than one substitution can be similarly indicated; e.g., a mutein having Leu substituted for Phe at position 82 and Asp for Asn at position 111 is referred to as human IL-4 mutein (Leu⁸², Asp¹¹¹). Deletions are indicated by "Δ's". For example, a mutein lacking Gln at position 71 is referred to as human IL-4 mutein Δ⁷¹. An insertion is indicated by "IS(Xaa)". For example, a mutein with a Leu inserted after Gln at position 71 is referred to as human IL-4 mutein IS⁷¹(Leu). Thus, human IL-4 mutein (Ser¹³, Δ⁷¹, IS⁹⁴(Gly)) represents the native human IL-4 sequence which has been modified by replacing Thr by Ser at position 13, deleting Gln at position 71, and inserting Gly immediately after Ala at position 94. Insertion of multiple amino acids at the same site is indicated by ISⁱ(Xaa₁-Xaa₂-Xaa₃-...), where Xaa₁-Xaa₂-Xaa₃... is the sequence inserted after position i. N-terminal additions are indicated by superscript "0", e.g. IS⁰(Xaa), and a sequence of deletions, for example of amino acids 6-10, is designated either as Δ⁶⁻¹⁰ or as (Δ⁶, Δ⁷, Δ⁸, Δ⁹, Δ¹⁰).

Most preferably cassette mutagenesis is employed to generate human IL-4 muteins. As described more fully below, a synthetic human IL-4 gene has been constructed with a sequence of unique restriction endonuclease sites spaced approximately uniformly along the gene. The uniqueness of the restriction sites should be retained when the gene is inserted into an appropriate vector, so that segments of the gene can be conveniently excised and replaced with synthetic oligonucleotides (i.e. "cassettes") which code for desired muteins.

Determination of the number and distribution of unique restriction sites entails the consideration of several factors including (1) preexisting restriction sites in the expression vector, (2) whether species-specific or genera-specific codon usage is desired, and (3) the convenience and reliability of synthesizing and/or sequencing the segments between the unique restriction sites.

### IV. Biological Properties and Assays for IL-4 Activity.

Mammalian IL-4 of the invention is defined in terms of biological activities and/or homology with the disclosed embodiments. Mammalian IL-4s of the invention include proteins and muteins (of the disclosed native polypeptides) which are homologous to the disclosed native polypeptides and which exhibit both BCGF activity and TCGF activity. Mammalian IL-4s of the invention are alternatively defined by their biological activities (defined more fully below) which include BCGF activity and TCGF activity (which is referred to herein as IL-4 activity) as well as at least one or more activities selected from the group of activities consisting of MHC antigen induction activity, Fc-epsilon receptor induction activity, GM-CSF stimulated granulocyte colony growth potentiating activity, interleukin-2 TCGF potentiating activity, and IgG₁- and IgE-induction activity.

It is believed that IL-4s are species-specific in their activities. That is, for example, human IL-4 exhibits TCGF activity as assayed by human T cell lines, but not as assayed by murine T cell lines. And conversely, murine IL-4 exhibits TCGF activity as assayed by murine T cell lines, but not as assayed by human T cell lines.

### A. TCGF Activity

Several standard assays have been described for TCGF activity, e.g. Devos et al., Nucleic Acids Research, Vol. 11, pgs. 4307-4323 (1983); Thurman et al., J. Biol. Response Modifiers, Vol. 5, pgs 85-107 (1986); and Robert-Guroff et al., Chapter 9 in Guroff, Ed. Growth and Maturation Factors (John Wiley, New York, 1984). Generally, the TCGF assays are based on the ability of a factor to promote the proliferation of peripheral T lymphocytes or IL-2 dependent T cell lines, e.g. Gillis et al. J. Immunol., Vol. 120, pg. 2027 (1978). Proliferation can be determined by standard techniques, e.g. tritiated thymidine incorporation, or by colorimetric methods, Mosmann, J. Immunol. Meth., Vol. 65, pgs. 55-63 (1983).

By way of example, human TCGF activity can be assayed by the following steps: (1) washed human peripheral blood lymphocytes (about 2 x 10⁵ in 50 microliters) previously stimulated with phytohemagglutinin (PHA) for 7 days and subsequently cultured for 7 days with IL-2 are added to a microtiter well; (2) dilutions (50 microliter) of the TCGF-containing material are added to each well; (3) the lymphocytes are incubated 72 hours at 37°C; (4) tritiated thymidine (about 20 microliters, 20 microcuries/ml) is added to each well; and (5) cells are harvested onto filter paper strips, washed, and counted in a scintillation counter.

As described more fully in the examples, some forms of IL-4 have the capability of potentiating the TCGF activity of IL-2. "Potentiation" as used herein in reference to such activity means that the maximal level of proliferation in a TCGF assay system caused by IL-2 is increased by the addition of IL-4.

### B. BCGF Activity

BCGF activity is defined by an assay disclosed by Howard et al., J. Exp. Med, Vol. 155, pgs. 914-923 (1982). Assays for BCGF are reviewed generally by Howard and Paul, in Ann. Rev. Immunol., Vol. 1, pgs. 307-333 (1983). Briefly, BCGF activity is measured by the degree to which purified resting B cells are stimulated to proliferate in the presence of a submitogenic concentration of anti-IgM, or like antigen. By way of example, assay of human BCGF activity can be carried out by the following steps:

Enriched B cell populations are obtained from peripheral blood, spleen, tonsils, or other standard sources by Ficoll/Hypaque density gradient centrifugation (e.g. Pharmacia) and two cycles of rosetting with 2-aminoethylisothiouronium bromide-treated sheep erythrocytes to eliminate T cells. Such B cell preparations should contain more than 95% surface Ig⁺ cells and more than 95% cells positive for human B-cell specific antigen, as determined by the anti-human B-cell specific monoclonal antibody B1 available from Coulter (Hialeah, FL). T cell contamination should be less than 1% as determined by staining with anti-Leu-1 monoclonal antibodies (Becton-Dickinson, Mountain View, CA) or OKT 11 antibodies (Ortho Diagnostics, Westwood, MA). 3 milliliter cultures of such B lymphocytes (about 5 x 10⁵ per ml in Yssel's medium, Yssel et al., J. Immunol. Meth., Vol. 65, pgs. 55-63 (1984)), are activated by either Staphylococcus aureus Cowan I strain (SAC) (e.g. 0.01% solution of SAC, which is available from Calbiochem under the tradename Pansorbin, or which can be prepared as described by Falkoff et al., J. Immunol., Vol. 129, pg. 97-102 (1982)) or anti-IgM antibodies (e.g. BRL, Gaithersburg, MD) coupled to beads, e.g. 5 microgram/ml of Immunobeads available from Bio-Rad (Richmond, CA). The B cells are cultured either for 24 hours (in the case of SAC) or 72 hours (for anti-IgM beads) and then repurified by Ficoll/Hypaque density centrifugation to remove SAC particles, beads, nonviable cells, and the like. B cell proliferation is measured by plating about 5 x 10⁴ B lymphocytes in 50 microliters of medium in 0.2 ml flat-bottomed microtiter wells. Various dilutions of the materials suspected of having BCGF activity are added in a final volume of 50 microliters. Tritiated thymidine incorporation is determined after 48 hours (anti-IgM cultures) or 72 hours (SAC cultures). Similar assays are also disclosed by Muraguchi et al., J. Immunol., Vol. 129, pgs. 1104-1108 (1982); and Yoshizaki et al., J. Immunol., Vol. 128, pgs. 1296-1301 (1981).

### C. MHC Antigen Induction.

It has been demonstrated that IL-4 can induce the expression of MHC antigens (e.g., Ia in mice) in various cell types of the immune system, particularly B cells. Roehm et al., in J. Exp. Med., Vol. 160, pgs. 679-694, presented evidence that a factor exhibiting BCGF activity was also capable of inducing the expression of MHC antigens on normal resting B cells. Assays for MHC antigen induction are generalizations of the assays for murine B cells presented in that reference. Briefly, immune system cells are exposed to IL-4, and then expression of particular MHC antigens on the cells' surfaces are determined by labeled antibodies specific for that antigen. The degree of induction is determined by comparison of the induced cells with controls. Several different antibodies can be employed for any given species. Several hybridomas are available from the ATCC which produce monoclonal anti-MHC antigen antibodies, and several are available commercially (for example, anti-HLA-DR produced by hybridomas under ATCC accession numbers HB103, HB109, RB110, or HB151; anti-I-A^{b,d} produced by hybridoma under ATCC accession number HB35; anti-HLA-DR L243 available from Becton Dickinson (Mountain View, CA); or the like). Some routine experimentation may be required to adapt the assay to a particular species, and to optimize conditions to give the most sensitive read-out of MHC antigen levels. For the human MHC antigen induction assay, purified B cells can be prepared as described above, or by similar techniques. Alternatively, MHC induction can be assayed on unpurified preparations of spleen cells. Antibody-labeled cells are preferably detected by flow cytometry, e.g. on a Becton Dickinson FACS-type instrument, or the equivalent.

### D. MCGF Activity

It is believed that IL-4s generally exhibit MCGF activity. However, because of the lack of adequate assay techniques, MCGF activity has only been demonstrated for rodent IL-4. Murine IL-4 MCGF assays are based on the proliferation of factor-dependent murine mast or basophil cell lines. In particular, MCGF activity can be assayed with the murine mast cell line MC/9, which is deposited with the ATCC under accession number CRL 8306 and is described in U.S. Patent 4,559,310 and in Nabel et al., Cell, Vol. 23, pg. 19 (1981). Murine MCGF assays are also described by Ihle et al., in J. Immunol., Vol. 127, pg. 794 (1981).

Preferably MCGF activity is determined by the colorimetric assay of Mosmann (cited above) with the use of MC/9 cells. Briefly, MC/9 cells are cultured in flat-bottom Falcon microtiter trays (10⁴ cells/well) in Dulbecco's modified medium supplemented with 4% fetal calf serum, 50 µM 2-mercaptoethanol, 2 mM glutamine, nonessential amino acids, essential vitamins, and varied concentrations of test supernatants in a final volume of 0.1 ml. Fifty micrograms of 3-(4,5-dimethylthiazol-2-yl)-2,5-diphenyl tetrazolium bromide (Sigma) in 10 µl of phosphate-buffered saline were added to each cell culture after a 20-hr incubation. Four hours later, 0.1 ml of 0.04 M HCl in isopropanol was added to solubilize the colored formazan reaction product. The absorbance at 570 nm (reference 630 nm) is measured on a Dynatek Microelisa Autoreader (MR580), or similar instrument.

### E. Fc-epsilon Receptor Induction.

It has been discovered that IL-4 induces Fc-epsilon expression on B cells and on T cells, but particularly on human B cells stimulated by anti-IgM antibodies, or like antigen. It has also been discovered that gamma interferon specifically inhibits IL-4-induced Fc-epsilon expression on B cells.

Preferably, the assay for Fc-epsilon receptor induction proceeds initially as for the BCGF assay. That is, purified B cells are obtained which are then stimulated with anti-IgM antibody (or like antigen) and are exposed to IL-4. Finally the cells are assayed for Fc-epsilon receptors.

Several assays are available for quantifying Fc-epsilon receptors on cell surfaces, e.g. Yodoi and Ishizaka, J. Immunol., Vol. 122, pgs. 2577-2583 (1979); Hudak et al., J. Immunol Meth., Vol. 84, pgs. 11-24 (1985); and Bonnefoy et al., J. Immunol. Meth., Vol. 88, pgs. 25-32 (1986). In particular, Fc-epsilon receptors can be measured by flow cytometry with labeled monoclonal antibodies specific for the receptors, e.g. using a Becton Dickinson FACS-IV, or like instrument. Fc-epsilon receptor specific monoclonals can be constructed using conventional techniques.

### F. IgG₁- and IgE-Induction.

IL-4 induces the secretion of IgE and IgG₁ isotypes in lipopolysaccharide (LPS)-activated B cells, e.g. Coffman et al., J. Immunol., Vol. 136, pgs. 4538-4541 (1986); Sideras, et al., Eur. J. Immunol., Vol. 15, pgs. 586-593 (1985). These activities can be measured by standard immunoassays for antibody isotype, as described by Coffman et al., J. Immunol., Vol. 136, pgs. 949-954 (1986). Briefly, B cells are LPS-activated by culturing them with, for example, about 4 micrograms/ml of Salmonella typhimurium LPS (available from Sigma) or about 50 microgram/ml LPS extracted from E. coli 055 (as described by Sideras et al., cited above). After 4 - 8 days' culture, supernatants are harvested for assaying. Standard isotype-specific ELISA-type assays can be used to measure the various isotype concentrations. Anti-isotype antibodies for the assay are available commercially, or can be obtained from the ATCC.

### G. Colony Stimulating Factor (CSF) Activity.

To determine CSF activity, hemopoietic cells, e.g. bone marrow cells or fetal cord blood cells, are made into a single cell suspension. The individual cells are then "immobilized" in a semi-solid (agar) or viscous (methylcellulose) medium containing nutrients and usually fetal calf serum. In the presence of an appropriate stimulating factor, individual cells will proliferate and differentiate. Since the initial cells are immobilized, colonies develop as the cells proliferate and mature. These colonies can be scored after 7-14 days, Burgess, A., Growth Factors and Stem Cells, pgs. 52-55, Academic Press, New York [1984]. (For specific application to the growth of granulocytes and macrophages, see Bradely, T. and Metcalf, D., Aust. J. Exp. Biol. Med. Sci. Vol. 44, pgs. 287-300 [1966], and see generally Metcalf, D., Hemopoietic Colonies, Springer-Verlag, Berlin [1977]). If desired, individual colonies can be extracted, placed on microscope slides, fixed and stained with Wright/Geimsa (Todd-Sanford, Clinical Diagnosis by Laboratory Methods, 15th Edition, Eds. Davidson and Henry [1974]). Morphological analysis of cell types present per single colony can then be determined.

Bone marrow cells collected from patients with nonhematologic disease are layered over Ficoll (type 400, Sigma Chemical Co., St. Louis, MO), centrifuged (2,000 rpm, 20 min), and the cells at the interface removed. These cells are washed twice in Iscove's Modified Dulbecco's Medium containing 10% fetal calf serum (FCS), resuspended in the same medium and the adherent cells removed by adherence to plastic Petri dishes. The nonadherent cells are added at 10⁵ cells/ml to Iscove's Medium containing 20% FCS, 50 µM 2-mercaptoethanol, 0.9% methylcellulose and varied concentrations of either supernatants known to contain colony stimulating activity or test supernatants. One ml aliquots are plated in 35 mm petri dishes and cultured at 37°C in a fully humidified atmosphere of 6% CO₂ in air. Three days after the initiation of the culture, 1 unit of erythropoietin is added to each plate. Granulocyte-macrophage colonies and erythroid bursts are scored at 10-14 days using an inverted microscope.

Cord blood cells collected in heparin are spun at 2,000 rpm for 6 min. The white blood cells at the interface between the plasma and red blood cell peak are transferred to a tube containing 0.17 N ammonium chloride and 6% FCS. After 5 min on ice, the suspension is underlaid with 4 ml FCS and centrifuged for 6 mins at 2,000 rpm. The cell pellet is washed with Dulbecco's phosphate buffered saline and put through the Ficoll and plastic adherence steps as described above for bone marrow cells. The low density nonadherent cells are collected and placed at 10⁵ cells/culture in the semi-solid culture medium as described above.

At the end of the assays, the cellular composition is determined after applying the individual colonies to glass slides and staining with Wright-Giemsa. Eosinophils are determined by staining with Luxol Fast Blue (Johnson, G. and Metcalf, D., Exp. Hematol. Vol. 8, pgs. 549-561 [1980]).

"Potentiation" as used herein in reference to GM-CSF-stimulated granulocyte growth means that granulocyte colonies in the assays described above are larger when GM-CSF is used with IL-4 than when GM-CSF is used alone to stimulate colony growth.

### V. Purification and Pharmaceutical Compositions

The polypeptides of the present invention expressed in E. coli, in yeast or in other cells can be purified according to standard procedures of the art, including ammonium sulfate precipitation, fractionation column chromatography (e.g., ion exchange, gel filtration, electrophoresis, affinity chromatography, etc.) and ultimately crystallization (see generally "Enzyme Purification and Related Techniques, "Methods in Enzymology, 22: 233-577 [1977], and Scopes, R., Protein Purification: Principles and Practice, Springer-Verlag, New York [1982]). Once purified, partially or to homogeneity, the polypeptides of the invention may be used for research purposes, e.g., as a supplement to cell growth media (e.g., minimum essential medium Eagle, Iscove's modified Dulbecco Medium or RPMI 1640; available from Sigma Chemical Company (St. Louis, MO) and GIBCO Division (Chagrin Falls, OH)) and as an antigenic substance for eliciting specific immunoglobulins useful in immunoassays, immunofluorescent stainings, etc. (See generally Immunological Methods, Vols. I & II, Eds. Lefkovits, I. and Pernis, B., Academic Press, New York, N.Y. [1979 & 1981]; and Handbook of Experimental Immunology, ed. Weir, D., Blackwell Scientific Publications, St. Louis, MO [1978].)

The polypeptides of the present invention may also be used in pharmaceutical compositions, e.g., to enhance natural defense against various infections. Thus, patients with rheumatoid arthritis, in need of a transplant, or with immunodeficiency caused by cancer chemotherapy, advanced age, immunosuppressive agents, etc., may be treated with such polypeptides. The compositions can selectively stimulate various components of the immune system, either alone or with other agents well known to those skilled in the art. In particular, the compositions may include other immune-reactive agents, such as lymphokines (e.g. IL-1, IL-2, etc.), any of the colony stimulating factors, immunoglobulins, etc., in view of the potentiating activities of the polypeptides of the present invention. The polypeptides will also find use in situations (in vivo or in vitro) in which enhanced cellular proliferation or immunoglobulin production is desired.

Pharmaceutical compositions of this invention can be prepared by mixing these polypeptides with preferably inert, pharmaceutically acceptable carriers. Suitable carriers and processes for their preparation are well known in the art (see, e.g., Remington's Pharmaceutical Sciences and U.S. Pharmacopeia: National Formulary, Mack Publishing Company, Easton, PA [1984]). The preferred course of administration is parenteral and can include use of mechanical delivery systems.

Preferably, the pharmaceutical composition is in unit dosage form, each unit containing an appropriate quantity of the active component. The quantity of active compound in a unit dose of preparation may be varied or adjusted from 1 µg to 100 mg, according to the particular application and the potency of the active ingredient. The composition can, if desired, also contain other therapeutic agents.

The dosages may be varied depending upon the requirement of the patient, the severity of the condition being treated and the particular compound being employed. The term "effective amount" as used herein is meant to take these factors into account when dosages are considered. Determination of the proper dosage for a particular situation is within the skill of the art. Generally, treatment is initiated with smaller dosages which are less than the optimum dose of the compound. Thereafter, the dosage is increased by small amounts until the optimum effect under the circumstances is reached. For convenience, the total daily dosage may be divided and administered in portions during the day.

### VI. Expression Systems

A wide range of expression systems (i.e. host-vector combinations) can be used to produce the proteins and muteins of the present invention. Possible types of host cells include but are not limited to cells from bacteria, yeast, insects, mammals, and the like. Optimizing the expression of a particular protein or mutein depends on many factors, including (1) the nature of the protein or mutein to be expressed, e.g. the expressed product may be poisonous to some host systems, (2) whether, and what type of, post-translational modifications are desired, e.g. the extent and kind of glycosylation desired may affect the choice of host, (3) the nature of the 5' and 3' regions flanking the coding region of the protein or mutein of interest, e.g. selection of promoters and/or sequences involved in the control of translation is crucial for efficient expression, (4) whether transient or stable expression is sought, (5) the ease with which the expressed product can be separated from the proteins and other materials of the host cells and/or culture medium, (6) the ease and efficiency of transfecting hosts which transiently express the protein or mutein of interest, (7) the scale of cell culture employed to express the protein or mutein of interest, (8) whether the protein or mutein of interest is expressed fused to a fragment of protein endogenous to the host, and like factors.

In general prokaryotes are preferred for cloning the DNA sequences of the invention. General guides for implementing prokaryotic expression systems are provided by Maniatis et al., Molecular Cloning: A Laboratory Manual (Cold Spring Harbor Laboratory, N.Y., 1982); Perbal, A Practical Guide to Molecular Cloning (John Wiley & Sons, N.Y., 1984); Glover, DNA Cloning: A Practical Approach, Vol. I and II (IRL Press, Oxford, 1985); and de Boer et al., "Strategies for Optimizing Foreign Gene Expression in Escherichia coli," in Genes: Structure and Expression, Kroon, ed. (John Wiley & Sons, N.Y., 1983). For example, E. coli K12 strain 294 (ATCC No. 31446) is particularly useful. Other microbial strains which may be used include E. coli strains such as E. coli B, and E. coli X1776 (ATCC No. 31537). These examples are, of course, intended to be illustrative rather than limiting.

Prokaryotes may also be used for expression. The aforementioned strains, as well as E. coli W3110 (Fs⁻, λ⁻, prototrophic, ATCC No. 27325), bacilli such as Bacillus subtilis, and other enterobacteriaceae such as Salmonella typhimurium or Serratia marcesans, and various pseudomonas species may be used.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, E. coli is frequently transformed using pBR322, a plasmid derived from an E. coli species (Bolivar, et al., Gene Vol. 2, pg. 95 (1977)). pBR322 contains genes for ampicillin and tetracycline resistance and thus provides easy means for identifying transformed cells. The pBR322 plasmid or other microbial plasmid must also contain, or be modified to contain, promoters which can be used by the microbial organism for expression of its own proteins. Those promoters most commonly used in recombinant DNA construction include the β-lactamase (penicillinase) and lactose promoter systems (Chang et al, Nature, Vol. 275, pg. 615 (1978); Itakura, et al, Science, Vol. 198, pg. 1056 (1977); Goeddel, et al, Nature Vol. 281, pg. 544 (1979)) and a tryptophan (trp) promoter system (Goeddel, et al, Nucleic Acids Res., Vol. 8, pg. 4057 (1980); EPO Appl. Publ. No. 0036776). While these are the most commonly used, other microbial promoters have been discovered and utilized, and details concerning their nucleotide sequences have been published, enabling a skilled worker to ligate them functionally with plasmid vectors (Siebenlist et al, Cell Vol. 20, pg. 269 (1980)).

In addition to prokaryotes, eukaryotic microbes, such as yeast cultures, may also be used. Saccharomyces cerevisiae, or common baker's yeast, is the most commonly used among eukaryotic microorganisms. For expression in Saccharomyces, a commonly-used plasmid is YRp7 (Stinchcomb, et al, Nature, Vol. 282, pg 39 (1979); Kingsman et al, Gene, Vol. 7, pg. 141 (1979); Tschemper, et al, Gene, Vol. 10, pg. 157 (1980)). This plasmid already contains the trpl gene which provides a selection marker for a mutant strain of yeast lacking the ability to grow in tryptophan, for example ATCC No. 44076 or PEP4-1 (Jones, Genetics, Vol. 85, pg. 12 (1977)). The presence of the trpl lesion as a characteristic of the yeast host cell genome then provides an effective environment for detecting transformation by growth in the absence of tryptophan.

Suitable promoting sequences in yeast vectors include the promoters for 3-phosphoglycerate kinase (Hitzeman, et al., J. Biol. Chem., Vol. 255, pg. 2073 (1980)) or other glycolytic enzymes (Hess, et al, J. Adv. Enzyme Reg., Vol. 7, pg. 149 (1968); Holland, et al, Biochemistry, Vol. 17, pg. 4900 (1978)), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. In constructing suitable expression plasmids, the termination sequences associated with these genes are also ligated into the expression vector at a position 3' of the sequence to be expressed, to provide polyadenylation of the mRNA and termination. Other promoters, which have the additional advantage of transcription controlled by growth conditions, are the promoter regions for alcohol dehydrogenase 2, isocytochrome C, acid phosphatase, degradative enzymes associated with nitrogen metabolism, the aforementioned glyceraldehyde-3-phosphate dehydrogenase, and enzymes responsible for maltose and galactose utilization (Holland, ibid.). Any plasmid vector containing a yeast-compatible promoter, origin of replication and termination sequences is suitable.

In addition to microorganisms, cultures of cells derived from multicellular organisms may also be used as hosts. In principle, any such cell culture is workable, whether vertebrate or invertebrate. However interest has been greatest in vertebrate cells, and propagation of vertebrate cells in culture (tissue culture) has become a routine procedure in recent years [Tissue Culture, Academic Press, Kruse and Patterson, editors (1973)]. Examples of such useful host cell lines are VERO and HeLa cells, Chinese hamster ovary (CHO) cell lines, and W138, BHK, COS7, mouse myeloma (ATCC No. TIB 19 or TIB 20), and MDCK cell lines. Expression vectors for such cells ordinarily include (if necessary) an origin of replication and a promoter located in front of the gene to be expressed, along with any necessary ribosome binding sites, RNA splice sites, polyadenylation site, and transcriptional terminator sequences.

For use in mammalian cells, the control functions on the expression vectors are often provided by viral material. For example, commonly used promoters are derived from polyoma, Adenovirus 2, and most frequently Simian Virus 40 (SV40). The early and late promoters of SV40 virus are particularly useful because both are obtained easily from the virus as a fragment which also contains the SV40 viral origin of replication (Fiers, et al, Nature, Vol. 273, pg 113 (1978). Smaller or larger SV40 fragments may also be used, provided that there is included the approximately 250 bp sequence extending from the HindIII site toward the BglI site located in the viral origin of replication. Further, it is also possible, and often desirable, to utilize promoter or control sequences normally associated with the desired gene sequence, provided that such control sequences are compatible with the host cell systems.

An origin of replication may be provided either by an exogenous origin on the vector, e.g., on one derived from SV40 or other viral (e.g. Polyoma, Adeno, VSV, BPV, etc.) source, or endogeneously by the host cell chromosomal replication mechanism. It is often sufficient to integrate the vector into the host cell chromosome.

In selecting a preferred host cell for transfection by the vectors of the invention which comprise DNA sequences encoding both t-PA and DHFR protein, it is appropriate to select the host according to the type of DHFR protein employed. If wild type DHFR protein is employed, it is preferable to select a host cell which is deficient in DHFR, thus permitting the use of the DHFR coding sequence as a marker for successful transfection in selective medium which lacks hypoxanthine, glycine, and thymidine. Such a appropriate host cell is the Chinese hamster ovary (CHO) cell line deficient in DHFR activity, prepared and propagated as described by Urlaub and Chasin, Proc. Natl. Acad. Sci. (USA) Vol. 77, pg. 4216 (1980).

On the other hand, if DHFR protein with low binding affinity for MTX is used as the controlling sequence, it is not necessary to use DHFR-resistant cells. Because the mutant DHFR is resistant to methotrexate, MTX-containing media can be used as a means of selection provided that the host cells are themselves methotrexate-sensitive. Most eukaryotic cells which are capable of absorbing MTX appear to be methotrexate-sensitive. One such useful cell line is a CHO line, CHO-K1 ATCC No. CCL 61.

Invertebrate expression systems include the larvae of silk worm, Bombyx mori, infected by a baculovirus vector, BmNPV, described by Maeda et al. in Nature, Vol. 315, pgs. 892-894 (1985), and in Saibo Koguku, Vol. 4, pgs. 767-779 (1985).

### EXAMPLES

The following examples serve to illustrate the present invention. Selection of vectors and hosts as well as the concentration of reagents, temperatures, and the values of other variables are only to exemplify application of the present invention and are not to be considered limitations thereof.

### MATERIALS AND METHODS

T Cell lines C1.Ly1⁺2⁻/9 (referred to as C1.1), Nabel, G., Greenberger, J.S., Sakakeeny, M.A. and Cantor, H. (1981) Proc. Natl. Acad. Sci. USA 78, 1157-1161, and GK15-1 (supplied by M. Giedlin, DNAX Research Institute of Molecular and Cellular Biology, Inc.; DNAX) were resuspended at 5 x 10⁵ cells/ml in RPMI 1640, 50 µm 2ME, 1% FCS and 2µg/ml Con A for 24 hours. The supernatants were collected and stored at -70°C.

A cloned mast cell line, MC/9 (Nable, G., Galli, S.J., Dvorak, H.F. and Cantor, H. (1981) Nature 291, 332-334), was obtained from G. Nabel (Dana Farber Cancer Institute; DFCI). The mast cell lines MM3 (provided by R. Coffman, DNAX) and Dx-2 (derived by D. Rennick, DNAX) were characterized by the absence of myelomonocytic-associated markers and by the presence of IgE receptors and histamine levels greater than 250 ng/10⁶ cells. The myeloid NFS-60 cell line was provided by J. Ihle (Frederick Cancer Research Facility; FCRF). The NFS-60 cell line was subcloned to obtain an IL-3-dependent clone. The T cell line HT2 was obtained from S. Strober (Stanford University, Palo Alto, CA); CTLL-2 was supplied by W. Farrar (FCRF); and Ly 23/4 was supplied by G. Nabel (DFCI). The mast cell and T cell lines were grown in RPMI 1640, 10% FCS, 50µM 2ME supplemented with recombinant IL-3 or IL-2.

Histamine levels of several IL-3-dependent cell lines were assayed using the method of Shore, P.A., Burkhalter, A., and Conn, V.H. (1959) J. Pharm. Exp. Ther. 127, 182-186. T Cell and mast cell growth factor activities were determined by [³H]-thymidine incorporation or by a colorimetric assay as described by Mosmann, T. in J. Immunol. Methods 65: 55-63 (1983).

IL-3 purified from WEHI 3 supernatant was a gift of J. Ihle (FCRF). Recombinant IL-2, IL-3, GM-CSF and INF-γ were utilized in the form of supernatant from COS-7 monkey kidney cells transfected with the corresponding cDNA clones. One unit of IL-2, IL-3 or GM-CSF was defined as that amount of factor which stimulated 50% maximum [³H]-thymidine incorporation of factor-dependent cell lines (Rennick, D. et al. (1985) J. Immunol. 134: 910-919). One unit of IFN-γ protects 50% of murine L cells from the cytopathic effect of vesicular stomatitis virus (Schreiber, R. et al. (1982) J. Exp. Med. 156: 677-689).

B-cells were prepared from a single cell suspension of mouse spleen cells by depleting T-cells and macrophages according to standard procedures (Howard, M. et al., J. Exp. Med. 155: 914 (1982)). The purified factor described herein was assessed for its ability to induce Ia expression on B-cells using cytofluorometric analysis and to stimulate B-cell proliferation using an anti-Ig co-stimulation assay as described by Roehm, N. et al., J. Exp. Med. 160: 679.

Protein determinations were based either on UV absorption (280 nm or 220 nm) or standard curves constructed using a dye binding assay (Bradford, M. (1976) Annal. Biochem. 72: 248-254). Supernatants were initially concentrated 20 fold with a Pellicon cassette unit (Millipore, Bedford, MA), or (following reverse phase chromatography) by solvent evaporation in a Speedvac (Savant, Farmingdale, NY). SDS PAGE was performed using the Laemmli system (Laemmli, U. (1970) Nature 227: 680-685) with a 12% separating gel. Marker proteins were Pharmacia low MW standards (Pharmacia, Uppsala, Sweden). Gels were silver-stained as described by Merril, C. et al. (1981) Science 211: 1437-1438. To assess directly MCGF and TCGF activity as a function of molecular weight, samples with or without prior reduction with 50 mM DTT were electrophoresed, the gels sliced to 1 mm sections and crushed, and protein was eluted overnight at 4°C into 0.5 ml assay media supplemented with 5 mg/ml ovalbumin (Sigma, St. Louis, MO).

Chromatography was performed at 18°C on a Pharmacia FPLC system equipped with a Kratos Spectroflow 773 UV detector (Kratos, Ramsey, NJ). Cation exchange chromatography utilized a Pharmacia Mono S column (0.5 x 5 cm) equilibrated with 50 mM sodium phosphate, 1 mM EDTA, pH 7.0. Supernatants in the same buffer were applied to the column and eluted with a NaCl gradient to 1M. For reverse phase chromatography, a Pharmacia C8 column (0.5 x 2 cm) was used. The sample, diluted with 0.1% TFA to pH 2, was loaded onto the column and eluted with acetonitrile gradients containing 0.1% TFA.

Isoelectric points of MCGF and TCGF activities were estimated by chromatofocussing on a Pharmacia Mono P column (0.5 x 20 cm). The sample, in 0.025 M bis tris, pH 7.1, was loaded onto the "Mono P" column equilibrated with the same buffer. Gradient elution was effected with Pharmacia Polybuffer 74, pH 4.0 (1:10 dilution, pH adjusted with 0.2 M aminodiacetic acid). Effluent pH was continuously determined with a Pharmacia pH monitor. Prior to injections, all samples were filtered through a Millex GV 0.2µ unit (Millipore).

Fractions from SDS PAGE or chromatographic runs were assayed for their ability to support proliferation of three cell lines, NFS-60 (IL-3), MC/9 (MCGF), and HT2 (TCGF).

For purification of this factor to homogeneity, 8L of Con-A-activated C1.1 supernatant was dialized into 20mM Hepes buffer, pH 7.0, then passed over a Pharmacia Mono S 10/10 cation exchange column equilibrated with the same buffer. Peak activity eluted with a linear salt gradient at 0.2M NaCl. This material was pooled, concentrated, re-dialized into 20 mM Hepes buffer, pH 7.0, and loaded onto a Heparin-Sepharose column equilibrated with the same buffer. Peak activity eluted at 0.45M NaCl with a linear gradient to 2M NaCl. This material was pooled, diluted 10x with 0.10/TFA/H₂O, pH 2, and fractionated on a Vydac reverse phase column (c4). Application of a linear gradient of acetonitrile, 0.1% TFA, released the activity at 42% acetonitrile.

### Example I. de novo Preparation of Murine IL-4 cDNAs from C1.Ly1⁺2⁻/9 Cells and Transient Expression in COS 7 Monkey Cells.

cDNA clones coding for IL-4 were isolated from the murine helper T cell line C1.Ly1⁺2⁻/9, which is deposited with the ATCC under accession number CRL 8179 and described by Nabel et al., in Cell, Vol. 23, pgs. 19-28 (1981), and in Proc. Natl. Acad. Sci., Vol. 78, pgs. 1157-1161 (1981). Other murine cells known to produce BCGF activity include the EL-4 line, available from the ATCC under accession number TIB 39. The procedures used in this example have been disclosed in Lee et al., Proc. Natl. Acad. Sci., Vol. 83, pgs. 2061-2065 (1986). Briefly, a pcD cDNA library was constructed with messenger RNA (mRNA) from concanavalin A (ConA)-induced C1.Ly1⁺2⁻/9 cells following the procedure of Okayama and Berg, described above. IL-3 and GM-CSF clones were eliminated from a large sublibrary of randomly selected clones by hybridization with ³²P-labeled cDNA probes. Pools and/or individual clones from the remainder of the sublibrary were screened for IL-4 cDNA by transfecting COS 7 monkey cells and testing culture supernatants for MCGF and TCGF activity.

### A. Induction of IL-4 Production.

C1.Ly1⁺2⁻/9 cells were induced to produce IL-4 mRNA by ConA as follows. The cells are cultured at 5 x 10⁵/ml in Dulbecco's Modified Eagles medium (DME) with 4% heat-inactivated fetal calf serum, 5 x 10⁻⁵ M 2-mercaptoethanol (2-ME), 2mM glutamine, non-essential amino acids, essential vitamins and 2 µg/ml ConA. After 12-14 hrs' incubation at 37°C in 10% CO₂, the cell suspension is centrifuged at 1500 rpm for 10 minutes. The cell pellets are collected and frozen immediately at -70°C.

### B. Isolation of mRNA

Total cellular DNA was isolated from cells using the guanidine isothiocyanate procedure of Chirgwin, J. et al. (Biochemistry, 18: 5294-5299 [1979]). Frozen cell pellets from ConA-induced C1.Ly1⁺2⁻/9 cells (12 hrs after stimulation) were suspended in guanidine isothiocyanate lysis solution. Twenty ml of lysis solution was used for 1.5 x 10⁸ cells. Pellets were resuspended by pipetting, and then DNA was sheared by 4 passes through a syringe using a 16 gauge needle. The lysate was layered on top of 20 ml of 5.7 M CsCl, 10 mM EDTA in 40 ml polyallomer centrifuge tube. This solution was centrifuged at 25,000 rpm in a Beckman SW28 rotor (Beckman Instruments, Inc., Palo Alto, CA) for 40 hrs at 15°C. The guanidine isothiocyanate phase containing DNA was pipetted off from the top, down to the interface. The walls of the tube and interface were washed with 2-3 ml of guanidine isothiocyanate lysis solution. The tube was cut below the interface with scissors, and the CsCl solution was decanted. RNA pellets were washed twice with cold 70% ethanol. Pellets were then resuspended in 500 µl of 10 mM Tris.HCl pH 7.4, 1 mM EDTA, 0.05% SDS. 50 µl of 3M sodium acetate was added and RNA was precipitated with 1 ml ethanol. About 0.3 mg total RNA was collected by centrifuging and the pellets washed once with cold ethanol.

Washed and dried total RNA pellet was resuspended in 900 µl of oligo (dT) elution buffer (10 mM Tris.HCl, pH 7.4, 1 mM EDTA, 0.5% SDS). RNA was heated for 3 min. at 68°C and then chilled on ice. 100 µl of 5 M NaCl was added. The RNA sample was loaded onto a 1.0 ml oligo (dT) cellulose column (Type 3, Collaborative Research, Waltham, MA) equilibrated with binding buffer (10 mM Tris.HCl pH 7.4, 1 mM EDTA, 0.5 M NaCl, 0.5% SDS.) Flow-through from the column was passed over the column twice more. The column was then washed with 20 ml binding buffer. PolyA⁺ mRNA was collected by washing with elution buffer. RNA usually eluted in the first 2 ml of elution buffer. RNA was precipitated with 0.1 volume 3 M sodium acetate (pH 6) and two volumes of ethanol. The RNA pellet was collected by centrifugation, washed twice with cold ethanol, and dried. The pellet was then resuspended in water. Aliquots were diluted, and absorbance at 260 nm was determined.

### C. Construction of pcD cDNA Library

### 1) Preparation of Vector Primer and Oligo(dG)-Tailed Linker DNAs.

The procedure of Okayama & Berg (Mol. & Cell. Biol. Vol. 2, pgs 161-170 [1982]) was used with only minor modifications. The pcDVl and pLl plasmids are described by Okayama & Berg (Mol. & Cell. Biol. 3: 380-389 [1983]) and are available from Pharmacia (Piscataway, N.J.). Specifically, a modified pcDVI plasmid was used which contained an NsiI site at the previous location of the KpnI site.

An 80 µg sample of pcDVl DNA was digested at 30°C with 20 U of KpnI endonuclease in a reaction mixture of 450 µl containing 6 mM Tris.HCl (pH 7.5), 6 mM MgCl₂, 6 mM NaCl, 6 mM 2-ME, and 0.1 mg of bovine serum albumin (BSA) per ml. After 16 hr the digestion was terminated with 40 µl of 0.25 M EDTA (pH 8.0) and 20 µl of 10% sodium dodecyl sulfate (SDS); the DNA was recovered after extraction with water-saturated 1:1 phenol-CHCl₃ (hereafter referred to as phenol-CHCl₃) and then by precipitation with ethanol. Homopolymer tails averaging 60, but not more than 80, deoxythymidylate (dT) residues per end were added to the NsiI endonuclease-generated termini with calf thymus terminal transferase as follows: The reaction mixture (38 µl) contained sodium cacodylate-30 mM Tris.HCl pH 6.8 as buffer, with 1 mM CoCl₂, 0.1 mM dithiothreitol, 0.25 mM dTTP, the NsiI endonuclease-digested DNA, and 68 U of the terminal deoxynucleotidyl transferase (P-L Biochemicals, Inc., Milwaukee, WI). After 30 min. at 37°C the reaction was stopped with 20 µl of 0.25 M EDTA (pH 8.0) and 10 µl of 10% SDS, and the DNA was recovered after several extractions with phenol-CHCl₃ by ethanol precipitation. The DNA was then digested with 15 U of EcoRI endonuclease in 50 µl containing 10 mM Tris.HCl pH 7.4, 10 mM MgCl₂, 1 mM dithiothreitol, and 0.1 mg of BSA per ml for 5 hr at 37°C. The large fragment, containing the SV40 polyadenylation site and the pBR322 origin of replication and ampicillin-resistance gene, was purified by agarose (1%) gel electrophoresis and recovered from the gel by a modification of the glass powder method (Vogelstein, B. & Gillespie, D., Proc. Natl. Acad. Sci. 76: 615-619 [1979]). The dT-tailed DNA was further purified by absorption and elution from an oligo (dA)-cellulose column as follows: The DNA was dissolved in 1 ml of 10 mM Tris.HCl pH 7.3 buffer containing 1 mM EDTA and 1 M NaCl, cooled at 0°C, and applied to an oligo (dA)-cellulose column (0.6 by 2.5 cm) equilibrated with the same buffer at 0°C and eluted with water at room temperature. The eluted DNA was precipitated with ethanol and dissolved in 10 mM Tris.HCl pH 7.3 with 1 mM EDTA.

The oligo (dG)-tailed linked DNA was prepared by digesting 75 µg of pLl DNA with 20 U of PstI endonuclease in 450 µl containing 6 mM Tris.HCl pH 7.4, 6 mM MgCl₂, 6 mM 2-ME, 50 mM NaCl, and 0.01 mg of BSA per ml. After 16 hr at 30°C the reaction mixture was extracted with phenol-CHCl₃ and the DNA was precipitated with alcohol. Tails of 10 to 15 deoxyguanylate (dG) residues were then added per end with 46 U of terminal deoxynucleotidyl transferase in the same reaction mixture (38 µl) as described above, except that 0.1 mM dGTP replaced dTTP. After 20 min. at 37°C the mixture was extracted with phenol-CHCl₃, and after the DNA was precipitated with ethanol it was digested with 35 U of HindIII endonuclease in 50 µl containing 20 mM Tris.HCl pH 7.4, 7 mM MgCl₂, 60 mM NaCl, and 0.1 mg of BSA at 37°C for 4 hr. The small oligo (dG)-tailed linker DNA was purified by agarose gel (1.8%) electrophoresis and recovered as described above.

### 2) cDNA Library Preparation:

Step 1: cDNA synthesis. The reaction mixture (10 µl) contained 50 mM Tris.HCl pH 8.3, 8 mM MgCl₂, 30 mM KCl, 0.3 mM dithiothreitol, 2 mM each dATP, dTTP, dGTP, and dCTP, 20 µCi ³²P-dCTP (3000 Ci/mmole), 3 µg PolyA⁺ RNA from Con-A induced T-cells, 60 units RNasin (a tradenamed ribonuclease inhibitor from Promega Biotec, Inc., Madison, WI), and 2 µg of the vector-primer DNA (15 pmol of primer end), and 45 U of reverse transcriptase. The reaction was incubated 60 min at 42°C and then stopped by the addition of 1 µl of 0.25 M ETDA (pH 8.0) and 0.5 µl of 10% SDS; 40 µl of phenol-CHCl₃ was added, and the solution was blended vigorously in a Vortex mixer and then centrifuged. 40 µl of 4 M ammonium acetate and 160 µl of ethanol were added to the aqueous phase, and the solution was chilled with dry ice for 15 min., warmed to room temperature with gentle shaking to dissolve unreacted deoxynucleoside triphosphates that had precipitated during chilling, and centrifuged for 10 min. in an Eppendorf microfuge. The pellet was dissolved in 10 µl of 10 mM Tris.HCl pH 7.3 and 1 mM EDTA, mixed with 10 µl of 4 M ammonium acetate, and reprecipitated with 40 µl of ethanol, a procedure which removes more than 99% of unreacted deoxynucleotide triphosphates. The pellet was rinsed with ethanol.

Step 2: Oligodeoxycytidylate [oligo (dC)] addition. The pellet containing the plasmid-cDNA:mRNA was dissolved in 20 µl of 140 mM sodium cacodylate-30 mM Tris.HCl pH 6.8 buffer containing 1 mM CoCl₂, 0.1 mM dithiothreitol, 0.2 µg of poly (A), 70 µM dCTP, 5 µCi ³²P-dCTP, 3000 Ci/mmole, and 60 U of terminal deoxynucleotidyl transferase. The reaction was carried out at 37°C for 5 min. to permit the addition of 10 to 15 residues of dCMP per end and then terminated with 2 µl of 0.25 M EDTA (pH 8.0) and 1 µl of 10% SDS. After extraction with 20 µl of phenol-CHCl₃, the aqueous phase was mixed with 20 µl of 4 M ammonium acetate, the DNA was precipitated and reprecipitated with 80 µl of ethanol, and the final pellet was rinsed with ethanol.

Step 3: HindIII endonuclease digestion. The pellet was dissolved in 30 µl of buffer containing 20 mM Tris.HCl pH 7.4, 7 mM MgCl₂, 60 mM NaCl, and 0.1 mg of BSA per ml and then digested with 10 U of HindIII endonuclease for 2 hr at 37°C. The reaction was terminated with 3 µl of 0.25 M EDTA (pH 8.0) and 1.5 µl of 10% SDS and, after extraction with phenol-CHCl₃ followed by the addition of 30 µl of 4 M ammonium acetate, the DNA was precipitated with 120 µl of ethanol. The pellet was rinsed with ethanol and then dissolved in 10 µl of 10 mM Tris.HCl (pH 7.3) and 1 mM EDTA, and 3 µl of ethanol was added to prevent freezing during storage at -20°C.

Step 4: Cyclization mediated by the oligo (dG)-tailed linker DNA. A 9 µl sample of the HindIII endonuclease-digested oligo (dC)-tailed cDNA:mRNA plasmid (about 90% of the sample) was incubated in a mixture (90 µl) containing 10 mM Tris.HCl pH 7.5, 1 mM EDTA, 0.1 M NaCl, and 1.8 pmol of the oligo (dG)-tailed linker DNA at 65°C for 5 min., shifted to 42°C for 60 min, and then cooled to 0°C. The mixture (90 µl) was adjusted to a volume of 900 µl containing 20 mM Tris.HCl pH 7.5, 4 mM MgCl₂, 10 mM (NH₄)₂SO₄, 0.1 M KCl, 50 µg of BSA per ml, and 0.1 mM β-NAD; 6 µg of E. coli DNA ligase were added and the solution was then incubated overnight at 12°C.

Step 5: Replacement of RNA strand by DNA. To replace the RNA strand of the insert, the ligation mixture was adjusted to contain 40 µM of each of the four deoxynucleoside triphosphates, 0.15 mM β-NAD, 4 µg of additional E. coli DNA ligase, 16 U of E. coli DNA polymerase I (PolI,) and 9U of E. coli RNase H. This mixture (960 µl) was incubated successively at 12°C and at room temperature for 1 hr each to promote optimal repair synthesis and nick translation by PolI.

### Step 6: Transformation of E. coli.

Transformation was carried out using minor modifications of the procedure described by Cohen et al. (Proc. Nat. Acad. Sci. U.S.A., 69: 2110-2114 [1972]). E. coli K-12 strain MC1061 (Casadaban, M. and Cohen, S., J. Mol. Biol. 138: 179-207 [1980]) was grown to 0.5 absorbancy unit at 600 nm at 37°C in 300 ml of L-broth. The cells were collected by centrifugation and suspended in 30 ml of 10 mM Pipes (pH 7), 60 mM CaCl₂, 15% glycerol, and centrifuged at 0°C for 5 min. The cells were resuspended in 24 ml of the above buffer and incubated again at 0°C for 5 min.; then 1.0 ml aliquots of the cell suspensions were mixed with 0.1 ml of the DNA solution (step 5) and incubated at 0°C for 20 min. Next the cells were kept at 42°C for 2 min. and thereafter at room temperature for 10 min.; then 1 liter of L-broth was added, and the culture was incubated at 37°C for 60 min. Ampicillin was added to a concentration of 50 µg/ml. The culture was shaken for an additional 10 hrs. at 37°C. Dilutions of this culture were spread on L-broth agar containing 50 µg/ml ampicillin. After incubation at 37°C for 12 to 24 hr, individual colonies were picked with sterile tooth-picks. In all, approximately 1 x 10⁵ independent cDNA clones were generated.

### D. Screening the pcD Library.

10⁴ single clones were picked at random from the T-cell cDNA library and propagated individually in wells of microtiter dishes containing 200 µl L-broth with ampicillin at 50 µg/ml and dimethyl sulfoxide at 7%. To focus only on the novel MCGF activity, 53 IL-3 cDNA clones and one GM-CSF cDNA clone were identified by hybridization with the appropriate ³²P-labelled cDNA probes constructed from the clones disclosed by Lee et al., Proc. Natl. Acad. Sci., Vol. 82, pgs. 4360-4364 (1985), and by Yokota et al., Proc. Natl. Acad. Sci., Vol. 81, pgs. 1070-1074 (1984), and were eliminated. This procedure was carried out as follows: Each plate of 96 cultures was replicated onto nitrocellulose filters for hybridization screening. Hybridizations were performed in 6XSSPE (1XSSPE = 180 mM NaCl; 10 mM sodium phosphate, pH 7.4; 1 mm EDTA), 0,1% SDS, 100 µg/ml E. coli tRNA, 50% formamide, for 16 hrs. at 42°C. Hybridizing clones were identified by autoradiography of the washed filter. These clones were removed by sterilizing the microtiter wells containing these clones with ethanol prior to the preparation of clone pools. Pools containing up to 48 cDNA clones were prepared from the microtiter cultures. Two hundred such pools were grown up in 1 liter cultures of L-broth containing 100 µg/ml ampicillin. Plasmid DNA was isolated from each culture and purified by twice banding through CsCl gradients. The DNA representing each pool was transfected into COS7 monkey cells as follows. (COS7 cells are described by Gluzman in Cell, Vol. 23, pgs. 175-180 (1981), and are available from the ATCC under accession number CRL 1651.)

One day prior to transfection, approximately 10⁶ COS 7 monkey cells were seeded onto individual 100 mm plates in DME containing 10% fetal calf serum and 2 mM glutamine. To perform the transfection, the medium was aspirated from each plate and replaced with 4 ml of DME containing 50 mM Tris.HCl pH 7.4, 400 µg/ml DEAE-Dextran and 50 µg of the plasmid DNAs to be tested. The plates were incubated for four hours at 37°C, then the DNA-containing medium was removed, and the plates were washed twice with 5 ml of serum-free DME. DME containing 150 µM Chloroquine was added back to the plates which were then incubated for an additional 3 hrs at 37°C. The plates were washed once with DME, and then DME containing 4% fetal calf serum, 2 mM glutamine, penicillin and streptomycin was added. The cells were then incubated for 72 hrs at 37°C. The growth medium was collected and evaluated in the various bioassays.

An initial set of plasmid pools was screened primarily by using proliferation assays for TCGF and MCGF activities with the HT-2 cell line (described more fully below) and MC/9 cell line, respectively. Among the first 110 pools assayed on these two cell lines, eight produced significant activity in the HT-2 TCGF assay. Several of these pools had weak but significant MCGF activity, but because the MCGF activities were generally weaker and more variable, we did not rely on this assay for identifying positive pools.

Approximately half of the COS supernatants from the random pool transfections were also assayed for Ia-inducing activity on mouse B cells. Among the pools tested, each pool shown to be active for TCGF activity was found also to have Ia-inducing activity. Thus, there was a perfect correlation between the TCGF activity and the Ia-inducing activity.

One pool, 2a, which was reproducibly the most active in all assays, was subdivided into 48 smaller subpools. Two subpools were positive for both MCGF and TCGF activities. The single clone, 2A-E3, common to both subpools was then grown individually and its plasmid DNA was transfected into COS 7 cells as described above. The resulting COS supernatant was then assayed for the presence of various activities, including MCGF, TCGF, Ia-inducing, and IgE- and IgG-enhancing activities.

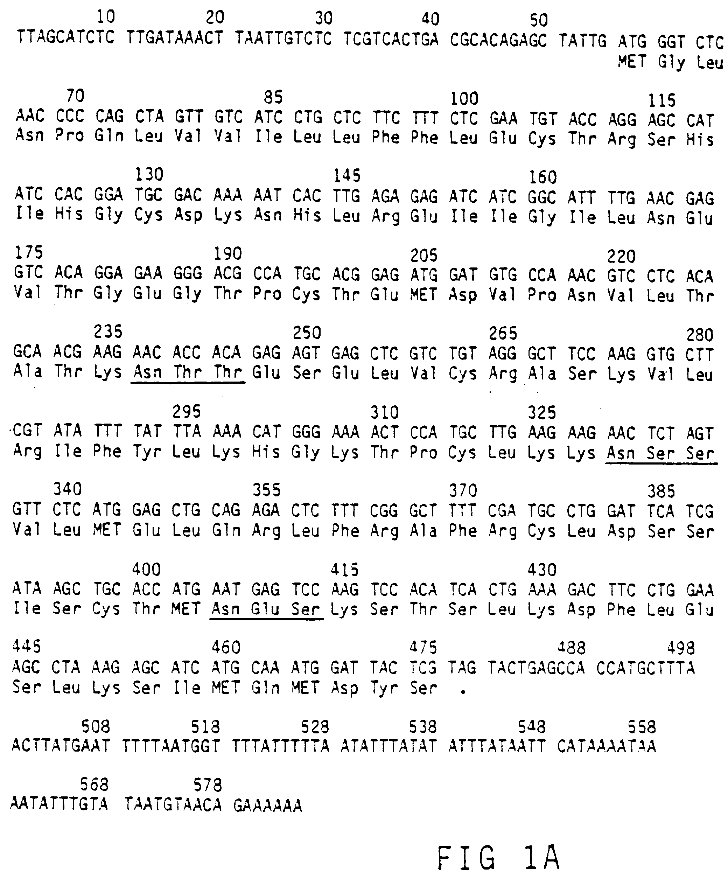

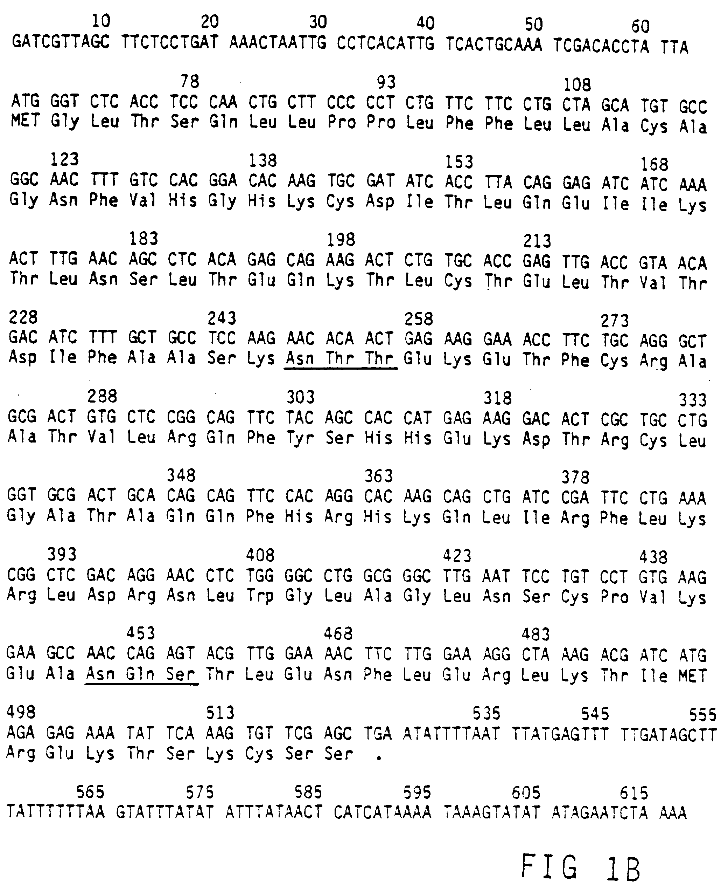

A 366 base-pair-long PstI fragment isolated from clone 2A-E3 (Figure 1A) and labelled with ³²P was used as a probe to screen pools which had been positive for biological activity as well as other untested pools. The screening was performed by hybridization to filters replicated with the microtiter cultures as described above. Nine hybridizing clones were isolated and their DNA analyzed by restriction mapping. All pools which exhibited biological activity contained at least one hybridizing clone which shared a common restriction cleavage map with clone 2A-E3. The frequency of hybridizing clones among the 10⁴ which were picked suggests a frequency of approximately 0.2% in the total library. Of the hybridizing clones which were tested, approximately 90% expressed a functional protein.

### E. Biological Activities of Culture Supernatants of COS 7 Monkey Cells Transfected with pcD-2A-E3.

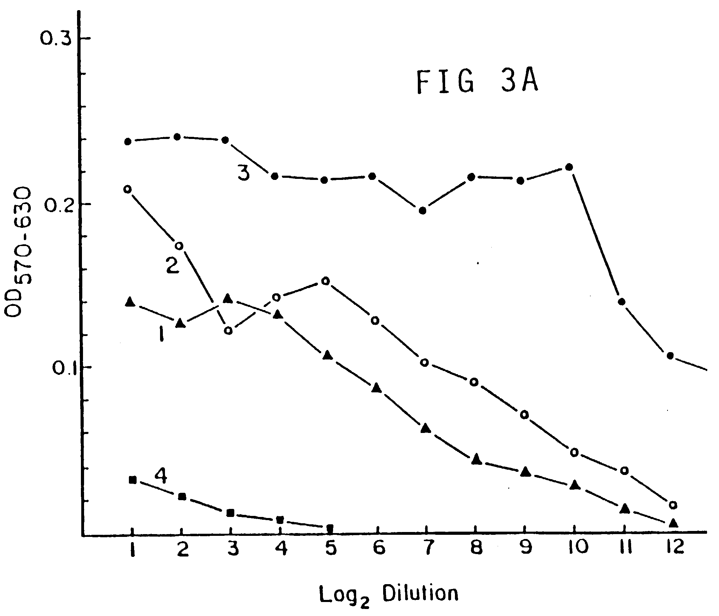

Supernatant from COS 7 cells transfected with pcD-2A-E3 was tested for TCGF activity on the murine helper T cell line HT-2, described by Watson in J. Exp. Med., Vol. 150, pg. 1510 (1979). Proliferation of the HT-2 cells, as determined by the colorimetric assay of Mosmann (cited above), was used as a measure of TCGF activity (degree of proliferation being correlated to optical density (OD) from 570 to 630 nm). Figure 3A illustrates the relative TCGF activities at various dilutions of (i) supernatant from COS 7 cells transfected with pcD-2A-E3 (curve 1), (ii) supernatant from C1.Ly1⁺2⁻/9 cultures (curve 2), (iii) supernatant from COS 7 cells transfected with a pcD plasmid carrying IL-2 cDNA (curve 3), and (iv) supernatant from COS 7 cells transfected with a pcD plasmid containing no cDNA insert (i.e. a "mock" transfection) (curve 4).

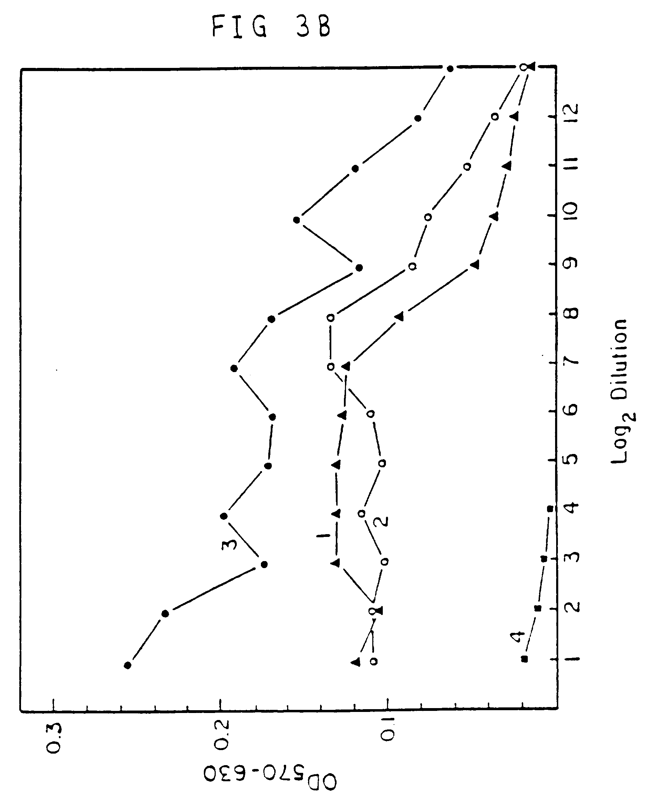

Similarly, supernatants from pcD-2A-E3 transfected COS 7 cells were tested for MCGF activity on MC/9 cells, again using the colorimetric assay of Mosmann to measure MC/9 proliferation. Figure 3B illustrates relative MCGF activity of (i) supernatant from COS 7 cells transfected with pcD-2A-E3 (curve 1), (ii) supernatant from COS 7 cells transfected with a pcD plasmid carrying IL-3 cDNA (curve 2), (iii) supernatant from C1.Ly1⁺2⁻/9 cells (curve 3), and (iv) supernatant from mock transfected COS 7 cells (curve 4).

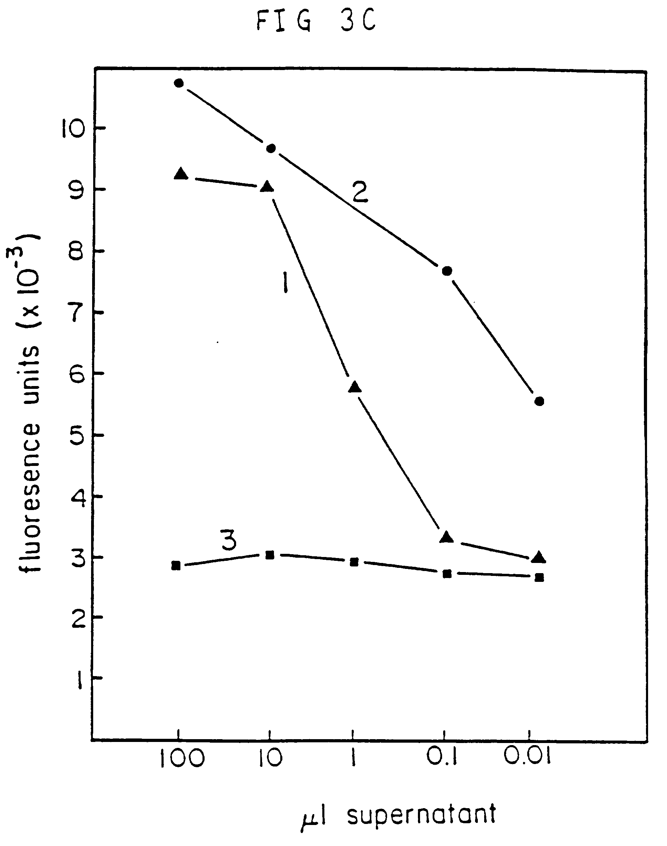

Figure 3C illustrates the results of an Ia induction assay conducted on (i) supernatant of COS 7 cells transfected with pcD-2-E3 (curve 1), (ii) supernatants of C1.Ly1⁺2⁻/9 cells (curve 2), and (iii) supernatants of mock transfected COS 7 cells (curve 3). The Ia-induction assay was carried out as described by Roehm et al. (cited above). Several DBA/2 mice (2-3 months old) were sacrificed and the spleens obtained surgically. The erythrocytes were lysed by hypotonic shock using 0.87% ammonium chloride. Then the T-cells were lysed by using cytotoxic monoclonal antibodies directed against T-cell-specific surface markers (Thy-1, Lyt-1 and Lyt-2) followed by incubation in rabbit complement. The dead cells were then removed using ficoll-hypaque density gradients. Adherent cells had been removed previously by adherence to plastic petri dishes at 37°C. At this time the cells were washed, counted and scored for viability. Approximately one million cells were incubated in 0.5 ml of tissue culture medium (RPMI 1640 or Minimal essential medium-MEM/Earle'S salts) (Gibco) supplemented with 10% fetal calf serum, 2-mercaptoethanol and various antibiotics (penicillin, streptomycin and gentamicin). In experiments where the positive control consisted of supernatants from T-cells induced with the T-cell mitogen Concanavalin A, 10 mg/ml (final concentration) of alpha-methyl-mannoside was added to neutralize the mitogen. After 24 hours' incubation, the cells were harvested and prepared for staining with anti-I-A^{d} or anti-I-A^{bd} monoclonal antibodies. These antibodies were used as first-stage antibodies conjugated to either the hapten N.I.P. or biotin. The staining was then completed by incubating the cells with fluoresceinated second-stage reagents (either anti-NIP antibodies or avidin). The intensity of fluorescence staining was then determined using either a fluorescence-activated cell sorter (Becton-Dickinson, Mountain View, CA) or a Cytofluorograph (Ortho Diagnostics, Cambridge, MA). Fluorescence units in Figure 3C are calculated by multiplying the percentage of positive cells in each sample by the intensity of fluorescent staining.

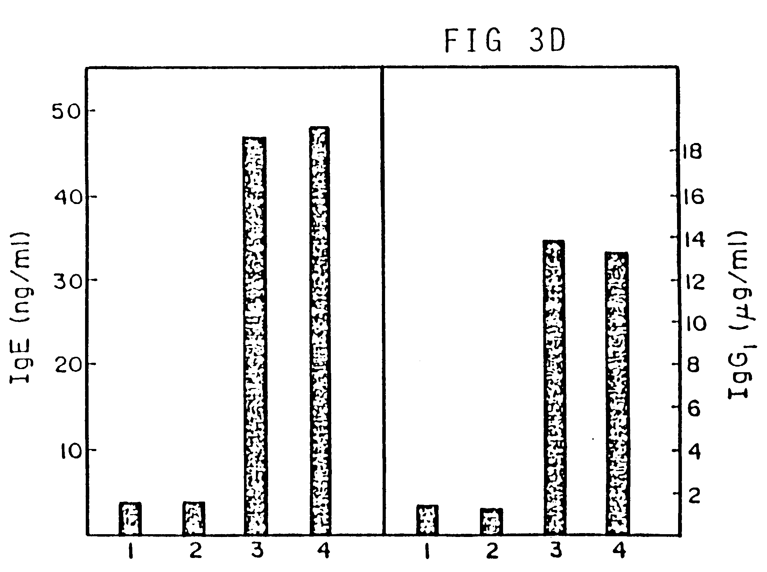

Figure 3D graphically illustrates the degrees by which IgE and IgG₁ production are induced in T-cell-depleted mouse spleen cells by (i) COS 7 medium alone (bar 1), (ii) 20% supernatant from mock transfected COS 7 cells (bar 2), (iii) 10% supernatant from C1.Ly1⁺2⁻/9 cells plus 20% supernatant from mock transfected COS 7 cells (bar 3), and (iv) 20% supernatant from pcD-2A-E3-transfected COS 7 cells (bar 4). Levels of IgE and IgG₁ were determined by the isotype-specific ELISA described above.

Murine IL-4 was found to enhance the MCGF activity of IL-3 in MC/9 cells: Smith and Rennick, Proc. Natl. Acad. Sci., Vol. 83, pgs. 1857-1861 (1986). Murine IL-4 was also found to enhance GM-CSF-stimulated proliferation of the IL-3-dependent cell line, NFS-60, described by Holmes et al., in Proc. Natl. Acad. Sci., Vol. 82, pgs. 6687-6691 (1985).

### F. Structure of pcD-2A-E3 and Nucleotide Sequence of Its cDNA Insert.

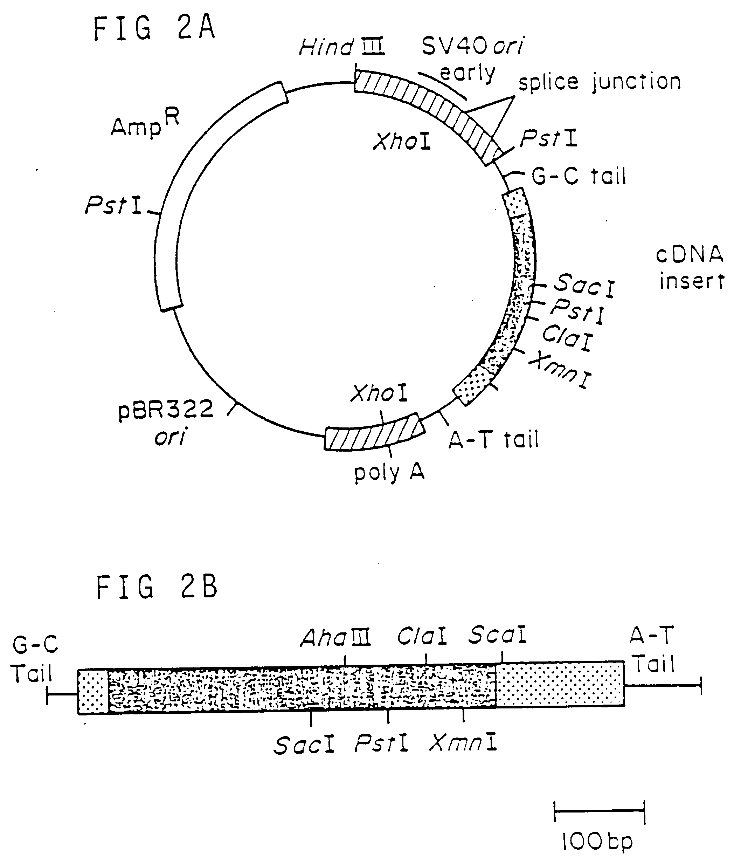

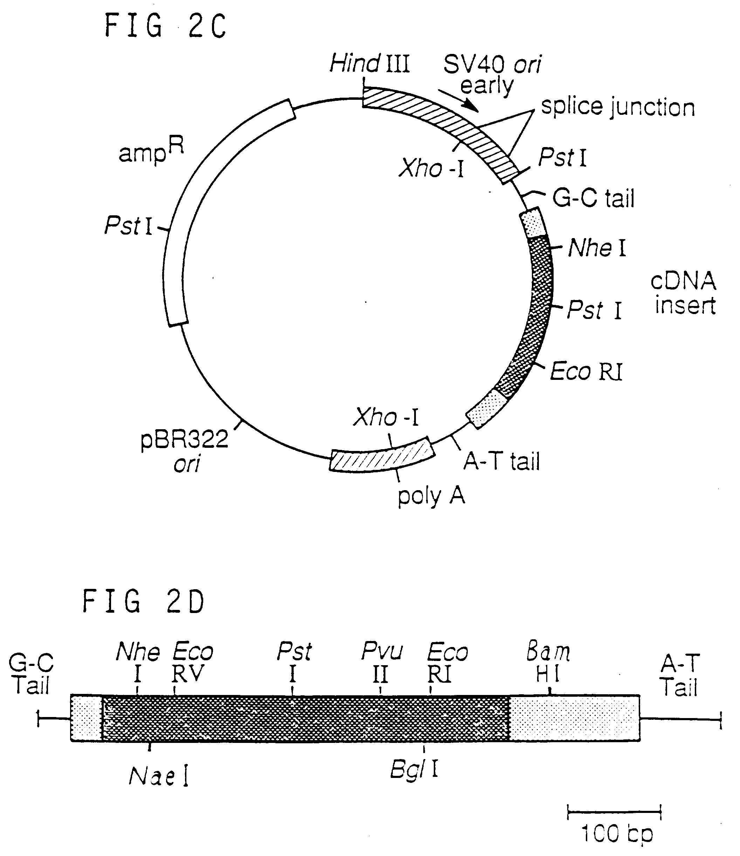

The structure of pcD-2A-E3 is illustrated diagramatically in Figure 2A, and an expanded restriction map of its insert is illustrated in Figure 2B. The insert was sequenced using both the Maxam and Gilbert approach (Methods in Enzymology, Vol. 65, pgs. 499-560 (1980)) and the Sanger approach (Proc. Natl. Acad. Sci., Vol. 74, pgs. 5463-5467 (1977)). The sequence is illustrated in Figure 1A, along with the deduced amino acid sequence for the longest open reading frame in-phase with the first ATG start codon. The single long open reading frame in the mouse 2A-E3 cDNA clone consists of 140 amino acid residues. Because this lymphokine is a secreted protein, a hydrophobic leader sequence would be expected to precede the sequence for the mature secreted form of the protein. Analysis of the hydrophobicity of the polypeptide and comparison with a proposed consensus sequence for the processing of signal peptides (Perlman et al., J. Mol. Biol., Vol. 167, pgs. 391-409 (1983)) suggest that cleavage of the precursor polypeptide would occur following the serine residue at amino acid position 20 in Figure 1A. Grabstein et al., J. Exp. Med., Vol. 163, pgs. 1405-1414 (1986), has confirmed that the N-terminal sequence of secreted murine IL-4 begins at the position 21 His of Fiqure 1A.

### Example II. Preparation of Human IL-4 Via a Murine cDNA Probe to a Human Helper T Cell cDNA Library and Transient Expression in COS 7 Monkey Cells and Mouse L Cells.

cDNA clones coding for IL-4 were isolated from cDNA libraries constructed from an induced human helper T cell, 2F1, and induced human peripheral blood lymphocytes (PBLs) by way of a murine cDNA probe. Other human cell lines known to produce BCGF activity include variants of the CEM line, available from the ATCC under accession numbers CCL 119, CRL 8436, and TIB 195, and described by Foley et al., in Cancer, Vol. 18, pgs. 522-529 (1965), and by Ligler, in Lymphokine Research, Vol. 3, pgs. 183-191 (1984).

A human helper T-cell clone, 2F1, and human PBLs were grown in Iscove's medium supplemented with 3% fetal calf serum. The 2F1 cells were activated with ConA (10 µg/ml) and PBLs were stimulated with 1 µg/ml PNA for 12 hours, after which ConA at 5 µg/ml was added. The cells were harvested 4 hr (2F1) or 10 hr (PBLs) after addition of ConA.

mRNA extraction and cDNA library construction were carried out as described in Example I. A PstI fragment was isolated from the mouse pcD-2A-E3 cDNA clone, labeled by nick translation (1 x 10⁸ cpm/µg) and used to probe nitrocellulose filters containing plasmid DNA preparations from ten pools, each representing approximately 1 x 10³ clones of 2F1 cDNA library. Low stringency hybridization conditions (overnight at 42°C) were used: 6xSSPE (1xSSPE=180 mM NaCl/10mM sodium phosphate, pH 7.4/1 mM EDTA) (Maniatis, T. et al., Molecular Cloning: A Laboratory Manual (Cold Spring Harbor Laboratory, N.Y., 1982)), 20% (vol/vol) formamide, 0.1% sodium dodecyl sulfate, yeast carrier tRNA at 100 µl. The filters were washed with 2 x SSPE, 0.1% sodium dodecyl sulfate at 37°C.

A single clone (pcD-46) was identified in one of the ten pools. Additional clones were obtained by screening the PBL cDNA libraries with a probe constructed from the NheI-EcoRI fragment of pcD-46 (illustrated in the restriction map of Figure 2D). Analysis by restriction enzymes indicated that the PBL clones were identical in structure to pcD-46.

It was discovered that a guanidine-rich region in the 5', or upstream, direction from the coding region insert of pcD-46 inhibited expression of the IL-4 polypeptide. Consequently, the insert of pcD-46 was recloned to remove the guanidine-rich region. The resulting clone is designated pcD-125. It was also discovered that expression was improved by transfecting mouse L cells with pcD-46.

The vector pcD-125 was formed as follows: pcD-46 was cleaved with Sau3A to isolate a fragment containing the 5' 162 nucleotides of the cDNA insert (eliminating the GC segment) and then the fragment was inserted into the BglII site of p101. The plasmid p101 was derived from pcD-mouse IL-3 (see, Yokota, T. et al., [1984] cited above) and is deleted for the sequence from the PstI site at the 5' end of the cDNA to a BglII site within the mouse IL-3 cDNA. A BglII site is included at the junction of the deleted sequence. The Sau3A fragment is fused to the SV40 promoter as in pcD-46, except for the GC segment. The remainder of the human cDNA was then reconstructed with a HindIII/NheI fragment from pcD-46 which carries the 3' end of the cDNA, the SV40 poly-A site and all of the pBR322 sequences of pcD-46.

Supernatants of the pcD-46- and pcD-125-transfected COS 7 and L cells were assayed for BCGF and TCGF activity. TCGF was assayed with a human helper T cell line JL-EBV transformed with Epstein-Barr virus, and phytohemagglutinin-stimulated human peripheral blood lymphocytes.

The human helper T-cell clone JL-EBV was stimulated with irradiated (4500R) cells of a human EBV-transformed B-cell line, and subsequently maintained in RPMI 1640 medium containing 10% human AB serum, 50 micromolar 2-mercaptoethanol (2ME) and recombinant human IL-2. Human PBLs were stimulated with PHA (20 microgram/ml) and maintained in RPMI 1640 containing 10% fetal calf serum, 50 micromolar 2ME and recombinant human IL-2. Five to ten days after stimulation, JL-EBV cells or PHA blasts were used as targets in a two-day TCGF assay using the Mosmann colorimetric method (described above), or in a three-day TCGF assay using [³H] thymidine incorporation.

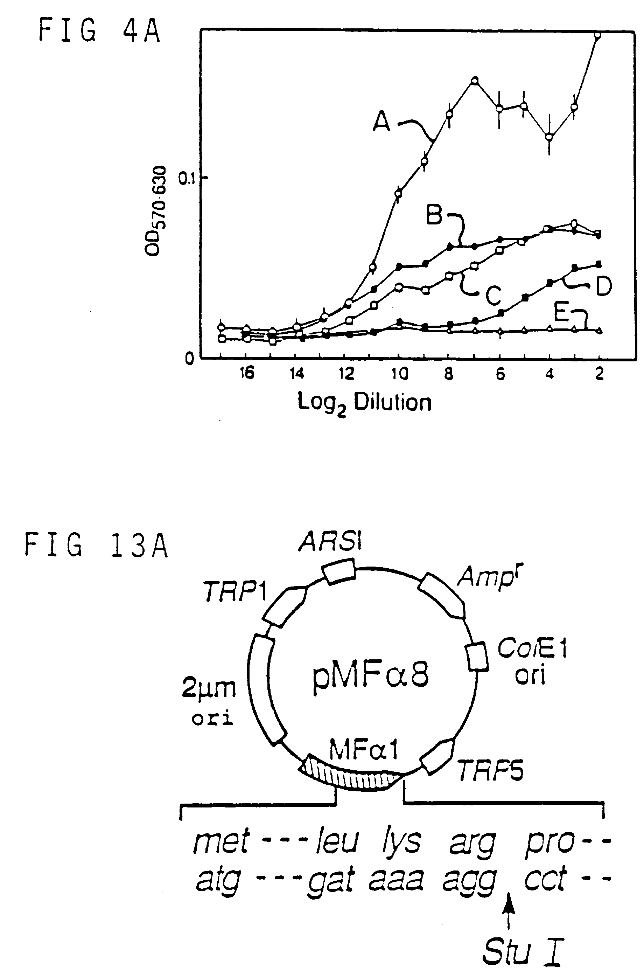

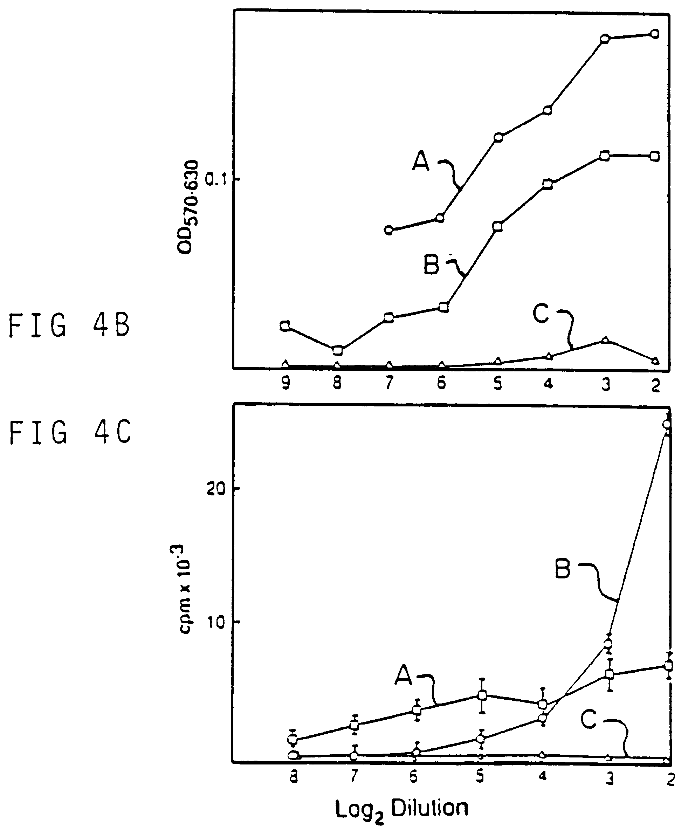

Figure 4A illustrates the TCGF activities measured by JL-EBV cells (colorimetric assay) of (i) supernatant from COS 7 cells transfected with pcD plasmids expressing human IL-2 (curve A); (ii) supernatant from L cells transfected with pcD-125 (curve B); (iii) supernatant from COS 7 cells transfected with pcD-125 (curve C), (iv) supernatant from COS 7 cells transfected with pcD-46 (curve D), and (v) supernatant from mock transfected COS 7 cells (curve E). Figure 4B illustrates the TCGF activities measured by PHA-stimulated PBLs (colorimetric assay) of (i) supernatant from COS 7 cells transfected with pcD plasmids expressing human IL-2 (curve A), (ii) supernatant from COS 7 cells transfected with pcD-125 (curve B), and (iii) supernatant from mock transfected COS 7 cells (curve C). Figure 4C illustrates the TCGF activities measured by PHA-stimulated PBLs (tritiated thymidine incorporation assay) of (i) supernatant from COS 7 cells transfected with pcD-125 (curve A), (ii) supernatant from COS 7 cells transfected with pcD plasmids expressing human IL-2 (curve B), and (iii) supernatant from mock transfected COS 7 cells (curve C).

BCGF activity of various dilutions of pcD-125 transfection supernatants were compared with the BCGF activity of a BCGF ("commercial BCGF") described by Maizel et al., Proc. Natl. Acad. Sci., Vol. 79, pgs. 5998-6002 (1982), and commercially available from Cytokine Technology International (Buffalo, NY). Table II illustrates the BCGF activities of various dilutions of COS 7 transfection supernatants on anti-IgM antibody preactivated B cells. B cells were prepared as described in the assay section above.

**Table II.**

| Effect of the IL-4 cDNA transfection supernatants on anti-IgM-preactivated B cells | | | | |
|---|---|---|---|---|
| % (vol/vol) of supernatants added | ³H-Thymidine Incorporation (cpm) | | | |
| | Mock-transfection | Clone 125 | Mock-transfection + 10 % BCGF | Clone 125 + 10 % BCGF |
| 0 | 278 | 278 | 1835 | 1835 |
| 0.2 | 189 | 144 | 1362 | 2303 |
| 1 | 323 | 1313 | 1699 | 3784 |
| 5 | 408 | 4314 | 1518 | 7921 |
| 15 | 397 | 4289 | 1093 | 8487 |

Table III illustrates the BCGF activities of various dilutions of COS 7 transfection supernatants on SAC-preactivated B cells (prepared as described above).

**Table III.**

| Activity of the IL-4 cDNA transfection supernatants on SAC-preactivated B cells | | | | |
|---|---|---|---|---|
| % (vol/vol) of supernatants added | ³H-Thymidine Incorporation (cpm) | | | |
| | Mock-transfection | Clone 125 | Mock-transfection + 10 % BCGF | Clone 125 + 10 % BCGF |
| 0 | 2237 | 2237 | 12,992 | 12,992 |
| 0.2 | 1789 | 2682 | 13,126 | 5,655 |
| 1 | 740 | 2374 | 13,714 | 6,765 |
| 5 | 1285 | 2826 | 5,848 | 10,023 |
| 15 | 1560 | 4701 | 10,128 | 10,924 |

Although the human IL-4 of the invention and commercial BCGF both display BCGF activity, Mehta et al., in J. Immunol., Vol. 135, pgs. 3298-3302 (1985), demonstrated that TCGF activity can be biochemically separated from the BCGF activity of commercial BCGF, indicating that the activities are caused by separate molecules. Thus, human IL-4 and commercial BCGF are different molecules because TCGF activity is inseparable from BCGF activity in human IL-4, using standard biochemical fractionation techniques.

Supernatants from COS-7 cells transfected with plasmids bearing the human IL-4 cDNA induce the proliferation of normal human T cells and the human T-cell clone JL-EBV, and activity that is similar to mouse IL-4. However, the maximum extent of proliferation of human T cells induced in response to human IL-4 is about half of that induced by human IL-2. The proliferation-inducing activity of IL-4 could not be inhibited by monoclonal antibodies against IL-2 or against the IL-2 receptor when tested. These results suggest that IL-4 acts directly on T cells and not by way of induction of IL-2 and that its activity is not mediated by the IL-2 receptor. The COS-human IL-4 supernatants also stimulate the proliferation of human B cells preactivated with optimal concentrations of anti-IgM antibodies coupled to beads and have additive proliferative capacity with commercial BCGF at saturation levels of the BCGF assay. This suggests that human IL-4 and commercial BCGF operate on B cells by different routes, e.g. possibly by different sets of receptors. The supernatants did not significantly induce proliferation of B cells preactivated with SAC, whereas commercial BCGF purified from supernatants of PBL cultures stimulated with PHA strongly induced the proliferation of SAC-preactivated human B cells. These results further indicate that the human IL-4 cDNA encodes a BCGF activity distinct from that present in the commercial BCGF.

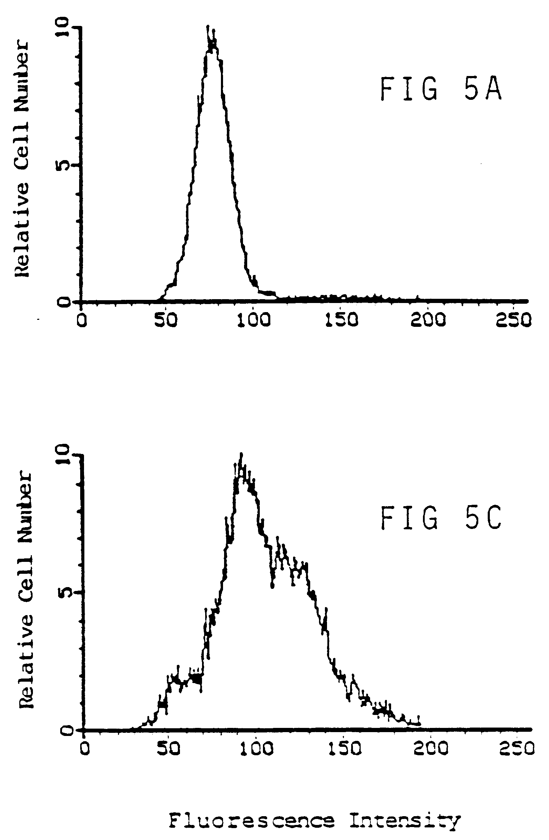

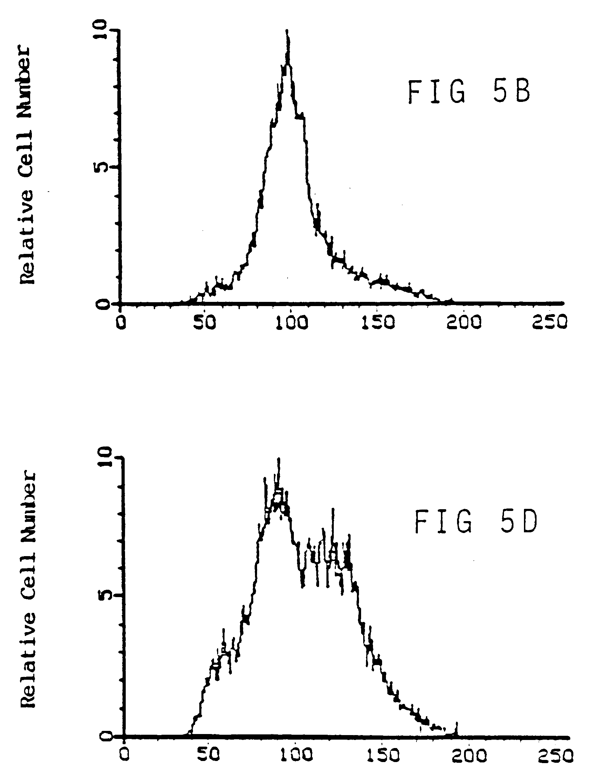

Supernatant of pcD-125-transfected COS 7 cells was also tested for its ability to induce Fc-epsilon receptors on tonsilar B cells. Human tonsil cells were dispersed into a single cell suspension using standard techniques. The B cell population was enriched using the protocol described above, and the enriched cells were stimulated with anti-IgM antibody for 24 hours in culture medium at 37°C. Fc-epsilon receptor-bearing cells were assayed by a Becton Dickinson FACS IV cell sorter using a fluorescently labeled monoclonal antibody specific for the receptor by means of the technique disclosed by Bonnefoy et al., in J. Immunol. Meth., Vol. 88, pgs. 25-32 (1986). Figures 5A-5D are histograms illustrating cell frequency (ordinate) versus fluorescent intensity (abscissa). Fluorescence intensity is proportional to the number of Fc-epsilon receptors present on a cell. In all the Figures the cells have been stimulated with anti-IgM. Figures 5A through 5D correspond to exposures to media consisting of 0%, 0.1%, 1%, and 10% supernatant from pcD-125-transfected COS 7 cells.

The DNA sequence of the cDNA insert of clone no. 46 was determined and is shown in Figure 1B. The cDNA insert is 615 bp long, excluding the poly(A) tail. There is a single open reading frame, with the first ATG codon located at 64 nucleotides from the 5' end followed by 153 codons ending with the termination codon TAG at nucleotide positions 523-525. The N-terminal segment of the predicted polypeptide is hydrophobic, as would be expected for a secreted protein.

A comparison between the coding regions of a human and a mouse cDNA of the present invention revealed that the regions of the human cDNA coding sequence in pcD-46 covered by amino acid positions 1-90 and 129-149 share approximately 50% homology with the corresponding regions of the mouse cDNA (2A-E3) coding sequence. These regions, and 5' and 3' untranslated regions, share about 70% homology between the two cDNA sequences from the different species, whereas the region covered by amino acids 91-128 of the human protein shares very limited homology with the corresponding mouse region. In all, six of the seven cysteine residues in the human protein are conserved in the related mouse protein. Some amino acid sequence homology exists between a native form of a human polypeptide of the present invention and mouse IL-3. Amino acid residues 7-16 and 120-127 are 50% and 55% homologous, respectively, to residues 16-27 and 41-49 of the mouse IL-3 precursor polypeptide (Yokota, T. et al., Proc. Natl. Acad. Sci. U.S.A. 81: 1070-1074 [1984]).

As described more fully below, human IL-4 purified from pcD-125-transfected COS 7 supernatants was found to be the 129 amino acid polypeptide having the sequence illustrated by Figure 1C.

### Example III. Enhanced Expression of Human IL-4 in COS 7 Monkey cells by Using an Epstein-Barr Virus (EBV)-Derived Vector Containing an RSV-LTR Promoter.

A 10-20 fold enhancement of human IL-4 expression was obtained by recloning the XhoI fragment of pcD-125 into an EBV-derived vector containing a Rous sarcoma virus long-terminal repeat (RSV-LTR) promoter. The EBV-derived vector and the RSV-LTR promoter are described by Gorman et al., Proc. Nat. Acad. Sci., Vol. 79, pgs. 6777-6781 (1982); and Yates et al., Nature, Vol. 313, pgs. 812-815 (1985).

A HindIII/XhoI fragment containing the RSV-LTR promoter was isolated from a pcD plasmid previously constructed from the RSV-LTR containing AccI/HindIII fragment described by Gorman et al. (cited above) and a commercially available pcD vector (e.g. Pharmacia). The above HindIII/XhoI fragment and a HindIII/XhoI fragment from a pL1 plasmid (Pharmacia) containing an SV40 origin-of -replication (ori) are spliced into plasmid pcDV1 (available from Pharmacia), the orientation of the SV40 ori region not being critical. Between an AatII site and an NdeI site, the resulting pcD vector contains in sequence (from the AatII site) an SV40 ori region, an RSV-LTR promoter, and the SV40 poly A region. After the XhoI fragment of pcD-125 is isolated and inserted into the xhoI site of the newly constructed pcD vector, the unique AatII and NdeI sites on the vector are converted into SalI sites using standard techniques. Briefly, the pcD vector is digested with AatII and NdeI, the IL-4 containing fragment is isolated, and the isolated fragment is treated with T4 DNA polymerase in the presence of appropriate concentrations of the nucleoside triphosphates. The 5'→ 3' DNA polymerase activity of T4 DNA polymerase fills in the 5' protruding ends of the restriction cuts, and the 3'→ 5' exonuclease activity of T4 DNA polymerase digests the 3' protruding ends of the restriction cuts to leave a blunt-ended fragment, to which kinased SalI linkers (New England Biolabs) are ligated using T4 DNA ligase.

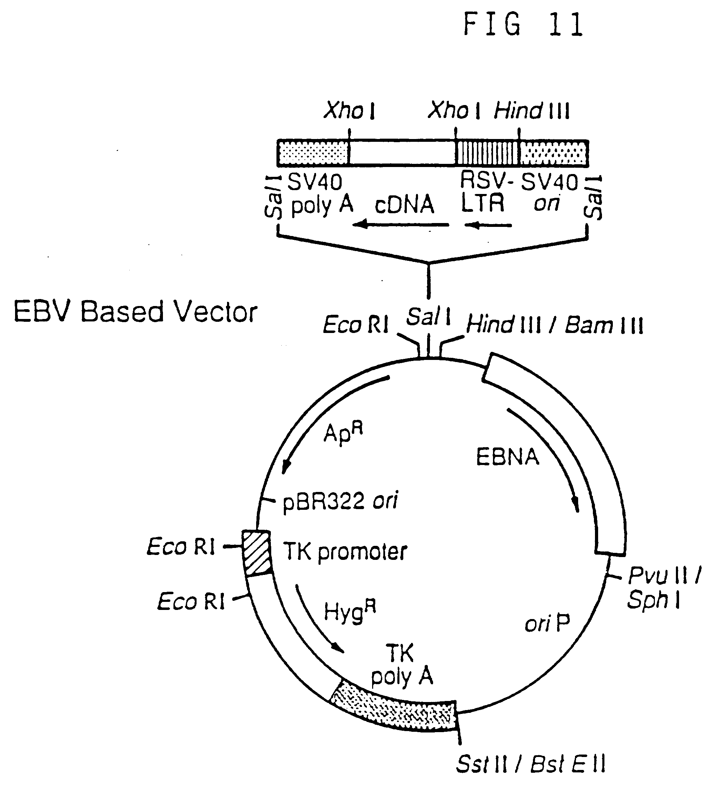

The above SalI fragment (illustrated in Figure 11) is inserted in the EBV-derived vector p201 described by Yates et al. (cited above) at the location of a unique ClaI site, which had been converted to a SalI site using standard techniques. Briefly, p201 (illustrated in Figure 11) is digested with ClaI and treated with DNA polymerase I (Klenow fragment) and appropriate concentrations of nucleoside triphosphates. This procedure fills in the protruding ends of the ClaI cut to leave a blunt-ended fragment. Next, the blunt ends are ligated to a kinased SalI linker. The resulting EBV-derived vector containing the RSV-LTR promoter and human IL-4 cDNA insert is referred to herein as pEBV-178.

pEBV-178 was transfected into COS 7 cells using standard techniques and the culture supernatants were assayed for TCGF activity as a measure of IL-4 expression.

### Example IV. Expression of Native Human IL-4 and Mutein IS⁰ (Ala-Glu-Phe) in E. coli

Two vectors containing human IL-4 cDNA inserts were constructed for expression of human IL-4 in E. coli: a pIN-III secretion vector which contains the signal peptide sequence of the ompA protein ("pIN-III-ompA2"), and a pUC12 plasmid containing a trpP promoter and an adjacent ribosome binding site (RBS) region ("TRPC11").

### A. pIN-III-ompA2

Two vectors were constructed using the pIN-III-ompA2 plasmid, which is described by Ghrayeb et al., in EMBO Journal, Vol. 3, pgs. 2437-2442 (1984), and Masui et al., in Biotechnology, Vol. 2, pgs. 81-85 (1984).

The first vector, designated pIN-III-ompA2(1), was constructed by ligating, in series, the EcoRI/BamHI-digested pIN-III-ompA2 plasmid, a synthetic linker, and the BamHI/EcoRV fragment of pcD-125. The synthetic linker used in this construction resulted in the secretion of a biologically active IL-4 polypeptide having the three extra N-terminal amino acids Ala-Glu-Phe-; i.e. mutein IL⁰(Ala-Glu-Phe) was secreted. The synthetic linker consisted of the following sequences of nucleotides:

AA TTC CAC AAG TGC GAT
G GTG TTC ACG CTA

EcoRI/BamHI-digested pIN-III-ompa2 and the BamHI/EcoRV fragment of pcD-125 were mixed in a standard ligation solution (e.g. Maniatis et al., cited above) containing 0.1 micromolar of the synthetic linker. E. coli strain Ab1899 was infected by the pIN-III-ompA2(1) plasmid and transformants were selected by colony hybridization using a ³²P-labeled IL-4 cDNA probe. Human IL-4 extracts for assaying were obtained as follows. After sonication, the bacterial cultures were centrifuged, and the supernatant removed from the pellet. The pellet was treated with 1% SDS, 2mM dithiothreitol, and 6M guanidine. The material was recentrifuged, the supernatant discarded, and the pellet treated at 45°C with 3% SDS and 2mM dithiothreitol. The material was again centrifuged, and the supernatant assayed by SDS-PAGE.

pIN-III-ompA(2) was constructed so that the native human IL-4 would be expressed. The three amino acid addition in the PIN-III-ompA(1) construction was eliminated by site-specific mutagenesis of the ompA signal peptide sequence of pIN-III-ompA2. The site-specific mutagenesis was carried out as disclosed by Zoller and Smith (cited above). Briefly, the XbaI/BamHI fragment of pIN-III-ompA2 containing the coding sequence for the ompA signal peptide (see Fig. 1 in Ghrayeb et al., cited above) was purified, mixed with purified XbaI/BamHI-digested replicating form (RF) of M13mp19, ligated, transfected into E. coli K-12 JM101, and plated. A clear plaque in the presence of IPTG and X-gal was selected and propagated, and single stranded DNAs were prepared, e.g. according to the procedures disclosed by Messing in Methods in Enzymology, Vol. 101 (Academic Press, New York, 1983). Separately, the following oligonucleotide primer (23-mer) containing the indicated base substitutions (boxed) was synthesized and phosphorylated: 5' - GGAATTCAGAAGCT TG C G GCTAC - 3'. This sequence introduces a second HindIII site in the signal peptide coding region of the mutated pIN-III-ompA2. The oligonucleotide primer was annealed to the M13mp19 RF containing the XbaI/BamHI fragment of pIN-III-ompA2, and treated with DNA polymerase in the presence of appropriate concentrations of nucleoside triphosphates. The resulting RFs were used to transfect JM101 E. coli, and mutant-containing plaques were screened by a labeled oligonucleotide probe. The sequence of the selected RF was confirmed by dideoxy sequencing using a universal M13 primer. The selected RF was propagated, isolated, and digested with XbaI and BamHI, and the purified XbaI/BamHI fragment was inserted into an XbaI/BamHI-digested pIN-III-ompA2. To form pIN-III-ompA2(2), the mutant pIN-III-ompA2 was propagated, purified, digested with HindIII and BamHI, and mixed with the BamHI/EcoRV fragment of pcD-125 in a standard ligation solution containing 0.1 micromolar of the following synthetic linker: 
A GCT CAC AAG TGC GAT
GTG TTC ACG CTA
 E. coli strain Ab1899 was infected by the pIN-III-ompA2(2) plasmid and transformants were selected by colony hybridization using a ³²P-labeled IL-4 cDNA probe. IL-4 extracts, prepared as described above, exhibited TCGF activity comparable to supernatants of pcD-125 COS7 cells.

### B. TRPC11

The TRPC11 vector was constructed by ligating a synthetic consensus RBS fragment to ClaI linkers (ATGCAT) and by cloning the resulting fragments into ClaI-restricted pMT11hc (which had been previously modified to contain the ClaI site). pMT11hc is a small (2.3 kilobase) high copy, AMP^{R}, TET^{S} derivative of pBR322 that bears the EcoRI-HindIII polylinker region of the πVX plasmid (described by Maniatis et al., cited above). It was modified to contain the ClaI site by restricting pMT11hc with EcoRI and BamHI, filling in the resulting sticky ends and ligating with ClaI linker (CATCGATG), thereby restoring the EcoRI and BamHI sites and replacing the SmaI site with a ClaI site.

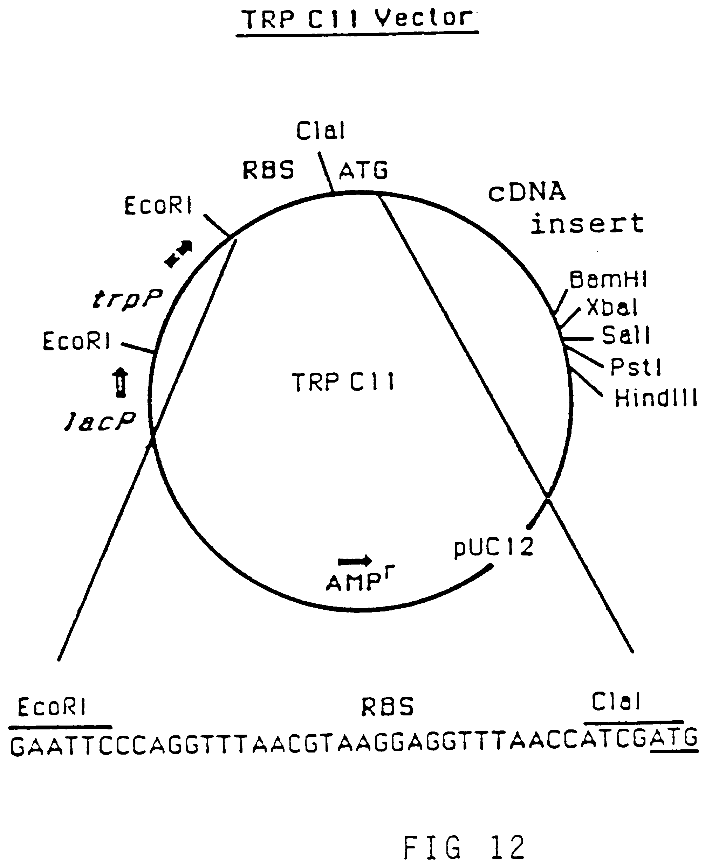

One transformant from the TRPCII construction had a tandem RBS sequence flanked by ClaI sites. One of the ClaI sites and part of the second copy of the RBS sequence were removed by digesting this plasmid with PstI, treating with Bal31 nuclease, restricting with EcoRI and treating with T4 DNA polymerase in the presence of all four deoxynucleotide triphosphates. The resulting 30-40 bp fragments were recovered via PAGE and cloned into SmaI-restricted pUC12. A 248 by E. coli trpP-bearing EcoRI fragment derived from pKC101 (described by Nichols et al. in Methods in Enzymology, Vol. 101, pg. 155 (Academic Press, N.Y. 1983)) was then cloned into the EcoRI site to complete the TRPC11 construction, which is illustrated in Figure 12.

TRPC11 was employed as a vector for human IL-4 cDNA by first digesting it with ClaI and BamHI, purifying it, and then mixing it with the EcoRV/BamHI fragment of pcD-125 in a standard ligation solution containing 0.1 micromolar of the following synthetic linker: 
TCG ATG CAC AAG TGC GAT
AC GTG TTC ACG CTA
 The insert-containing vector was selected as described above and propagated in E. coli K-12 strain JM101. IL-4 was extracted as follows. JM101 cells were sonicated in their culture medium and centrifuged. The pellet was resuspended in 4M guanidine and 2mM dithiothreitol, and again centrifuged. The supernatant was tested for biological activity and found to exhibit TCGF activity comparable to that of supernatants of pcD-125-transfected COS7 cells.

### Example V. Preparation of Bovine IL-4 cDNAs Via Mouse and Human IL-4 cDNA Probes to a Bovine Helper T Cell cDNA Library and Transient Expression in COS 7 Monkey Cells.

cDNA clones coding for IL-4 are isolated from cDNA libraries constructed from induced bovine peripheral blood lymphocytes (PBLs) by way of combined mouse and human cDNA probes. Alternative sources of bovine cDNAS include several bovine cell lines maintained in the ATCC's NBL animal line collection. Procedures are substantially identical to those described in Example II. Cells are harvested about 10 hours after induction by ConA. mRNA extraction and cDNA library construction are carried out as in Example II.

The mouse and human cDNA probes can be used together as a mixture or sequentially to detect bovine IL-4 cDNAs. As in Example II, the PstI fragment is isolated from the mouse pcD-2A-E3 cDNA clone. Likewise the PstI fragment is isolated from the human pcD-125 cDNA clone. Several other fragments are also available to construct probes from. Either together or separately the isolated PstI fragments are labeled by nick translation (about 1 x 10⁸ cpm/microgram) and are used to probe nitrocellulose filters containing plasmid DNA preparations from 10 pools, each representing about 1000 clones of the induced PBL cDNA library. Filter hybridization is carried out as in Example II. Positive scoring clones are identified and propagated.

### Example VI. Expression of Native Human IL-4 and Muteins Δ¹⁻⁴ and IS⁰(Gly-Asn-Phe-Val-His-Gly) in Saccharomyces cerevisiae

Native human IL-4 cDNA and two mutants thereof were cloned into the plasmid pMF-alpha8 and expressed in the yeast Saccharomyces cerevisiae. The construction and application of pMF-alpha8 for expressing non-yeast proteins is described fully in Miyajima et al., Gene, Vol. 37, pqs. 155-161 (1985); and Miyajima et al., EMBO Journal, Vol. 5, pgs, 1193-1197 (1986). pMF-alpha8 is deposited with the American Type Culture Collection (Rockville, MD) under accession number 40140, and a map of the plasmid is given in Figure 13A (designations in the figure are defined fully in Miyajima et al., Gene, cited above).

### A. Human IL-4 Mutein Δ¹⁻⁴.

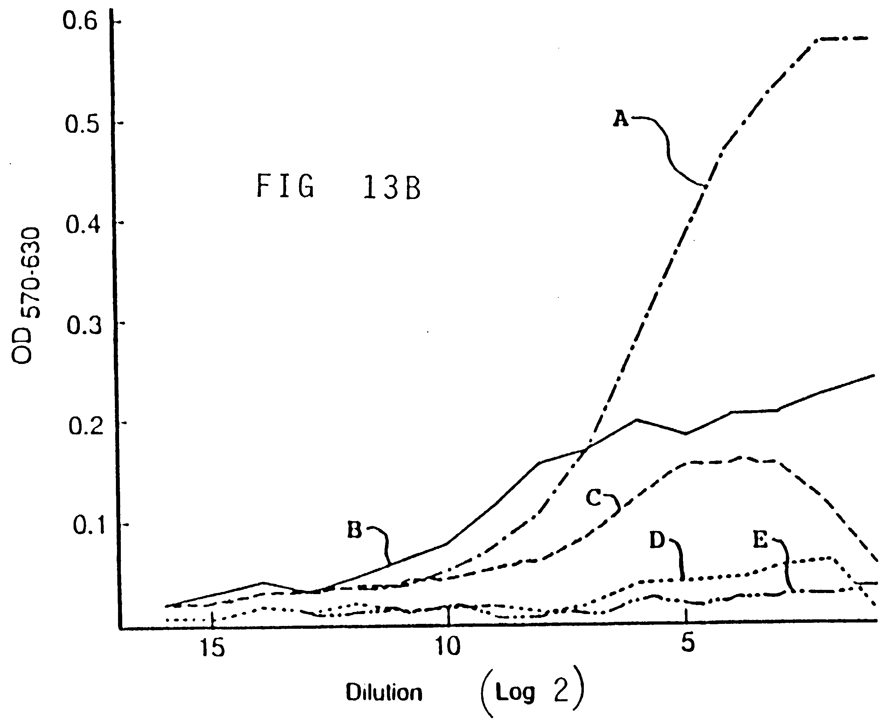

Plasmid pcD-125 was isolated and digested with EcoRV and BamHI. The EcoRV/BamHI fragment containing the human IL-4 cDNA was isolated, treated with DNA polymerase I (Klenow fragment) to fill in the BamRI cut, and kinased (i.e. phosphorylated). pMF-alpha8 was digested with StuI and combined with the kinased EcoRV/BamHI fragment of pcD-125 in a standard ligation solution to form plasmid phIL-4-2. phIL-4-2 was used to transform S. cerevisiae 20B-12 (MATalpha trpl-289 pep4-3), which was obtained from the Yeast Genetic Stock Center, University of California, Berkeley. Yeast cells were grown in synthetic medium containing 0.67% Yeast Nitrogen Base without amino acids, 2% glucose, and 0.5% Casamino acids (Difco). The yeast cells were transformed with the plasmids by the lithium acetate method of Ito et al., J. Bacteriol., Vol. 153, pgs. 163-168 (1983), and transformants were selected in synthetic medium lacking tryptophan. Supernatant of a transformant culture was tested for TCGF activity. Figure 13B (curve D) illustrates the TCGF activity of several dilutions of the supernatant from phIL-4-2-transformed yeast cells in comparison with other factors (Curve A -- human IL-2; Curve B -- supernatant from pcD-125-transfected COS 7 cells; and Curve C -- supernatants from phIL-4-1-transformed yeast cells). Curve E illustrates the TCGF activity of supernatant from yeast that had been transformed with pMF-alpha8 lacking the IL-4 cDNA insert, i.e. the "mock" transformant.

### B. Human IL-4 Mutein IS⁰(Gly-Asn-Phe-Val-His-Gly).

The pMF-alpha8 insert for expression of mutein IL⁰(Gly-Asn-Phe-Val-His-Gly) was prepared exactly as for mutein Δ¹⁻⁴, except that the NaeI/BamHI fragment from pcD-125 was used. The resulting plasmid was designated phIL-4-1. Several dilutions of supernatant from phIL-4-l-transformed yeast cells were tested for TCGF activity. The results are illustrated by Curve C of Figure 13B. The supernatants were also tested for BCGF activity on both anti-IgM and SAC-activated B lymphocytes. The assays were performed as described above, and the results are given in Table IV.

**Table IV.**

| BCGF Activity of Supernatants of phIL-4-3 Transformed Yeast Cells | | |
|---|---|---|
| % (vol/vol) of supernatants added | [³H]-Thymidine Incorporation (cpm) | |
| | SAC-activated B Lymphocytes | Anti-IgM Bead Activated B Lymphocytes |
| .0 | 3633 ± 1239 | 641 ± 69 |
| 0.09 | 7610 ± 310 | 13221 ± 472 |
| 0.19 | 9235 ± 181 | ----------- |
| 0.39 | 10639 ± 786 | 16681 ± 310 |
| 0.78 | 10372 ± 572 | 18090 ± 1248 |
| 1.56 | 9905 ± 328 | 17631 ± 1216 |
| 3.12 | 11354 ± 836 | 18766 ± 1179 |
| 6.25 | 10481 ± 541 | 19810 ± 1349 |
| 12.5 | 9641 ± 30 | 18136 ± 1126 |
| 25. | 8253 ± 857 | 14750 ± 1125 |

### C. Expression of Native Human IL-4 in Yeast.

cDNA coding for native human IL-4 was cloned into pMF-alpha8 by first inserting bases upstream of the N-terminal His codon to form a KpnI restriction site. After cleavage by KpnI and treatment by DNA polymeraseI, the blunt-ended IL-4 cDNA was inserted into the StuI site of pMF-alpha8. The KpnI site was formed by use of standard site-specific mutagenesis. Briefly, pcD-125 was digested with BamHI, and the fragment containing the entire human IL-4 cDNA was isolated and inserted into the BamHI site of M13mp8. Single-stranded M13mp8 containing the insert was isolated and hybridized to the following synthetic oligonucleotide which served as a primer: 5' - TCCACGGA GGTAC CACAAGTG - 3' The inserted nucleotides are boxed. The plasmid containing the mutated IL-4 cDNA was identified by an oligonucleotide probe, propagated, isolated, and treated with KpnI and BamHI. The KpnI/BamHI fragment was isolated, treated with DNA polymerase I (Klenow fragment) to generate blunt ends, kinased, and ligated with StuI-digested pMF-alpha8. Yeast was transformed by the resulting pMF-alpha8 plasmids, designated phIL-4-3, as described above, and supernatants were tested for TCGF activity. The supernatants displayed TCGF activity comparable to that observed for supernatants of phIL-4-1-transformed yeast.

### Example VII. Construction and Expression of a Synthetic Human IL-4 Gene in E. coli

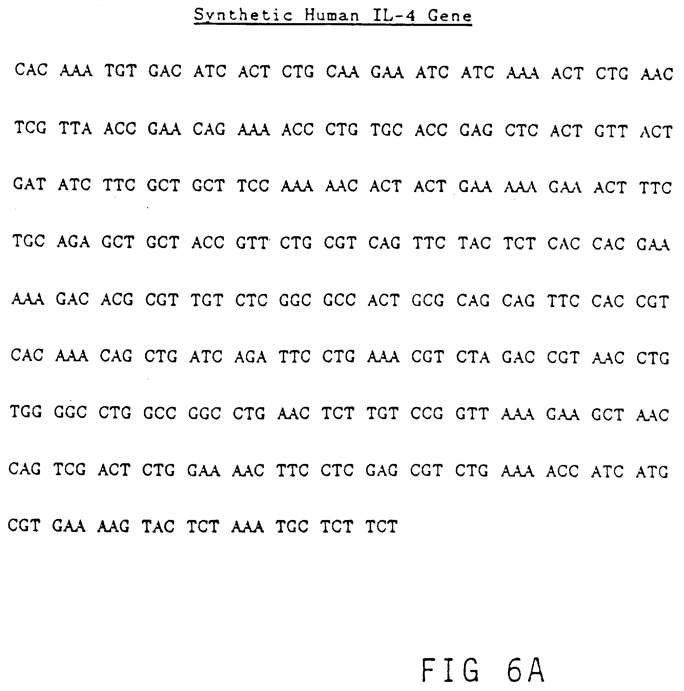

A synthetic human IL-4 gene is constructed which substantially comprises bacterial-preferred codons, includes a series of unique restriction endonuclease sites (referred to herein as "unique restriction sites"), and permits rapid and convenient expression of a wide variety of human IL-4 muteins. The nucleotide sequence of the synthetic gene is illustrated in Figure 6A. Techniques for constructing and expressing the synthetic gene of this example are standard in the art of molecular biology, e.g. especially Sproat and Gait, Nucleic Acids Research, Vol. 13, pgs. 2959-2977 (1985); and Ferretti et al., Proc. Natl. Acad. Sci., Vol. 83, pgs. 599-603 (1986); but also Mullenbach et al., J. Biol. Chem., Vol. 261, pgs. 719-722 (1986); Wells et al., Gene, Vol. 34, pgs. 315-323 (1985); and Estell et al., Science, Vol. 233, pgs. 659-663 (1986). Briefly, the synthetic human IL-4 gene is assembled from a plurality of chemically synthesized double-stranded DNA fragments. Base sequences of the synthetic gene are selected so that the assembled synthetic gene contains a series of unique restriction sites.

The series of unique restriction sites defines a series of segments which can be readily excised and replaced with segments having altered base sequences. The synthetic fragments are inserted either directly or after ligation with other fragments into a suitable vector, such as a pUC plasmid, or the like. The above-mentioned segments roughly correspond to the "cassettes" of Wells et al. (cited above). The synthetic fragments are synthesized using standard techniques, e.g. Gait, Oligonucleotide Synthesis: A Practical Approach (IRL Press, Oxford, UK, 1984). Preferably an automated synthesizer is employed, such as an Applied Biosystems, Inc. (Foster City, CA) model 380A. pUC plasmids and like vectors are commercially available, e.g. Pharmacia-PL, or Boehringer-Mannheim. Cloning and expression can be carried out in standard bacterial systems, for example E. coli K-12 strain JM101, JM103, or the like, described by Viera and Messing, in Gene, Vol. 19, pgs. 259-268 (1982).

Restriction endonuclease digestions and ligase reactions are performed using standard protocols, e.g. Maniatis et al., Molecular Cloning: A Laboratory Manual (Cold Spring Harbor Laboratory, New York, 1982).

The alkaline method (Maniatis et al., cited above) is used for small-scale plasmid preparations. For large-scale preparations a modification of the alkaline method is used in which an equal volume of isopropanol is used to precipitate nucleic acids from the cleared lysate. Precipitation with cold 2.5 M ammonium acetate is used to remove RNA prior to cesium chloride equilibrium density centrifugation and detection with ethidium bromide.

For filter hybridizations Whatman 540 filter circles are used to lift colonies which are then lysed and fixed by successive treatments with 0.5M NaOH, 1.5M NaCl; 1M Tris.HCl pH8.0, 1.5M NaCl (2 min each); and heating at 80°C (30 min). Hybridizations are in 6xSSPE, 20% formamide, 0.1% sodium dodecylsulphate (SDS), 100 µg/ml E. coli tRNA, 100 µg/ml Coomassie Brilliant Blue G-250 (Biorad) at 42°C for 6 hrs using ³²P-labelled (kinased) synthetic DNAs. (20xSSPE is prepared by dissolving 174 g of NaCl, 27.6 g of NaH₂PO₄·H₂O, and 7.4 g of EDTA in 800 ml of H₂O, adjusting pH to 7.4 with NaOH, adjusting volume to 1 liter, and sterilizing by autoclaving.)

Filters are washed twice (15 min, room temperature) with 1xSSPE, 0.1% SDS. After autoradiography (Fuji RX film), positive colonies are located by aligning the regrown colonies with the blue-stained colonies on the filters.

DNA is sequenced by either the chemical degradation method of Maxam and Gilbert, Methods in Enzymology, Vol. 65, pg. 499 (1980), or the dideoxy method of Sanger et al. Proc. Natl. Acad. Sci., Vol. 74, pg. 5463 (1977). Templates for the dideoxy reactions are either single-stranded DNAs of relevant regions recloned into M13mp vectors, e.g. Messing et al. Nucleic Acids Res., Vol. 9, pg. 309 (1981), or double-stranded DNA prepared by the minialkaline method and denatured with 0.2M NaOH (5 min, room temperature) and precipitated from 0.2M NaOH, 1.43M ammonium acetate by the addition of 2 volumes of ethanol. Dideoxy reactions are done at 42°C.

DNA is synthesized by phosphoramidite chemistry using Applied Biosystems 380A synthesizers. Synthesis, deprotection, cleavage and purification (7M urea PAGE, elution, DEAE-cellulose chromatography) are done as described in the 380A synthesizer manual. Complementary strands of synthetic DNAs to be cloned (400ng each) are mixed and phosphorylated with polynucleotide kinase in a reaction volume of 50 µl. This DNA is ligated with 1 µg of vector DNA digested with appropriate restriction enzymes, and ligations are done in a volume of 50 µl at room temperature for 4 to 12 hours. Conditions for phosphorylation, restriction enzyme digestions, polymerase reactions, and ligation have been described (Maniatis et al., cited above). Colonies are scored for lacZ⁺ (when desired) by plating on L agar supplemented with ampicillin, isopropyl-1-thio-beta-D-galactoside (IPTG) (0.4 mM) and 5-bromo-4-chloro-3-indolyl-beta-D-galactopyranoside (x-gal) (40 µg/ml).

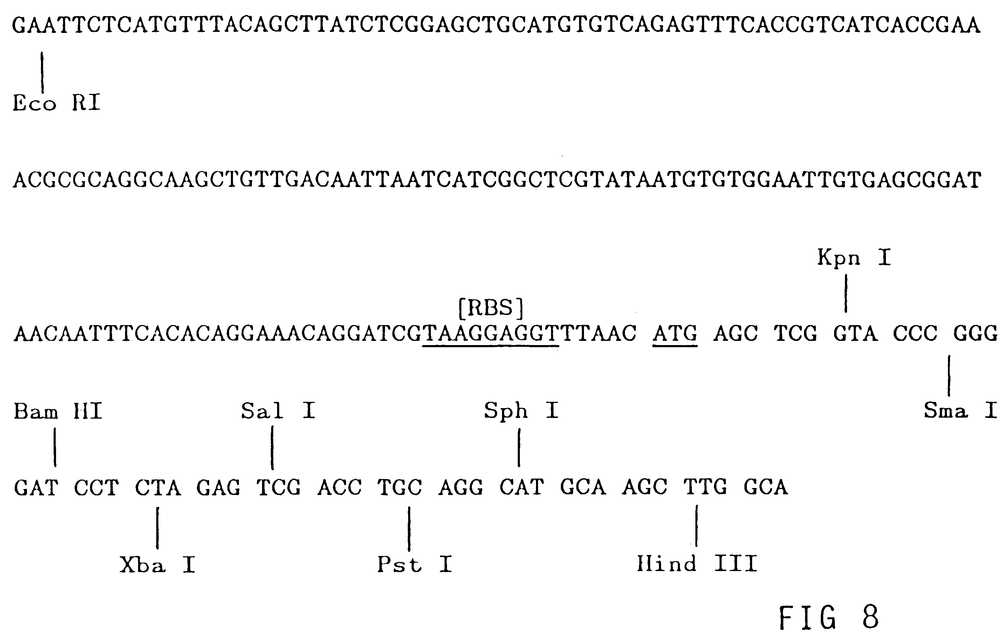

The TAC-RBS vector was constructed by a sequence of steps, starting with filling-in with DNA polymerase the single BamHI site of the tacP-bearing plasmid pDR540 (Pharmacia). The product was then ligated to unphosphorylated synthetic oligonucleotides (Pharmacia) which form a double-stranded fragment encoding a consensus ribosome binding site (RBS, GTAAGGAGGTTTAAC). After ligation, the mixture was phosphorylated and religated with the SstI linker ATGAGCTCAT. The resulting complex was then cleaved with SstI and EcoRI, and the 173 bp fragment isolated via polyacrylamide gel electrophoresis (PAGE) and cloned into EcoRI/SstI-restricted pUC18 (Pharmacia) (as described below). The sequence of the RBS-ATG-polylinker regions of the final construction (called TAC-RBS) is shown in Figure 8.

The synthetic human IL-4 gene is assembled into a pUC18 plasmid in six steps. At each step inserts free of deletions and/or inserts can be detected after cloning by maintaining the lacZ(α) gene of pUC18 in frame with the ATG start codon inserted in step 1. Clones containing deletion and/or insertion changes can be filtered out by scoring for blue colonies on L-ampicillin plates containing x-gal and IPTG. Alternatively, at each step sequences of inserts can be readily confirmed using a universal sequencing primer on small-scale plasmid DNA preparations, e.g. available from Boehringer Mannheim.

In step 1 the TAC-RBS vector is digested with SstI, treated with T4 DNA polymerase (whose 3' exonuclease activity digests the 3' protruding strands of the SstI cuts to form blunt-end fragments), and after deactivation of T4 DNA polymerase, treated with EcoRI to form a 173 base pair (bp) fragment containing the TAC-RBS region and having a blunt end at the ATG start codon and the EcoRI cut at the opposite end. Finally, the 173 bp TAC-RBS fragment is isolated.

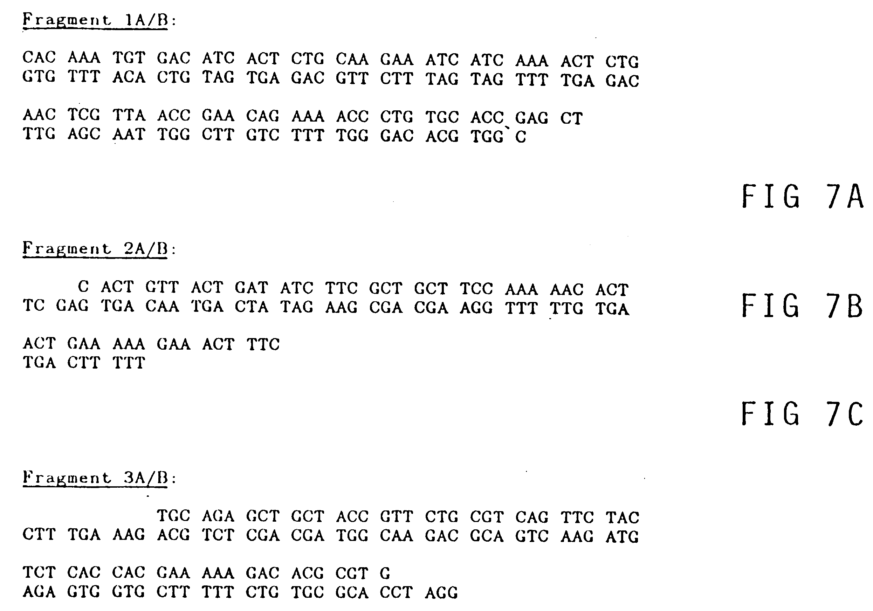

In step 2 the isolated TAC-RBS fragment of step 1 is mixed with EcoRI/SstI-digested plasmid pUC18 and synthetic fragment 1A/B, which as shown in Figure 7A has a blunt end at its upstream terminus and a staggered (sticky) end corresponding to an SstI cut at its downstream terminus. The fragments are ligated to form the pUC18 of step 2.

In step 3 synthetic fragments 2A/B and 3A/B (illustrated in Figure 7B and 7C) are mixed with SstI/BamHI-digested pUC18 of step 2 (after amplification and purification) and ligated to form pUC18 of step 3. Note that the downstream terminus of fragment 2A/B contains extra bases which form the BamHI staggered end. These extra bases are cleaved in step 4. Also fragments 2A/B and 3A/B have complementary 9-residue single-stranded ends which anneal upon admixture, leaving the upstream SstI cut of 2A/B and the downstream BamHI cut of 3A/B to ligate to the pUC18.

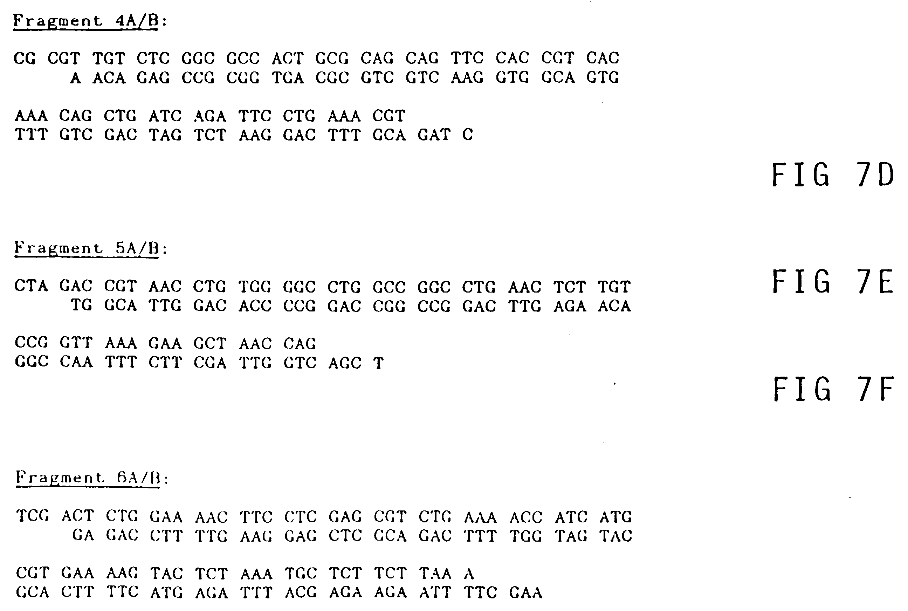

In step 4 MluI/XbaI-digested pUC18 of step 3 (after amplification and purification) is repurified, mixed with synthetic fragment 4A/B (Figure 7D), and ligated to form pUC18 of step 4.

In step 5 XbaI/SalI-digested pUC18 of step 4 (after amplification and purification) is mixed with synthetic fragment of 5A/B (Figure 7E) and ligated to form the pUC18 of step 5.

In step 6 SalI/HindIII-digested pUC18 of step 5 (after amplification and purification) is mixed with synthetic fragment 6A/B (Figure 7F) and ligated to form the final construction.

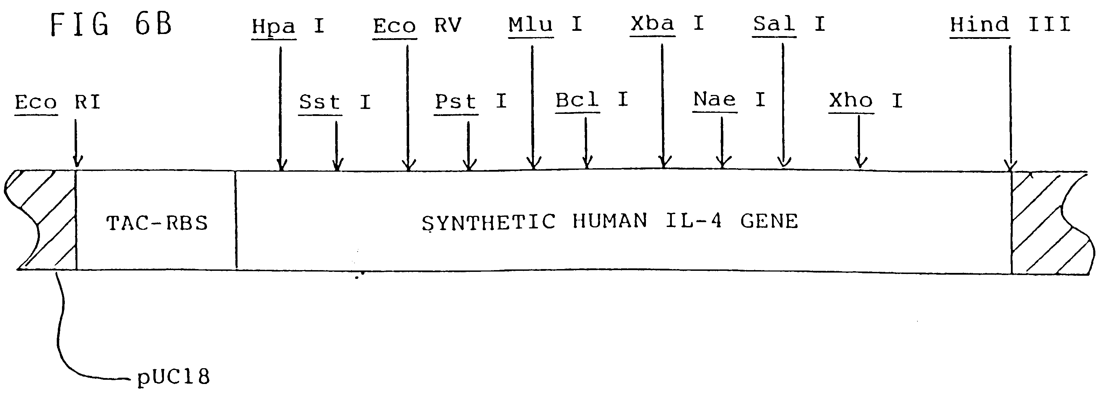

Figure 6B is a cleavage map of the unique restriction sites present in the pUC18 construction just described. When the disclosed synthetic human IL-4 gene is used as an insert of pUC18, each pair of unique restriction sites defines a segment which can be readily excised and replaced with altered synthetic segments. The set of unique restriction sites includes EcoRI, HpaI, SacI (SstI), EcoRV, PstI, MluI, BclI, XbaI, NaeI, SalI, XhoI, and HindIII.

The pUC18 containing the synthetic IL-4 gene is inserted in E. coli K-12 strain JM101. After culturing, protein is extracted from the JM101 cells and dilutions of the extracts are tested for biological activity.

### Example VIII. Construction and Expression of Human IL-4 Mutein Ile⁵² in E. coli.

Leu at position 52 (relative to the N-terminus of the native human IL-4) is changed to Ile to form human IL-4 mutein Ile⁵². The pUC18 plasmid of Example VII containing the synthetic human IL-4 gene of Figure 6A is digested with PstI and MluI and purified. The above purified pUC18 is mixed with the synthetic doublestranded fragment illustrated below and ligated. The altered part of the base sequence is boxed. The resulting pUC18 is transfected into E. coli K-12 strain JM101, or the like, and expressed.

GA GCT GCT ACC GTT ATC CGT
ACG TCT CGA CGA TGG CAA TAG GCA

CaG TTC TAC TCT CAC CAC GAA AAA
GTC AAG ATG AGA GTG GTG CTT TTT

GAC A
CTG TGC GC

### PstI/MluI Replacement Fragment For Generating Human IL-4 Mutein Ile⁵²

After culturing, protein is extracted from the JM101 cells using standard techniques, and dilutions of the extracts are tested for biological activity.

### Example IX. Construction and Expression of Human IL-4 Mutein (Ile⁵², Asp¹¹¹).

The modified pUC18 plasmid of Example VIII (containing the Ile⁵² coding sequence) is digested with SalI and XhoI, and the large fragment is isolated. The isolated fragment is mixed with the synthetic double-stranded fragment illustrated below in a standard ligation solution. The altered part of the sequence is boxed. The resulting plasmid is transfected into E. coli K-12 strain JM101, or the like, and expressed.

TCG ACT CTG GAA GAC TTC C
GA GAC CTT CTG AAG GAG CT

### SalI/XhoI Replacement Fragment

After culturing, protein is extracted from the JM101 cells using standard techniques, and dilutions of the extracts are tested for biological activity.

### Example X. Sequence of Human IL-4 Purified from Transfection Supernatants

Human IL-4 was purified from culture supernatants of cells transiently transfected with vectors containing human IL-4 cDNA. The sequence of the secreted native human IL-4 was determined from the purified material.

### A. Biological Assay for Purification.

TCGF activity was used to assay human IL-4 during the separation procedures. The assay was substantially the same as that described in Example II. Briefly, blood from a healthy donor was drawn into a heparinized tube and layered onto Ficoll-Hypaque; e.g., 5 ml of blood per 3 ml Ficoll-Hypaque in a 15 ml centrifuge tube. After centrifugation at 3000 x g for 20 minutes, cells at the interface were aspirated and diluted in a growth medium consisting of RPMI 1640 containing 10% fetal calf serum, 50 micromolar 2-mercaptoethanol, 20 microgram/ml phytohemagglutinin (PHA), and recombinant human IL-2. After 5-10 days of incubation at 37°C, the PHA-stimulated peripheral blood lymphocytes (PRLs) were washed and used in 2-day colorimetric assays: Mossmann, J. Immunol. Methods, Vol. 65, pgs. 55-63 (1983). Serial two-fold dilutions of the IL-4 standard (supernatants from either pcD-125- or pEBT-178-transfected COS 7 cells) or the fraction to be tested were performed in 96-well trays utilizing the growth medium described above to yield a final volume of 50 microliters/well. 50 microliters of the PHA-stimulated PBLs at about 4-8 x 10⁶ cells/ml were added to each well and the trays were incubated at 37°C for 2 days. Cell growth was then measured according to Mosmann (cited above).

Units of human IL-4 TCGF activity are defined with respect to supernatants of either pcD-125-transfected COS 7 cells (Example II) or pEBV-178-transfected COS 7 cells (Example III).

For purification, units are based on the activity of pcD-125-transfection supernatants, which are produced as follows. About 1 x 10⁶ COS 7 cells are seeded onto 100 mm tissue culture plates containing Dulbecco's Modified Eagle's medium (DME), 10% fetal calf serum, and 4 mM L-glutamine. About 24 hours after seeding, the medium is aspirated from the plates and the cells are washed twice with serum-free buffered (50 mM Tris) DME. To each plate is added 4 ml serum-free buffered DME (with 4 mM L-glutamine), 80 microliters DEAE-dextran, and 5 micrograms of pcD-125 DNA. The cells are incubated in this mixture for 4 hours at 30°C, after which the mixture is aspirated off and the cells are washed once with serum-free buffered DME. After washing, 5 ml of DME with 4 mM L-glutamine, 100 micromolar Chloroquine, and 2% fetal calf serum is added to each plate, and the cells are incubated for 3 hours and then twice washed with serum-free buffered DME. Next, 5 ml DME with 4 mM L-glutamine and 4% fetal calf serum is added and the cells are incubated at 37°C for 24 hours. Afterwards the cells are washed 1-3 times with DME or PBS, 5 ml serum-free DME (with 4 mM L-glutamine) is added, and the cells are incubated at 37°C until culture supernatants are harvested 5 days later.

One unit, as used herein, is the amount of factor which in one well (0.1 ml) stimulates 50% maximal proliferation of 2 x 10⁴ PHA-stimulated PBLs over a 48 hour period.

### B. Purification

Purification was accomplished by a sequential application of cation-exchange chromatography, gel filtration and reverse-phase high-pressure liquid chromatography. All operations were performed at 4°C.

After removal of the COS 7 cells by centrifugation, the supernatant was concentrated about 10 fold by ultrafiltration and stored at -80°C until further processed. IL-4 titers were determined by assaying for the ability of the protein to stimulate proliferation of phytohemagglutinin-induced human peripheral blood lymphocytes, i.e. by TCGF activity using the standard assay described above.

Concentrated COS 7 supernatant, having TCGF activity of about 10⁴-10⁶ units/ml and a protein content of about 15-20 mg/ml, is dialyzed against 2 changes of 50 mM sodium HEPES, pH 7.0 over a 24 hour period (each change being approximately 10-15 times the volume of one concentrate). The dialysate was applied to a column (1 x 2.5 cm) of S-Sepharose (flow rate: 0.2 ml/min) pre-equilibrated with 50 mM sodium HEPES, pH 7.0. The column was washed with 15 column volumes of equilibrating buffer followed by elution with 20 column volumes of a linear sodium chloride gradient extending from 0 to 0.5 M sodium chloride in 50 mM sodium HEPES, pH 7.0. The gradient was terminated by elution with 5 column volumes of uniform concentration: 50 mM sodium HEPES, 0.5 M NaCl, pH 7.0. 1.5 ml and 1.8 ml fractions were collected from respective batches. IL-4 titers were found for both chromatographies to elute between 300 mM and 500 mM sodium chloride.

Six fractions from each S-Sepharose column containing IL-4 titers were separately combined for total volumes of 9.0 and 10.8 ml. Both volumes were concentrated to 1.9 ml by ultrafiltration using an Amicon YM5 membrane (molecular weight cut-off: 5000). The recovery of protein from this step was about 80%. The concentrated IL-4 solution was applied to a Sephadex G-100 column (1.1 x 58 cm) pre-equilibrated in 50 mM HEPES, 0.4 M NaCl, pH 7.0, and the column was eluted with the same buffer at 0.15 ml/min. A total of 50 fractions (1.0 ml/fraction) were collected and analyzed for IL-4 titers. A peak in biological activity was observed at an apparent molecular weight of 22,000 daltons. The Sephadex G-100 was calibrated for apparent molecular determination with bovine serum albumin (65,000 daltons), carbonic anhydrase (30,000 daltons) and cytochrome C (11,700 daltons).

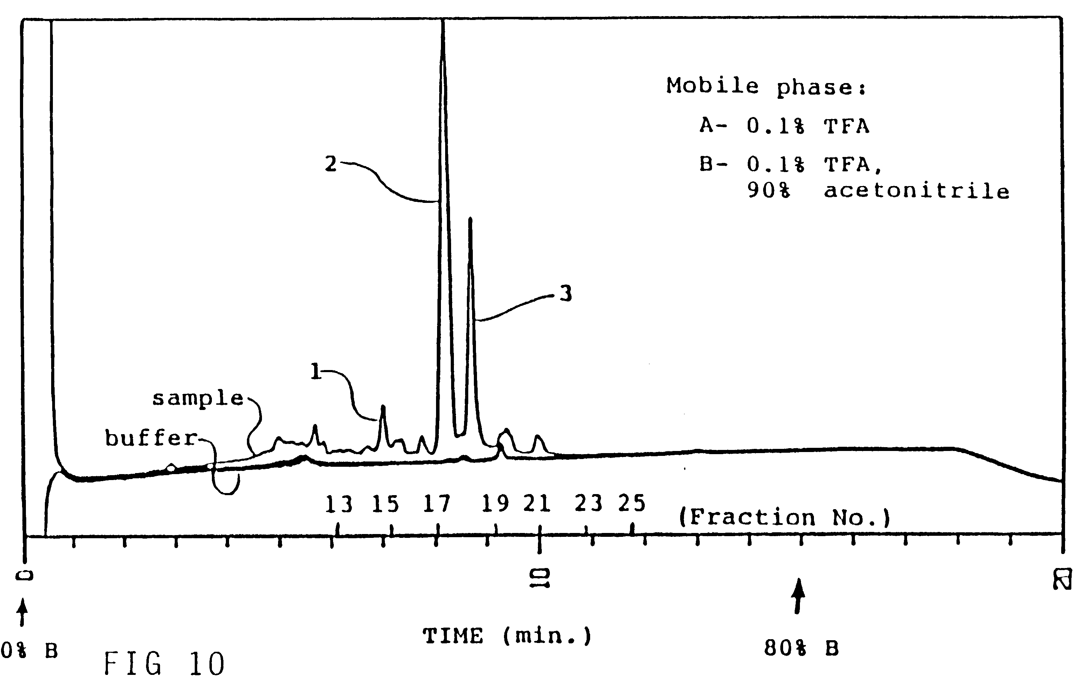

A fraction from the Sephadex G-100 column containing IL-4 activity was concentrated 3-4 fold in vacuo and was injected onto a Vydac C-4 guard column (4.6 x 20 mm). A linear gradient of 0 to 72% (v/v) acetonitrile in 0.1% (v/v) trifluoroacetic acid (TFA) was produced in 15 minutes at a column temperature of 35° and a flow rate of 1.0 ml/min. Three peaks resulted that were detected at 214 nm with retention times of 7, 8.2 and 8.7 min. (peaks 1, 2, and 3 of Figure 10, respectively). A 40 microliter aliquot of peak 2 (8.2 min. elution time) was lyophilized and redissolved in minimal essential medium containing 10% fetal calf serum. This solution showed a positive TCGF response. A 300 microliter aliquot of peak 2 was evaporated to dryness and redissolved in 200 µl of 0.1% (w/v) sodium dodecyl sulfate (SDS). A 2 µl aliquot was diluted in 200 µl of 1% (v/v) TFA and rechromatographed. The HPLC of this sample demonstrated a single peak at 215 nm. Peak 2 material indicated an activity of about 7 x 10⁸ units/mg.

### C. Amino Acid Sequence Analysis

Amino Acid sequence determination was performed by automated gas-phase Edman degradation (Hewick, R.M., Hunkapillar, M.W., Hood, L.E. and Dryer, W.J. (1981) J. Biol. Chem. 256: 7990) employing an Applied Biosystems microsequenator. A 90 microliter aliquot of the peak 2 HPLC fraction, dissolved in 0.1% SDS as described above, was applied to the glass fiber filter cartridge in the presence of Polybrene. Amino acid sequence information was obtained up to the 35th residue. The N-terminal sequence was determined as follows 
His - Lys - ___ - Asp - Ile - Thr - Leu - Gln - Glu -
Ile - Ile - Lys - Thr - Leu - Asn - Ser - Leu - Thr -
Glu - Gln - Lys - Thr - Leu - ___ - Thr - Glu - Leu -
___ - Val - Thr - Asp - Ile - Phe - Ala - Ala
 wherein the blanks indicate the lack of an identifiable amino acid.

Blanks in the the amino-terminus at positions 3 and 23 were consistent with the presence of cysteine, which cannot be detected in this system. The blank at position 28, corresponding to a threonine in the cDNA-predicted sequence, may have been due either to the variability of phenylthiohydantoin-threonine detection or to the presence of O-linked glycosylation or esterification.

A 100 microliter aliquot of the HPLC fraction on which the amino-terminal sequence was performed was evaporated to dryness and redissolved in 70% formic acid. A 50-fold molar excess of cyanogen bromide was added and the solution was allowed to stand at room temperature for 2.5 hours. The cleaved protein was sequenced on the Applied Biosystems gas-phase sequenator, as described above. Two sequences were identifiable: (1) Arg - Glu - Lys - Tyr - Ser - Lys (2) His - Lys - ___ - Asp - Ile - Thr Sequence (1) is identical to the sequence predicted from the cDNA to have been released following cyanogen bromide cleavage of the methionine residue at position 120. The last two residues of the C-terminus may have been present but may not have been detectable owing to insufficient sample. Sequence (2) is identical to the amino-terminal sequence obtained for the native IL-4 protein, as described above. The relative amounts of amino-terminal and carboxyl-terminal phenylthiohydantoin-amino acids released suggested equimolar amounts of both sequences in the sample. This result supports the conclusion that the protein sample that was sequenced contained predominantly a single polypeptide chain with amino and carboxyl termini predicted from the cDNA sequence of human IL-4.

### Example XI. Construction and Expression of Human IL-4 Mutein (Ile⁵², Δ⁷¹, IS⁹⁴(Ala))

The modified pUC18 plasmid of Example VIII (containing the Ile⁵² coding sequence) is digested with MluI and BclI, and the large fragment is isolated. The isolated fragment is mixed with the synthetic double-stranded fragment illustrated below in a standard ligation solution. The resulting plasmid is transfected into E. coli K-12 JM101, or the like, and propagated.

CG CGT TGT CTC GGC GCC ACT
A ACA GAG CCG CGG TGA

GCG CAG TTC CAC CGT CAC AAA GAG CT
CGC ↑ GTC AAG GTG GCA GTG TTT GTC GAC TAG
Deletion

### MluI/BclI Replacement Fragment

The modified plasmid is isolated and digested with XbaI and NaeI, and the large fragment is isolated. The isolated fragment is mixed with the synthetic double-stranded fragment illustrated below in a standard ligation solution. The added codon is boxed. The resulting plasmid is transfected into E. coli K-12 strain JM101, or the like, and expressed.

CTA GAC CGT AAC CTG TGG GGC
TG GCA TTG GAC ACC CCG

CTG GCC GCC
GAC CGG CGG

### XbaI/NaeI Replacement Fragment

After culturing, protein is extracted from the JM101 cells using standard techniques, and dilutions of the extracts are tested for biological activity.

### Example XII. Induction of DR Antigens on Cells from a Patient Suffering from Bare Lymphocyte Syndrome.

Bare lymphocyte syndrome is characterized by the lack of expression of class I and/or class II HLA antigens on cell surfaces, and is frequently associated with severe immunodeficiency, e.g. Touraine, Lancet, pgs. 319-321 (February 7, 1981); Touraine and Bethel, Human Immunology, Vol. 2, pgs. 147-153 (1981); and Sullivan et al., J. Clin. Invest., Vol. 76, pgs. 75-79 (1985). It was discovered that human IL-4 was capable of inducing the expression of the class II DR antigen on the surfaces of cells derived from a patient suffering from bare lymphocyte syndrome.

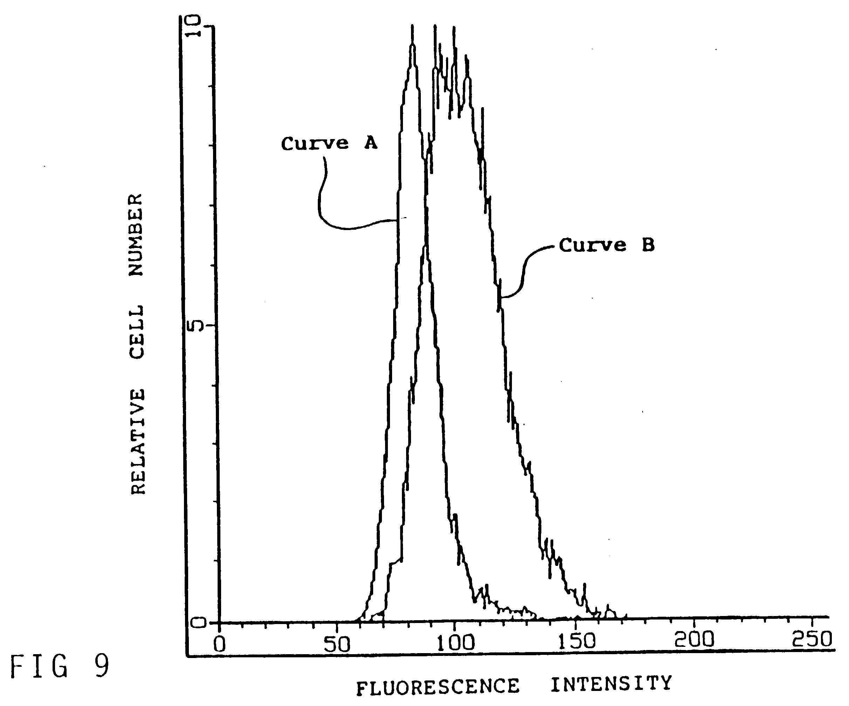

Peripheral blood lymphocytes (PBLs) were obtained from a patient suffering from non-expression of HLA class II antigens. B cells were purified from the PBLs essentially as described above, and a B cell line (designated UD31) was established by transformation with Epstein-Barr virus (EBV). The EBV-transformed cells were cultured for 48 hours in Yssel's defined medium (described above) with 2% fetal calf serum and a 5% (v/v) concentration of supernatant from pcD-125-transfected COS 7 cells. The cells were harvested, fixed, stained with fluorescently-labeled anti-oR monoclonal antibody (e.g. Becton Dickinson, L243), and analyzed by flow cytometry. Figure 9 illustrates histograms of cell frequency versus fluorescence intensity for a control population of the EBV-transformed cells harvested prior to IL-4 treatment (Curve A), and for the population of EBV-transformed cells after IL-4 treatment (Curve B).

### Example XIII. MCGF Activity of C1.Lyl⁺2⁻/9 (C1.1) Supernatants.

Purified IL-3 and supernatant from COS 7 cells transfected with an IL-3 cDNA clone (recombinant IL-3; IL-3^{R}) stimulated proliferation of the mast cell line MC/9 to the same extent (Fig. 14A). However, this level was a third to a half of that induced by C1.1 T cell supernatant. The presence of ConA in the T cell supernatant (2 µg/ml) does not contribute significantly to its MCGC activity. One explanation for the effect noted above is that IL-3 does not account for all of the MCGF activity produced by C1.1 cells. Alternatively, the mast cell line used for assays could (theoretically) be contaminated with a second factor-dependent population of cells. This latter possibility is excluded, however, because re-cloning the MC/9 cell line yielded subclones, each of which responds to IL-3 and the cell supernatant in the same way as the original MC/9 clone.

Since C1.1 supernatant also contains lymphokines other than IL-3, these were tested for their ability to promote the growth of MC/9 cells. However, MC/S cells were unresponsive to various concentrations of recombinant IL-2, IFN and GM-CSF, as well as to supernatant containing IL-1 (P388D 1 cells) (Fig. 14A). Moreover, supplementation of cultures containing varying concentrations of recombinant IL-3 with IL-2, GM-CSF, IFN-γ, or IL-1 did not stimulate proliferation of MC/9 cells above the level obtained with IL-3 alone. This indicates that the increased MCGF activity of the T cell supernatant is not due to its content of these other known factors.

### Comparative Studies with Other Factor-Dependent Cell Lines.

Two other mast cell lines, DX-2 and MM3, also gave higher proliferative responses with C1.1 supernatant than with IL-3 (representative data for DX-2 shown in Figure 14B). These cells also did not respond to IL-2, IFN-γ, GM-CSF or supernatant of P388D-1 cells. Thus, the enhanced proliferative response of MC/9 cells to the T-cell supernatant may be typical of mast cells in general. In contrast, granulocytic-type cells (NFS-60) were stimulated almost equally by IL-3 and C1.1 supernatant (Fig. 14C). Although NFS-60 cells were unresponsive to IL-2, IFN-γ and P388D-1 supernatant, there was a small but reproducible stimulation by GM-CSF. The fact that IL-3 and the T cell supernatant induce comparable levels of proliferation suggests that the NFS-60 cell line may be relatively insensitive to additional factors present in the T cell supernatant. Thus, NFS-60 cells were used to assay IL-3 during attempts to separate IL-3 from other MCGF activities by protein fractionation.

The TCGF activity of C1.1 supernatant was assessed using the IL-2 dependent T cell line, HT2. Saturating concentrations of C1.1 supernatant invariably stimulated ³H-thymidine incorporation well below the maximum level achieved with recombinant IL-2 (Fig. 14D). Similar results were obtained with two other cell lines. The supernatant does not contain an inhibitory substance since its addition to HT2 cultures containing IL-2 showed no reduction in ³H-thymidine incorporation (Fig. 14D). These results suggest C1.1 supernatant contains a TCGF activity distinct from IL-2. Biochemical evidence in support of this conclusion is presented below.

### Preliminary Chromatographic Fractionation of C1.1 Supernatant.

C1.1 supernatant was fractionated by various chromatographic methods with the immediate goal of separating IL-3 from other MCGF activities. Proliferation of NFS-60 cells was used to track IL-3 specifically against the background of total MCGF activity revealed by MC/9 proliferation. TCGF activity was assessed by HT2 cell proliferation.

When C1.1 supernatant was fractionated by cation exchange chromatography at neutral pH, approximately 98% of the loaded protein, 97% of the IL-3 units (assessed by NFS-60 proliferation) and 1% of the TCGF units appeared in the flow-through (Fig. 15). Elution of bound protein with a gradient of sodium chloride released TCGF activity at 0.19M NaCl (fraction 60). A small but reproducible peak of TCGF activity also appeared at 1M NaCl. No further significant TCGF activity could be eluted at higher NaCl concentrations (up to 3M). A small amount of IL-3 also eluted in approximately the same region as the TCGF peak (detectable NFS-60 response). Only fractions corresponding to peak TCGF activity (fractions 59-61) and the flow-through (fractions 1-20) stimulated high levels of MC/9 proliferation, comparable to the unfractionated supernatant (arrows, Fig. 15). Since titration curves suggested fractions 59-61 contained more of the high level MCGF activity than did the flow-through, attempts were made to deplete further the IL-3 activity co-eluting with the TCGF peak and again assess MC/9 proliferation levels. Fractions 59-61 were therefore rechromatographed twice more under identical conditions.

After the third column pass, more than 95% of the TCGF activity consistently eluted at 0.19 M NaCl. There was no measurable IL-3 (NFS-60) response) in the flow-through or in any of the column fractions. However, still co-eluting with the TCGF peak was an MCGF activity that now stimulated a plateau level of MC/9 proliferation that was significantly lower than that obtained with C1.1 supernatant or IL-3 (see below). Since these fractions were devoid of measurable IL-3 activity (absence of NFS-60 response), apparently the new MCGF by itself was incapable of stimulating MC/9 cells to the same degree as the crude T cell supernatant.

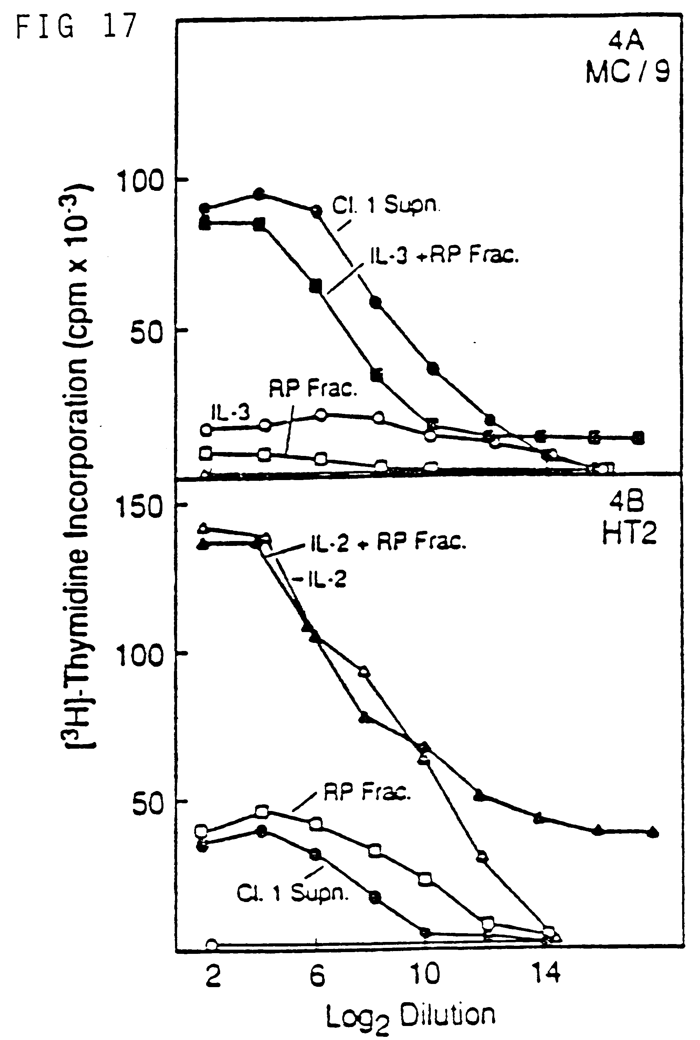

To further attempt separation of MCGF from TCGF, the 3X fractionated material (fractions 59-61) was diluted 10X with 0.1% TFA in H₂O to pH 2 and loaded directly onto a C8 reverse phase column. Fig. 16A shows the elution profile of bound protein released with a gradient of acetonitrile, 0.1% in TFA. No MCGF or TCGF activity appeared in the flow-through; both activities co-eluted at 37% acetonitrile (fractions 26-29). However, the saturating MCGF response was now only about one half that produced by IL-3 alone (Fig. 17A). When all fractions were re-assayed in the presence of saturating levels of IL-3, only fractions 26-29 showed an MC/9 proliferation response in excess of IL-3 alone (arrow, Fig. 16A). In fact, the magnitude of the response was similar to that of the unfractionated supernatant itself (Fig. 17A). Further, proliferation levels equivalent to those of unfractionated supernatant were also observed with fractions 26-29 were assayed in the presence of IL-3 containing flow-through or IL-3 purified form WEHI 3. Thus, the combination of IL-3 and the unique MCGF/TCGF stimulates levels of MC/9 proliferation characteristic of the original T cell supernatant.

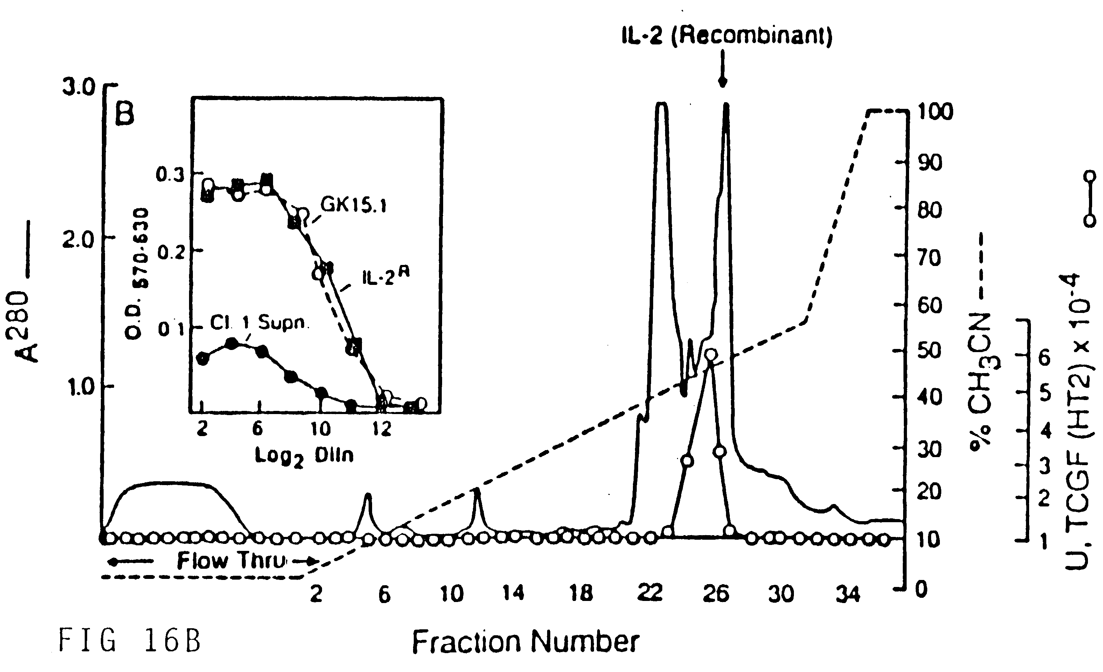

Altough reverse phase chromatography fails to resolve the MCGF from the TCGF, the TCGF was shown to be distinct from IL-2 by two criteria. First, when recombinant IL-2 in COS-7 cell supernatants (IL-2^{R}) and supernatant from an IL-2 producing (ConA stimulated) murine T cell line (GF15-1) are both chromatographed on the same column under identical conditions, the TCGF activity elutes at 45% acetonitrile in a sharp peak that does not overlap with the 37% acetonitrile position marking the C1.1 TCGF (Fig. 16B). Second, the saturating response of HT2 cells to IL-2^{R} is very different from that which characterizes the C1.1 supernatant (Fig. 16B, insert). Further, no obvious synergy was detected between the partially purified TCGF and IL-2 (Fig. 17B).

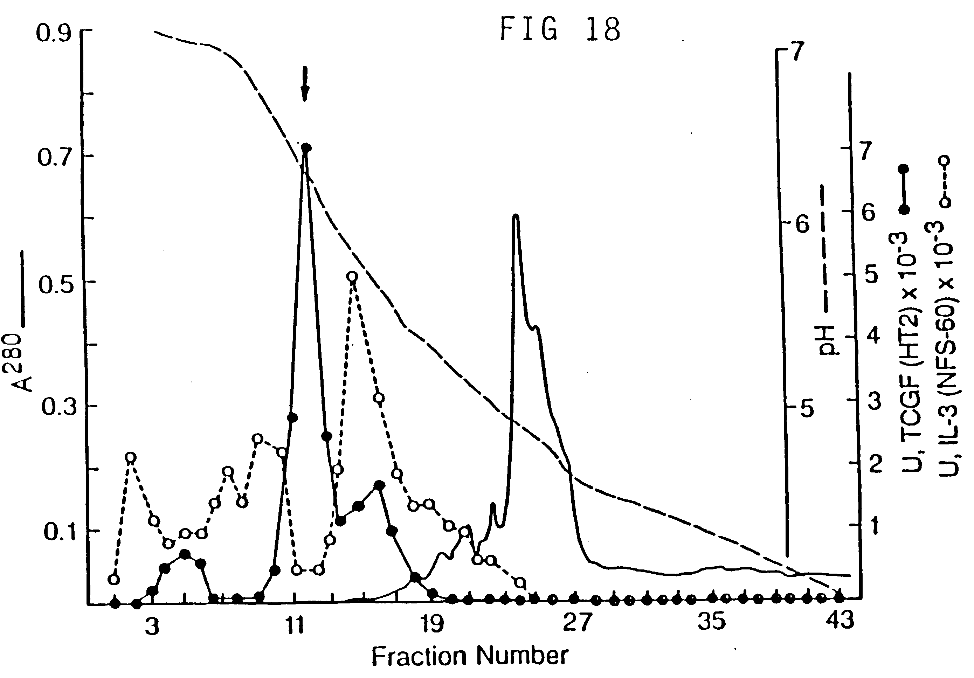

The chromatofocussing pattern of C1.1 supernatant is shown in Fig. 18. The IL-3 activity (NFS-60 response) displayed a very heterogeneous profile, including activity with pI values higher than 7.1, but the TCGF activity appeared more homogeneous, with the major TCGF species (fraction 12) showing an approximate pI of 6.2. This coincides with the maximum MCGF activity (MC/9 response) after addition of saturating levels of IL-3.

### SDS PAGE of C1.1 Supernatant and of Partially Purified Factor.

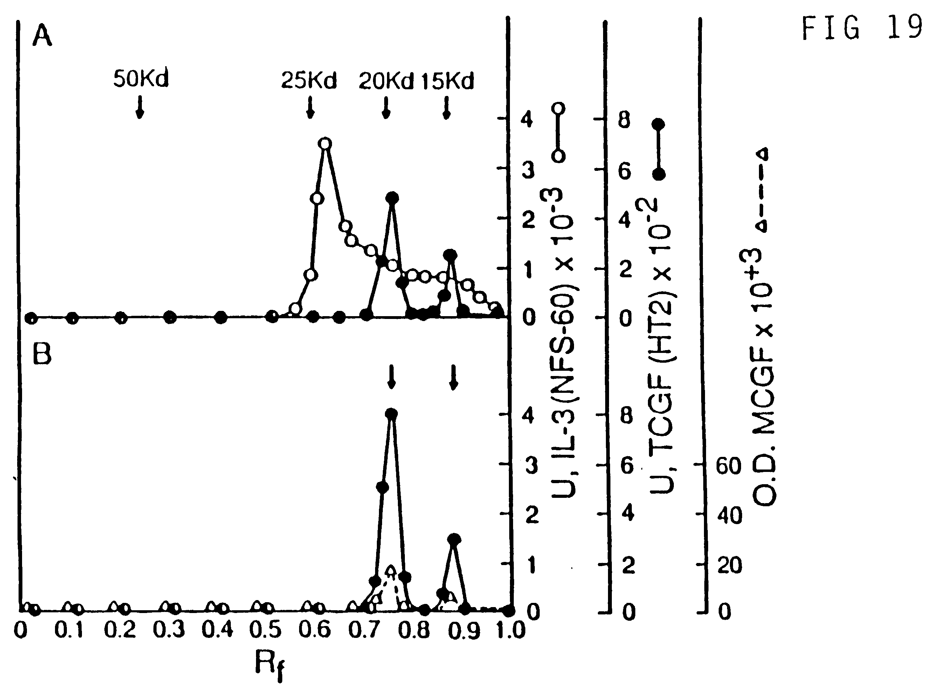

Figure 19 compares the electrophoretic profiles of unfractionated C1.1 supernatant (Fig. 19A) with that of the cation exchanged material, fractions 59-61 (Fig. 19B). Protein was eluted directly from gel slices and activities were determined by proliferation. Both the cation exchange fraction and C1.1 supernatant show TCGF peaks: at 20 Kd and 15 Kd under non-reducing conditions, and at 21 Kd and 16 Kd under reducing conditions. Since the cation exchanged material was depleted of IL-3, only low levels of MC/9 proliferation were induced and this MCGF activity was still coincident with TCGF peaks. Addition of saturating amounts of IL-3^{R} to all fractions raised the MC/9 proliferation response to C1.1 supernatant levels only in the peak MCGF/TCGF fractions (arrows, Fig. 19B). IL-3 activity of the supernatant was greatest at the 24 Kd position, but also appears in broad peaks about the 20 Kd region. Significantly, MC/9 proliferation levels well above that of IL-3 occur at the 20 Kd position of electrophoresed supernatant (arrow, Fig. 19A). A silver-stained SDS gel of the most highly purified MCGF/TCGF material (fraction 28 of the reverse phase column) also showed prominent protein bands at 20 Kd and 15 Kd.

Microgram quantities of the 20 kd form of the factor have been purified to homogeneity by successive fractionation of ConA-supernatants from activated C1.1 T-cells by (1) cation exchange chromatography, (2) Heparin Sepharose chromatography, and (3) c4 reverse phase chromatography as described previously. The final material shows only a single band at 20 kd by silver-stained SDS-PAGE, which corresponds to a single UV absorbing peak on the c4 elution profile. Biological characterization of the purified factor confirmed its ability to stimulate low levels of T-cell and mast cell proliferation and to enhance IL-3-dependent mast cell proliferation.

As shown in Table V, the factor possesses several B-cell stimulatory activities ascribed to BSF1 (Roehm, N.W. et al., J. Exp. Med. 169: 679-694 (1984); Howard, M. et al., Proc. Natl. Acad. Sci. U.S.A. 78: 5788 (1981); Roehm, N.W. et al., Proc. Natl. Acad. Sci. U.S.A. 81: 6149-6153 (1984). First, it induces increased expression of Ia on resting B-cells as compared to the control polulation (Table V). Second, the factor, in conjunction with anti-Ig antibodies, induces significant [³H]-thymidine incorporation, indicating that it functions as an accessory factor in the stimulation of B-cell proliferation.

**Table V**

| B-cell Stimulation Assays | | |
|---|---|---|
| Source | [³]-thymidine incorporation of anti-Ig treated B cells* | I-A induction** |
| Unfractionated C1.Lyl⁺2⁻/9 Supernatant | 33,788 cpm ± 5,454 | 162.3 |
| Purified factor | 44,125 cpm ± 2,775 | 157.1 |
| Medium (negative control) | 4,128 cpm ± 734 | 35.5 |

| | | |
|---|---|---|
| * results reported as average cpm ± SEM of triplicate cultures supplemented with saturating levels of factor. | | |
| ** results reported as average relative mean fluorescence of triplicate cultures supplemented with saturating levels of factor. | | |

These polypeptides can exhibit several biological activities, differing in both the apparent function mediated and the type of cell affected. As described, these activities include synergy in the induction of proliferation of B-cells and mast cell lines, early activation of resting B-cells, stimulation of the proliferation of T-cells, and selective enhancement of IgG₁ and IgE production of mitogen-activated B-cells. Purification of the polypeptides to homogeneity has clarified the socpe of these activities.

From the foregoing, it will be appreciated that the purified proteins of the present invention provide those skilled in the art with novel therapeutic capabilities. The ability to produce significant quantities of these factors will permit improved in vitro culture of selected mammalian cell lines, as well as an overall improved understanding of the mammalian immune response.

cDNA clones pcD-2A-E3, pcD-46 (pcD-2F1-13), pcD-125, and yeast vector pMF-alpha8 have been deposited with the American Type Culture Collection, Rockville, MD, USA (ATCC), under accession numbers 53330, 53337, 67029, and 40140, respectively. These deposits have been made under the Budapest Treaty (1977) on the International Recognition of the Deposit of Micro-organisms for the purposes of Patent Procedure.

## Claims (Claims for the following Contracting State(s): BE, CH, LI, DE, FR, GB, IT, LU, NL, SE)

1. An interleukin-4 protein characterised by possessing B-cell growth factor activity (BCGF) on human cells and T-cell growth factor activity (TCGF) on human cells.

2. Human interleukin-4 according to claim 1 in substantially pure form, and exhibiting in addition mast cell stimulatory activity.

3. Human interleukin-4 according to Claim 1 in the form of a recombinant polypeptide.

4. Human interleukin-4 as claimed in Claim 1 and having at least 9 x 10⁷ units/mg of TCGF (T-cell growth factor) activity and further exhibiting at least one activity, preferably at least two, selected from MHC antigen induction activity, Fc-epsilon receptor induction activity, GM-CSF stimulated granulocyte colony growth potentiating activity, interleukin-2 TCGF potentiating activity, and IgG₁- and IgE-induction activity, said activities being assayed on human cell lines.

5. A protein having human interleukin-4 activity and comprising a 3-fold substituted glycosylated or unglycosylated polypeptide having a sequence of amino acids defined by the formula: 
X(His) - X(Lys) - X(Cys) - X(Asp) - X(Ile) - X(Thr) -
X(Leu) - X(Gln) - X(Glu) - X(Ile) - X(Ile) - X(Lys) -
X(Thr) - X(Leu) - X(Asn) - X(Ser) - X(Leu) - X(Thr) -
X(Glu) - X(Gln) - X(Lys) - X(Thr) - X(Leu) - X(Cys) -
X(Thr) - X(Glu) - X(Leu) - X(Thr) - X(Val) - X(Thr) -
X(Asp) - X(Ile) - X(Phe) - X(Ala) - X(Ala) - X(Ser) -
X(Lys) - X(Asn) - X(Thr) - X(Thr) - X(Glu) - X(Lys) -
X(Glu) - X(Thr) - X(Phe) - X(Cys) - X(Arg) - X(Ala) -
X(Ala) - X(Thr) - X(Val) - X(Leu) - X(Arg) - X(Gln) -
X(Phe) - X(Tyr) - X(Ser) - X(His) - X(His) - X(Glu) -
X(Lys) - X(Asp) - X(Thr) - X(Arg) - X(Cys) - X(Leu) -
X(Gly) - X(Ala) - X(Thr) - X(Ala) - X(Gln) - X(Gln) -
X(Phe) - X(His) - X(Arg) - X(His) - X(Lys) - X(Gln) -
X(Leu) - X(Ile) - X(Arg) - X(Phe) - X(Leu) - X(Lys) -
X(Arg) - X(Leu) - X(Asp) - X(Arg) - X(Asn) - X(Leu) -
 
X(Trp) - X(Gly) - X(Leu) - X(Ala) - X(Gly) - X(Leu) -
X(Asn) - X(Ser) - X(Cys) - X(Pro) - X(Val) - X(Lys) -
X(Glu) - X(Ala) - X(Asn) - X(Gln) - X(Ser) - X(Thr) -
X(Leu) - X(Glu) - X(Asn) - X(Phe) - X(Leu) - X(Glu) -
X(Arg) - X(Leu) - X(Lys) - X(Thr) - X(Ile) - X(Met) -
X(Arg) - X(Glu) - X(Lys) - X(Tyr) - X(Ser) - X(Lys) -
X(Cys) - X(Ser) - X(Ser);
 wherein:
X(Ser) represents the group consisting of Ser, Thr, Gly, and Asn;
X(Arg) represents the group consisting of Arg, His, Gln, Lys, and Glu;
X(Leu) represents the group consisting of Leu, Ile, Phe, Tyr, Met, and Val;
X(Pro) represents the group consisting of Pro, Gly, Ala, and Thr;
X(Thr) represents the group consisting of Thr, Pro, Ser, Ala, Gly, His, and Gln;
X(Ala) represents the group consisting of Ala, Gly, Thr, and Pro;
X(Val) represents the group consisting of Val, Met, Tyr, Phe, Ile, and Leu;
X(Gly) represents the group consisting of Gly, Ala, Thr, Pro, and Ser;
X(Ile) represents the group consisting of Ile, Met, Tyr, Phe, Val, and Leu;
X(Phe) represents the group consisting of Phe, Trp, Met, Tyr, Ile, Val, and Leu;
X(Tyr) represents the group consisting of Tyr, Trp, Met, Phe, Ile, Val, and Leu;
X(His) represents the group consisting of His, Glu, Lys, Gln, Thr, and Arg;
X(Gln) represents the group consisting of Gln, Glu, Lys, Asn, His, Thr, and Arg;
X(Asn) represents the group consisting of Asn, Glu, Asp, Gln, and Ser;
X(Lys) represents the group consisting of Lys, Glu, Gln, His, and Arg;
X(Asp) represents the group consisting of Asp, Glu, and Asn;
X(Glu) represents the group consisting of Glu, Asp, Lys, Asn, Gln, His, and Arg;
X(Cys) represents the group consisting of Cys, Ser and Thr;
X(Trp) is Trp; and
X(Met) represents the group consisting of Met, Phe, Ile, Val, Leu, and Tyr.

6. A glycosylated or unglycosylated polypeptide as claimed in claim 5 which is 1-fold substituted.

7. A glycosylated or unglycosylated polypeptide as claimed in claim 5 which is 1-fold substituted within the region defined by amino acid residues 3-29 inclusive, 35-66 inclusive, 78-87 inclusive, 98-99 inclusive, and 105-125 inclusive.

8. A protein as claimed in any of claims 5 to 7 wherein:
X(Arg) is Arg;
X(Leu) represents the group consisting of Leu, Ile, and Met;
X(Pro) is Pro;
X(Ala) is Ala;
X(Val) is Val;
X(Ile) represents the group consisting of Ile, Met, and Leu;
X(Phe) is Phe;
X(Tyr) is Tyr;
X(His) is His;
X(Gln) is Gln;
X(Asn) is Asn;
X(Lys) is Lys;
X(Asp) is Asp;
X(Glu) is Glu;
X(Cys) represents the group consisting of Cys and Ser; and
X(Met) represents the group consisting of Met, Ile, and Leu;
in particular wherein said polypeptide is 1-fold substituted within the region defined by amino acid residues 3-29 inclusive, 35-66 inclusive, 78-87 inclusive, 98-99 inclusive, and 105-125 inclusive

9. Mature human interleukin-4 having the formula

-
-
-
-
-
-
-
-
-
-
-
-
.

10. Human interleukin-4 having the formula given in Claim 9 together with the following leader sequence:

Met-Gly-Leu-Thr-Ser-Gln-Leu-Leu-Pro
Pro-Leu-Phe-Phe-Leu-Leu-Ala-Cys-Ala-Gly-
Asn-Phe-Val-His-Gly

11. A glycosylated or unglycosylated polypeptide having human interleukin-4 activity and selected from 1-fold inserted, 1-fold deleted, and 1-fold substituted polypeptides of the formula: 
X(His) - X(Lys) - X(Cys) - X(Asp) - X(Ile) - X(Thr) -
X(Leu) - X(Gln) - X(Glu) - X(Ile) - X(Ile) - X(Lys) -
X(Thr) - X(Leu) - X(Asn) - X(Ser) - X(Leu) - X(Thr) -
X(Glu) - X(Gln) - X(Lys) - X(Thr) - X(Leu) - X(Cys) -
X(Thr) - X(Glu) - X(Leu) - X(Thr) - X(Val) - X(Thr) -
X(Asp) - X(Ile) - X(Phe) - X(Ala) - X(Ala) - X(Ser) -
X(Lys) - X(Asn) - X(Thr) - X(Thr) - X(Glu) - X(Lys) -
X(Glu) - X(Thr) - X(Phe) - X(Cys) - X(Arg) - X(Ala) -
X(Ala) - X(Thr) - X(Val) - X(Leu) - X(Arg) - X(Gln) -
X(Phe) - X(Tyr) - X(Ser) - X(His) - X(His) - X(Glu) -
X(Lys) - X(Asp) - X(Thr) - X(Arg) - X(Cys) - X(Leu) -
X(Gly) - X(Ala) - X(Thr) - X(Ala) - X(Gln) - X(Gln) -
X(Phe) - X(His) - X(Arg) - X(His) - X(Lys) - X(Gln) -
X(Leu) - X(Ile) - X(Arg) - X(Phe) - X(Leu) - X(Lys) -
X(Arg) - X(Leu) - X(Asp) - X(Arg) - X(Asn) - X(Leu) -
X(Trp) - X(Gly) - X(Leu) - X(Ala) - X(Gly) - X(Leu) -
X(Asn) - X(Ser) - X(Cys) - X(Pro) - X(Val) - X(Lys) -
X(Glu) - X(Ala) - X(Asn) - X(Gln) - X(Ser) - X(Thr) -
X(Leu) - X(Glu) - X(Asn) - X(Phe) - X(Leu) - X(Glu) -
X(Arg) - X(Leu) - X(Lys) - X(Thr) - X(Ile) - X(Met) -
X(Arg) - X(Glu) - X(Lys) - X(Tyr) - X(Ser) - X(Lys) -
X(Cys) - X(Ser) - X(Ser)
 wherein:
X(Ser) represents the group consisting of Ser, Thr, Gly, and Asn;
X(Arg) represents the group consisting of Arg, His, Gln, Lys, and Glu;
X(Leu) represents the group consisting of Leu, Ile, Phe, Tyr, Met, and Val;
X(Pro) represents the group consisting of Pro, Gly, Ala, and Thr;
X(Thr) represents the group consisting of Thr, Pro, Ser, Ala, Gly, His, and Gln;
X(Ala) represents the group consisting of Ala, Gly, Thr, and Pro;
X(Val) represents the group consisting of Val, Met, Tyr, Phe, Ile, and Leu;
X(Gly) represents the group consisting of Gly, Ala, Thr, Pro, and Ser;
X(Ile) represents the group consisting of Ile, Met, Tyr, Phe, Val, and Leu;
X(Phe) represents the group consisting of Phe, Trp, Met, Tyr, Ile, Val, and Leu;
X(Tyr) represents the group consisting of Tyr, Trp, Met, Phe, Ile, Val, and Leu;
X(His) represents the group consisting of His, Glu, Lys, Gln, Thr, and Arg;
X(Gln) represents the group consisting of Gln, Glu, Lys, Asn, His, Thr, and Arg;
X(Asn) represents the group consisting of Asn, Glu, Asp, Gln, and Ser;
X(Lys) represents the group consisting of Lys, Glu, Gln, His, and Arg;
X(Asp) represents the group consisting of Asp, Glu, and Asn;
X(Glu) represents the group consisting of Glu, Asp, Lys, Asn, Gln, His, and Arg;
X(Cys) represents the group consisting of Cys, Ser and Thr;
X(Trp) is Trp; and
X(Met) represents the group consisting of Met, Phe, Ile, Val, Leu, and Tyr;
especially wherein
X(Ser) is Ser;
X(Arg) represents the group consisting of Arg, His, and Lys;
X(Leu) represents the group consisting of Leu, Ile, Phe, and Met;
X(Pro) represents the group consisting of Pro and Ala;
X(Thr) is Thr;
X(Ala) represents the group consisting of Ala and Pro;
X(Val) represents the group consisting of Val, Met, and Ile;
X(Gly) is Gly;
X(Ile) represents the group consisting of Ile, Met, Phe, Val, and Leu;
X(His) represents the group consisting of His, Gln, and Arg;
X(Gln) represents the group consisting of Gln, Glu, and His;
X(Asn) represents the group consisting of Asn and Asp;
X(Lys) represents the group consisting of Lys and Arg;
X(Asp) represents the group consisting of Asp and Asn;
X(Glu) represents the group consisting of Glu and Gln; and
X(Met) represents the group consisting of Met, Phe, Ile, Val, and Leu;
in particular wherein said polypeptide is 1-fold inserted, y1-fold deleted, or y y1-fold substituted within the region defined by amino acid residues 3-29 inclusive, 35-66 inclusive, 78-87 inclusive, 98-99 inclusive, and 105-125 inclusive

12. Human interleukin-4 mutein Δ¹⁻⁴, human interleukin-4 mutein IS^{o}(Gly-Asn-Phe-Val-His-Gly) and human interleukin-4 mutein IS^{o}(Ala-Glu-Phe)

13. A pharmaceutical composition for stimulating the immune system comprising a therapeutically compatible carrier and an effective amount of human interleukin-4y or a polypeptide having human interleukin-4 activity as claimed in any of Claims 1 to 12.

14. A nucleic acid comprising a sequence of nucleotide bases capable of coding for a polypeptide as defined in any of Claims 1 to 12, preferably a sequence of nucleotide bases that is effectively homologous to a sequence of DNA in a cDNA insert of a vector selected from pcD-46 and pcD-125, in particular wherein said effectively homologous sequence is at least fifty percent homologous and preferably at least seventy-five percent homologous to said sequence of DNA in said cDNA insert of said vector selected from said group consisting of pcD-46 and pcD-125.

15. A compound selected from the group consisting of nucleic acids comprising a sequence of codons capable of coding for the polypeptides of any of claims 4 to 6, in particular wherein said sequence of codons is defined by the sequence of bases from position 64, preferably position 130, to position 522 in Figure 1B, especially the sequence of codons defined by the following formula:

CAC AAA TGT GAC ATC ACT CTG CAA GAA ATC ATC AAA
ACT CTG AAC TCG TTA ACC GAA CAG AAA ACC CTG TGC
ACC GAG CTC ACT GTT ACT GAT ATC TTC GCT GCT TCC
AAA AAC ACT ACT GAA AAA GAA ACT TTC TGC AGA GCT
GCT ACC GTT CTG CGT CAG TTC TAC TCT CAC CAC GAA
AAA GAC ACG CGT TGT CTC GGC GCC ACT GCG CAG CAG
TTC CAC CGT CAC AAA CAG CTG ATC AGA TTC CTG AAA
CGT CTA GAC CGT AAC CTG TGG GGC CTG GCC GGC CTG
AAC TCT TGT CCG GTT AAA GAA GCT AAC CAG TCG ACT
CTG GAA AAC TTC CTC GAG CGT CTG AAA ACC ATC ATG
CGT GAA AAG TAC TCT AAA TGC TCT TCT.

16. A method for producing an interleukin-4 protein characterised by possessing B-cell growth factor activity (BCGF) on human cells and T-cell growth factor activity (TCGF) on human cells, the method comprising maintaining under conditions suitable for expression of the nucleotide sequence into said polypeptide, a host having incorporated a vector comprising a nucleotide sequence coding for said polypeptide, wherein the nucleotide sequence is capable of being expressed by a host containing the vector and wherein the nucleotide sequence is at least 50 percent homologous to the insert of pcD-125; in particular a method wherein said nucleotide sequence is selected from those defined by claims 15 and wherein said host is preferably a mammalian cell.

17. A cell selected from yeast cells, bacterial cells, and mammalian cells, preferably from Escherichia coli cells, Saccharomyces cerevisiae cells, COS 7 monkey cells, chinese hamster ovary cells, mouse L cells, and mouse myeloma cells, said cell being transformed or transiently transfected by a vector comprising a nucleic acid sequence defined by claim 15.

18. A vector capable of transforming a bacterial host, the vector comprising a nucleic acid sequence defined by claim 15, in particular a vector selected from pIN-III-ompA2, TRPC11, and TAC-RBS,
or a vector capable of transforming a yeast host and comprising a nucleic acid sequence defined by claim 15, e.g. the vector pMF-alpha8,
or a vector capable of transforming or transiently transfecting a mammalian host, the vector comprising a nucleic acid sequence defined by claim 15, preferably consisting of a pcD plasmid, e.g., pcD-125 or pcD-46.

19. Use of human interleukin-4 as defined in any of Claims 1 to 12 for the preparation of a pharmaceutical composition for treating bare lymphocyte syndrome by stimulating expression of class II HLA-DR antigens on B cells.

## Claims (Claims for the following Contracting State(s): AT, GR, ES)

1. A method of producing an interleukin-4 protein which is characterized by possessing both B-cell growth factor activity (BCGF) on human cells and T-cell growth factor activity (TCGF) on human cells which process comprises culturing cells capable of expressing human interleukin-4 and recovering human interleukin-4.

2. A method according to claim 1 wherein human interleukin-4 exhibits in addition mast cell stimulatory activity and is recovered in substantially pure form.

3. A method according to claim 1 wherein said human interleukin-4 is a recombinant polypeptide.

4. A method according to Claim 1 wherein said produced human interleukin-4 has at least 9 x 10⁷ units/mg of TCGF (T-cell growth factor) activity and further exhibits at least one activity, preferably at least two, selected from MHC antigen induction activity, Fc-epsilon receptor induction activity, GM-CSF stimulated granulocyte colony growth potentiating activity, interleukin-2 TCGF potentiating activity, and IgG₁- and IgE-induction activity, said activities being assayed on human cell lines.

5. A method of producing a protein having human interleukin-4 activity and comprising a 3-fold substituted glycosylated or unglycosylated polypeptide having a sequence of amino acids defined by the formula: 
X(His) - X(Lys) - X(Cys) - X(Asp) - X(Ile) - X(Thr) -
X(Leu) - X(Gln) - X(Glu) - X(Ile) - X(Ile) - X(Lys) -
X(Thr) - X(Leu) - X(Asn) - X(Ser) - X(Leu) - X(Thr) -
X(Glu) - X(Gln) - X(Lys) - X(Thr) - X(Leu) - X(Cys) -
X(Thr) - X(Glu) - X(Leu) - X(Thr) - X(Val) - X(Thr) -
X(Asp) - X(Ile) - X(Phe) - X(Ala) - X(Ala) - X(Ser) -
X(Lys) - X(Asn) - X(Thr) - X(Thr) - X(Glu) - X(Lys) -
X(Glu) - X(Thr) - X(Phe) - X(Cys) - X(Arg) - X(Ala) -
X(Ala) - X(Thr) - X(Val) - X(Leu) - X(Arg) - X(Gln) -
X(Phe) - X(Tyr) - X(Ser) - X(His) - X(His) - X(Glu) -
X(Lys) - X(Asp) - X(Thr) - X(Arg) - X(Cys) - X(Leu) -
X(Gly) - X(Ala) - X(Thr) - X(Ala) - X(Gln) - X(Gln) -
X(Phe) - X(His) - X(Arg) - X(His) - X(Lys) - X(Gln) -
X(Leu) - X(Ile) - X(Arg) - X(Phe) - X(Leu) - X(Lys) -
X(Arg) - X(Leu) - X(Asp) - X(Arg) - X(Asn) - X(Leu) -
X(Trp) - X(Gly) - X(Leu) - X(Ala) - X(Gly) - X(Leu) -
X(Asn) - X(Ser) - X(Cys) - X(Pro) - X(Val) - X(Lys) -
X(Glu) - X(Ala) - X(Asn) - X(Gln) - X(Ser) - X(Thr) -
X(Leu) - X(Glu) - X(Asn) - X(Phe) - X(Leu) - X(Glu) -
X(Arg) - X(Leu) - X(Lys) - X(Thr) - X(Ile) - X(Met) -
X(Arg) - X(Glu) - X(Lys) - X(Tyr) - X(Ser) - X(Lys) -
X(Cys) - X(Ser) - X(Ser);
 wherein:
X(Ser) represents the group consisting of Ser, Thr, Gly, and Asn;
X(Arg) represents the group consisting of Arg, His, Gln, Lys, and Glu;
X(Leu) represents the group consisting of Leu, Ile, Phe, Tyr, Met, and Val;
X(Pro) represents the group consisting of Pro, Gly, Ala, and Thr;
X(Thr) represents the group consisting of Thr, Pro, Ser, Ala, Gly, His, and Gln;
X(Ala) represents the group consisting of Ala, Gly, Thr, and Pro;
X(Val) represents the group consisting of Val, Met, Tyr, Phe, Ile, and Leu;
X(Gly) represents the group consisting of Gly, Ala, Thr, Pro, and Ser;
X(Ile) represents the group consisting of Ile, Met, Tyr, Phe, Val, and Leu;
X(Phe) represents the group consisting of Phe, Trp, Met, Tyr, Ile, Val, and Leu;
X(Tyr) represents the group consisting of Tyr, Trp, Met, Phe, Ile, Val, and Leu;
X(His) represents the group consisting of His, Glu, Lys, Gln, Thr, and Arg;
X(Gln) represents the group consisting of Gln, Glu, Lys, Asn, His, Thr, and Arg;
X(Asn) represents the group consisting of Asn, Glu, Asp, Gln, and Ser;
X(Lys) represents the group consisting of Lys, Glu, Gln, His, and Arg;
X(Asp) represents the group consisting of Asp, Glu, and Asn;
X(Glu) represents the group consisting of Glu, Asp, Lys, Asn, Gln, His, and Arg;
X(Cys) represents the group consisting of Cys, Ser and Thr;
X(Trp) is Trp; and
X(Met) represents the group consisting of Met, Phe, Ile, Val, Leu, and Tyr.
which comprises culturing cells capable of expressing said protein and recovering said protein .

6. A method according to claim 5 wherein said glycosylated or unglycosylated polypeptide is 1-fold substituted.

7. A method according to claim 5 wherein said glycosylated or unglycosylated polypeptide is 1-fold substituted within the region defined by amino acid residues 3-29 inclusive, 35-66 inclusive, 78-87 inclusive, 98-99 inclusive, and 105-125 inclusive.

8. A method as claimed in any of claims 5 to 7 wherein:
X(Arg) is Arg;
X(Leu) represents the group consisting of Leu, Ile, and Met;
X(Pro) is Pro;
X(Ala) is Ala;
X(Val) is Val;
X(Ile) represents the group consisting of Ile, Met, and Leu;
X(Phe) is Phe;
X(Tyr) is Tyr;
X(His) is His;
X(Gln) is Gln;
X(Asn) is Asn;
X(Lys) is Lys;
X(Asp) is Asp;
X(Glu) is Glu;
X(Cys) represents the group consisting of Cys and Ser; and
X(Met) represents the group consisting of Met, Ile, and Leu;
in particular wherein said polypeptide is 1-fold substituted within the region defined by amino acid residues 3-29 inclusive, 35-66 inclusive, 78-87 inclusive, 98-99 inclusive, and 105-125 inclusive

9. A method according to any preceding claim wherein said human interleukin-4 has the formula

-
-
-
-
-
-
-
-
-
-
-
-
.

10. A method according to claim 9 wherein said human interleukin-4 has the formula given in claim 9 together with the following leader sequence

Met-Gly-Leu-Thr-Ser-Gln-Leu-Leu-Pro
Pro-Leu-Phe-Phe-Leu-Leu-Ala-Cys-Ala-Gly-
Asn-Phe-Val-His-Gly

11. A method according to any of claims 1 to 8 wherein said human interleukin-4 is selected from 1-fold inserted, 1-fold deleted, and 1-fold substituted polypeptides of the formula: 
X(His) - X(Lys) - X(Cys) - X(Asp) - X(Ile) - X(Thr) -
X(Leu) - X(Gln) - X(Glu) - X(Ile) - X(Ile) - X(Lys) -
X(Thr) - X(Leu) - X(Asn) - X(Ser) - X(Leu) - X(Thr) -
X(Glu) - X(Gln) - X(Lys) - X(Thr) - X(Leu) - X(Cys) -
X(Thr) - X(Glu) - X(Leu) - X(Thr) - X(Val) - X(Thr) -
 
X(Asp) - X(Ile) - X(Phe) - X(Ala) - X(Ala) - X(Ser) -
X(Lys) - X(Asn) - X(Thr) - X(Thr) - X(Glu) - X(Lys) -
X(Glu) - X(Thr) - X(Phe) - X(Cys) - X(Arg) - X(Ala) -
X(Ala) - X(Thr) - X(Val) - X(Leu) - X(Arg) - X(Gln) -
X(Phe) - X(Tyr) - X(Ser) - X(His) - X(His) - X(Glu) -
X(Lys) - X(Asp) - X(Thr) - X(Arg) - X(Cys) - X(Leu) -
X(Gly) - X(Ala) - X(Thr) - X(Ala) - X(Gln) - X(Gln) -
X(Phe) - X(His) - X(Arg) - X(His) - X(Lys) - X(Gln) -
X(Leu) - X(Ile) - X(Arg) - X(Phe) - X(Leu) - X(Lys) -
X(Arg) - X(Leu) - X(Asp) - X(Arg) - X(Asn) - X(Leu) -
X(Trp) - X(Gly) - X(Leu) - X(Ala) - X(Gly) - X(Leu) -
X(Asn) - X(Ser) - X(Cys) - X(Pro) - X(Val) - X(Lys) -
X(Glu) - X(Ala) - X(Asn) - X(Gln) - X(Ser) - X(Thr) -
X(Leu) - X(Glu) - X(Asn) - X(Phe) - X(Leu) - X(Glu) -
X(Arg) - X(Leu) - X(Lys) - X(Thr) - X(Ile) - X(Met) -
X(Arg) - X(Glu) - X(Lys) - X(Tyr) - X(Ser) - X(Lys) -
X(Cys) - X(Ser) - X(Ser)
 wherein:
X(Ser) represents the group consisting of Ser, Thr, Gly, and Asn;
X(Arg) represents the group consisting of Arg, His, Gln, Lys, and Glu;
X(Leu) represents the group consisting of Leu, Ile, Phe, Tyr, Met, and Val;
X(Pro) represents the group consisting of Pro, Gly, Ala, and Thr;
X(Thr) represents the group consisting of Thr, Pro, Ser, Ala, Gly, His, and Gln;
X(Ala) represents the group consisting of Ala, Gly, Thr, and Pro;
X(Val) represents the group consisting of Val, Met, Tyr, Phe, Ile, and Leu;
X(Gly) represents the group consisting of Gly, Ala, Thr, Pro, and Ser;
X(Ile) represents the group consisting of Ile, Met, Tyr, Phe, Val, and Leu;
X(Phe) represents the group consisting of Phe, Trp, Met, Tyr, Ile, Val, and Leu;
X(Tyr) represents the group consisting of Tyr, Trp, Met, Phe, Ile, Val, and Leu;
X(His) represents the group consisting of His, Glu, Lys, Gln, Thr, and Arg;
X(Gln) represents the group consisting of Gln, Glu, Lys, Asn, His, Thr, and Arg;
X(Asn) represents the group consisting of Asn, Glu, Asp, Gln, and Ser;
X(Lys) represents the group consisting of Lys, Glu, Gln, His, and Arg;
X(Asp) represents the group consisting of Asp, Glu, and Asn;
X(Glu) represents the group consisting of Glu, Asp, Lys, Asn, Gln, His, and Arg;
X(Cys) represents the group consisting of Cys, Ser and Thr;
X(Trp) is Trp; and
X(Met) represents the group consisting of Met, Phe, Ile, Val, Leu, and Tyr;
especially wherein
X(Ser) is Ser;
X(Arg) represents the group consisting of Arg, His, and Lys;
X(Leu) represents the group consisting of Leu, Ile, Phe, and Met;
X(Pro) represents the group consisting of Pro and Ala;
X(Thr) is Thr;
X(Ala) represents the group consisting of Ala and Pro;
X(Val) represents the group consisting of Val, Met, and Ile;
X(Gly) is Gly;
X(Ile) represents the group consisting of Ile, Met, Phe, Val, and Leu;
X(Phe) represents the group consisting of Phe, Met, Tyr, Ile, and Leu;
X(Tyr) represents the group consisting of Tyr and Phe;
X(His) represents the group consisting of His, Gln, and Arg;
X(Gln) represents the group consisting of Gln, Glu, and His;
X(Asn) represents the group consisting of Asn and Asp;
X(Lys) represents the group consisting of Lys and Arg;
X(Asp) represents the group consisting of Asp and Asn;
X(Glu) represents the group consisting of Glu and Gln; and
X(Met) represents the group consisting of Met, Phe, Ile, Val, and Leu;
in particular wherein said polypeptide is 1-fold inserted 1-fold deleted or 1-fold substituted within the region defined by amino acid residues 3-29 inclusive, 35-66 inclusive, 78-87 inclusive, 98-99 inclusive, and 105-125 inclusive

12. A method according to claim 11 wherein said produced polypeptide is human interleukin-4 mutein 1-4, human interleukin-4 mutein IS^{o}(Gly-Asn-Phe-Val-His-Gly) and human interleukin-4 mutein IS^{o}(Ala-Glu-Phe).

13. A method according to any preceding claim including the steps of:
constructing a vector comprising a nucleotide sequence coding for said polypeptide, wherein the nucleotide sequence is capable of being expressed by a host containing the vector and wherein the nucleotide sequence is at least 50 percent homologous to the insert of pcD-125;
incorporating the vector into the host; and
maintaining the vector-containing host under conditions suitable for expression of the nucleotide sequence into said polypeptide.

14. A method according to claim 13 wherein said nucleotide sequence is at least ninety percent homologous to the sequence of DNA in the cDNA insert of the vector selected from the group consisting of pcD-46 and pcD-125;
in particular wherein said sequence of codons is defined by the sequence of bases from position 64, preferably position 130, to position 522 in Figure 1B, especially the sequence of codons defined by the following formula:

CAC AAA TGT GAC ATC ACT CTG CAA GAA ATC ATC AAA
ACT CTG AAC TCG TTA ACC GAA CAG AAA ACC CTG TGC
ACC GAG CTC ACT GTT ACT GAT ATC TTC GCT GCT TCC
AAA AAC ACT ACT GAA AAA GAA ACT TTC TGC AGA GCT
GCT ACC GTT CTG CGT CAG TTC TAC TCT CAC CAC GAA
AAA GAC ACG CGT TGT CTC GGC GCC ACT GCG CAG CAG
TTC CAC CGT CAC AAA CAG CTG ATC AGA TTC CTG AAA
CGT CTA GAC CGT AAC CTG TGG GGC CTG GCC GGC CTG
AAC TCT TGT CCG GTT AAA GAA GCT AAC CAG TCG ACT
CTG GAA AAC TTC CTC GAG CGT CTG AAA ACC ATC ATG
CGT GAA AAG TAC TCT AAA TGC TCT TCT.

15. A method according to any preceding claim wherein said cultured cells are mammalian cells.

16. A method for the preparation of a pharmaceutical composition for stimulating the immune system, which comprises admixing a therapeutically compatible carrier and an effective amount of a human interleukin-4 or a polypeptide having human interleukin-4 activity as defined in any of claims 1 to 13.

17. A nucleic acid comprising a sequence of nucleotide bases capable of coding for a polypeptide as specified in any of Claims 1 to 12, preferably a sequence of nucleotide bases that is effectively homologous to a sequence of DNA in a cDNA insert of a vector selected from pcD-46 and pcD-125, in particular wherein said effectively homologous sequence is at least seventy-five percent homologous and preferably at least ninety percent homologous to said sequence of DNA in said cDNA insert of said vector selected from said group consisting of pcD-46 and pcD-125.

18. A nucleic acid comprising a sequence of codons capable of coding for the polypeptides defined in any of claims 1 to 12, in particular wherein said sequence of codons is defined by the sequence of bases from position 64, preferably position 130, to position 522 in Figure 1B, especially the sequence of codons defined by the following formula:

CAC AAA TGT GAC ATC ACT CTG CAA GAA ATC ATC AAA
ACT CTG AAC TCG TTA ACC GAA CAG AAA ACC CTG TGC
ACC GAG CTC ACT GTT ACT GAT ATC TTC GCT GCT TCC
AAA AAC ACT ACT GAA AAA GAA ACT TTC TGC AGA GCT
GCT ACC GTT CTG CGT CAG TTC TAC TCT CAC CAC GAA
AAA GAC ACG CGT TGT CTC GGC GCC ACT GCG CAG CAG
TTC CAC CGT CAC AAA CAG CTG ATC AGA TTC CTG AAA
CGT CTA GAC CGT AAC CTG TGG GGC CTG GCC GGC CTG
AAC TCT TGT CCG GTT AAA GAA GCT AAC CAG TCG ACT
CTG GAA AAC TTC CTC GAG CGT CTG AAA ACC ATC ATG
CGT GAA AAG TAC TCT AAA TGC TCT TCT.

19. A cell selected from yeast cells, bacterial cells, and mammalian cells, preferably from Escherichia coli cells, Saccharomyces cerevisiae cells, COS 7 monkey cells, chinese hamster ovary cells, mouse L cells, and mouse myeloma cells, said cell being transformed or transiently transfected by a vector comprising a nucleic acid sequence defined by claim 18.

20. A vector capable of transforming a bacterial host, the vector comprising a nucleic acid sequence defined by claim 17, in particular a vector selected from pIN-III-ompA2, TRPC11, and TAC-RBS,
or a vector capable of transforming a yeast host and comprising a nucleic acid sequence defined by claim 17, e.g. the vector pMF-alpha8,
or a vector capable of transforming or transiently transfecting a mammalian host, the vector comprising a nucleic acid sequence defined by claim 17, preferably consisting of a pcD plasmid, e.g., pcD-125 or pcD-46.

21. Use of an interleukin-4 as defined in any of claims 1 to 12 for the preparation of a pharmaceutical composition for treating bare lymphocyte syndrome by stimulating expression of class II HLA-DR antigens on B cells.

## Patentansprüche (Patentansprüche für folgende(n) Vertragsstaat(en): BE, CH, LI, DE, FR, GB, IT, LU, NL, SE)

1. Interleukin-4-Protein, dadurch gekennzeichnet, daß es die Wirkung eines B-Zellen-Wachstumsfaktors (BCGF) sowie eines T-Zellen-Wachstumsfaktors (TCGF) auf menschliche Zellen besitzt.

2. Human-Interleukin-4 gemäß Anspruch 1, das in einer im wesentlichen reinen Form vorliegt und zusätzlich mastzellenstimulierende Wirkung zeigt.

3. Human-Interleukin-4 gemäß Anspruch 1, das in Form eines rekombinanten Polypeptids vorliegt.

4. Human-Interleukin-4 gemäß Anspruch 1, das eine TCGF-Aktivität (T-Zellen-Wachstumsfaktoraktivität) von wenigstens 9 x 10⁷ Einheiten/mg aufweist und weiterhin wenigstens eine Wirkung zeigt, vorzugsweise wenigstens zwei, die ausgewählt ist aus MHC-Antigen-Induktionswirkung, Fc-ε-Rezeptor-Induktionswirkung, potenzierende Wirkung auf GM-CSF-stimuliertes Granulocytenkoloniewachstum, Interleukin-2-TCGF-potenzierende Wirkung sowie IgG₁- und IgE-Induktionswirkung, wobei die Wirkungen an Human-Zellinien bestimmt werden.

5. Protein mit der Wirkung von Human-Interleukin-4, das ein dreifach substituiertes glycosyliertes oder nicht glycosyliertes Polypeptid umfaßt, welches eine Aminosäuresequenz besitzt, die durch die Formel definiert ist: 
X(His) - X(Lys) - X(Cys) - X(Asp) - X(Ile) - X(Thr) -
X(Leu) - X(Gln) - X(Glu) - X(Ile) - X(Ile) - X(Lys) -
X(Thr) - X(Leu) - X(Asn) - X(Ser) - X(Leu) - X(Thr) -
X(Glu) - X(Gln) - X(Lys) - X(Thr) - X(Leu) - X(Cys) -
X(Thr) - X(Glu) - X(Leu) - X(Thr) - X(Val) - X(Thr) -
X(Asp) - X(Ile) - X(Phe) - X(Ala) - X(Ala) - X(Ser) -
X(Lys) - X(Asn) - X(Thr) - X(Thr) - X(Glu) - X(Lys) -
X(Glu) - X(Thr) - X(Phe) - X(Cys) - X(Arg) - X(Ala) -
X(Ala) - X(Thr) - X(Val) - X(Leu) - X(Arg) - X(Gln) -
X(Phe) - X(Tyr) - X(Ser) - X(His) - X(His) - X(Glu) -
X(Lys) - X(Asp) - X(Thr) - X(Arg) - X(Cys) - X(Leu) -
X(Gly) - X(Ala) - X(Thr) - X(Ala) - X(Gln) - X(Gln) -
X(Phe) - X(His) - X(Arg) - X(His) - X(Lys) - X(Gln) -
X(Leu) - X(Ile) - X(Arg) - X(Phe) - X(Leu) - X(Lys) -
X(Arg) - X(Leu) - X(Asp) - X(Arg) - X(Asn) - X(Leu) -

X(Trp) - X(Gly) - X(Leu) - X(Ala) - X(Gly) - X(Leu) -
X(Asn) - X(Ser) - X(Cys) - X(Pro) - X(Val) - X(Lys) -
X(Glu) - X(Ala) - X(Asn) - X(Gln) - X(Ser) - X(Thr) -
X(Leu) - X(Glu) - X(Asn) - X(Phe) - X(Leu) - X(Glu) -
X(Arg) - X(Leu) - X(Lys) - X(Thr) - X(Ile) - X(Met) -
X(Arg) - X(Glu) - X(Lys) - X(Tyr) - X(Ser) - X(Lys) -
X(Cys) - X(Ser) - X(Ser);
 wobei:
X(Ser) die Gruppe darstellt, die aus Ser, Thr, Gly und Asn besteht,
X(Arg) die Gruppe darstellt, die aus Arg, His, Gln, Lys und Glu besteht,
X(Leu) die Gruppe darstellt, die aus Leu, Ile, Phe, Tyr, Met und Val besteht,
X(Pro) die Gruppe darstellt, die aus Pro, Gly, Ala und Thr besteht,
X(Thr) die Gruppe darstellt, die aus Thr, Pro, Ser, Ala, Gly, His und Gln besteht,
X(Ala) die Gruppe darstellt, die aus Ala, Gly, Thr und Pro besteht,
X(Val) die Gruppe darstellt, die aus Val, Met, Tyr, Phe, Ile und Leu besteht,
X(Gly) die Gruppe darstellt, die aus Gly, Ala, Thr, Pro und Ser besteht,
X(Ile) die Gruppe darstellt, die aus Ile, Met, Tyr, Phe, Val und Leu besteht,
X(Phe) die Gruppe darstellt, die aus Phe, Trp, Met, Tyr, Ile, Val und Leu besteht,
X(Tyr) die Gruppe darstellt, die aus Tyr, Trp, Met, Phe, Ile, Val und Leu besteht,
X(His) die Gruppe darstellt, die aus His, Glu, Lys, Gln, Thr und Arg besteht,
X(Gln) die Gruppe darstellt, die aus Gln, Glu, Lys, Asn, His, Thr und Arg besteht,
X(Asn) die Gruppe darstellt, die aus Asn, Glu, Asp, Gln und Ser besteht,
X(Lys) die Gruppe darstellt, die aus Lys, Glu, Gln, His und Arg besteht,
X(Asp) die Gruppe darstellt, die aus Asp, Glu und Asn besteht,
X(Glu) die Gruppe darstellt, die aus Glu, Asp, Lys, Asn, Gln, His und Arg besteht,
X(Cys) die Gruppe darstellt, die aus Cys, Ser und Thr besteht,
X(Trp) Trp ist und
X(Met) die Gruppe darstellt, die aus Met, Phe, Ile, Val, Leu und Tyr besteht.

6. Glycosyliertes oder nicht glycosyliertes Polypeptid gemäß Anspruch 5, das einfach substituiert ist.

7. Glycosyliertes oder nicht glycosyliertes Polypeptid gemäß Anspruch 5, das innerhalb des Bereichs, der durch die Aminosäurereste 3-29 einschließlich, 35-66 einschließlich 78-87 einschließlich, 98-99 einschließlich und 105-125 einschließlich definiert ist, einfach substituiert ist.

8. Protein gemäß einem der Ansprüche 5 bis 7, wobei:
X(Arg) Arg ist,
X(Leu) die Gruppe darstellt, die aus Leu, Ile und Met besteht,
X(Pro) Pro ist,
X(Ala) Ala ist,
X(Val) Val ist,
X(Ile) die Gruppe darstellt, die aus Ile, Met und Leu besteht,
X(Phe) Phe ist,
X(Tyr) Tyr ist,
X(His) His ist,
X(Gln) Gln ist,
X(Asn) Asn ist,
X(Lys) Lys ist,
X(Asp) Asp ist,
X(Glu) Glu ist,
X(Cys) die Gruppe darstellt, die aus Cys und Ser besteht, und
X(Met) die Gruppe darstellt, die aus Met, Ile und Leu besteht,
wobei das Polypeptid insbesondere innerhalb des Bereichs, der durch die Aminosäurereste 3-29 einschließlich, 35-66 einschließlich, 78-87 einschließlich, 98-99 einschließlich und 105-125 einschließlich definiert ist, einfach substituiert ist.

9. Reifes Human-Interleukin-4 mit der Formel

-
-
-
-
-
-
-
-
-
-
-
-
.

10. Human-Interleukin-4 mit der in Anspruch 9 angegebenen Formel zusammen mit der folgenden Leitsequenz:

Met-Gly-Leu-Thr-Ser-Gln-Leu-Leu-Pro
Pro-Leu-Phe-Phe-Leu-Leu-Ala-Cys-Ala-Gly-
Asn-Phe-Val-His-Gly

11. Glycosyliertes oder nicht glycosyliertes Polypeptid mit der Wirkung von Human-Interleukin-4, das ausgewählt ist aus einfach insertierten, einfach deletierten und einfach substituierten Polypeptiden der Formel: 
X(His) - X(Lys) - X(Cys) - X(Asp) - X(Ile) - X(Thr) -
X(Leu) - X(Gln) - X(Glu) - X(Ile) - X(Ile) - X(Lys) -
X(Thr) - X(Leu) - X(Asn) - X(Ser) - X(Leu) - X(Thr) -
X(Glu) - X(Gln) - X(Lys) - X(Thr) - X(Leu) - X(Cys) -
X(Thr) - X(Glu) - X(Leu) - X(Thr) - X(Val) - X(Thr) -
X(Asp) - X(Ile) - X(Phe) - X(Ala) - X(Ala) - X(Ser) -
X(Lys) - X(Asn) - X(Thr) - X(Thr) - X(Glu) - X(Lys) -
X(Glu) - X(Thr) - X(Phe) - X(Cys) - X(Arg) - X(Ala) -
X(Ala) - X(Thr) - X(Val) - X(Leu) - X(Arg) - X(Gln) -
X(Phe) - X(Tyr) - X(Ser) - X(His) - X(His) - X(Glu) -
X(Lys) - X(Asp) - X(Thr) - X(Arg) - X(Cys) - X(Leu) -
X(Gly) - X(Ala) - X(Thr) - X(Ala) - X(Gln) - X(Gln) -
X(Phe) - X(His) - X(Arg) - X(His) - X(Lys) - X(Gln) -
X(Leu) - X(Ile) - X(Arg) - X(Phe) - X(Leu) - X(Lys) -
X(Arg) - X(Leu) - X(Asp) - X(Arg) - X(Asn) - X(Leu) -
X(Trp) - X(Gly) - X(Leu) - X(Ala) - X(Gly) - X(Leu) -
X(Asn) - X(Ser) - X(Cys) - X(Pro) - X(Val) - X(Lys) -
X(Glu) - X(Ala) - X(Asn) - X(Gln) - X(Ser) - X(Thr) -
X(Leu) - X(Glu) - X(Asn) - X(Phe) - X(Leu) - X(Glu) -
X(Arg) - X(Leu) - X(Lys) - X(Thr) - X(Ile) - X(Met) -
X(Arg) - X(Glu) - X(Lys) - X(Tyr) - X(Ser) - X(Lys) -
X(Cys) - X(Ser) - X(Ser)
 wobei:
X(Ser) die Gruppe darstellt, die aus Ser, Thr, Gly und Asn besteht,
X(Arg) die Gruppe darstellt, die aus Arg, His, Gln, Lys und Glu besteht,
X(Leu) die Gruppe darstellt, die aus Leu, Ile, Phe, Tyr, Met und Val besteht,
X(Pro) die Gruppe darstellt, die aus Pro, Gly, Ala und Thr besteht,
X(Thr) die Gruppe darstellt, die aus Thr, Pro, Ser, Ala, Gly, His und Gln besteht,
X(Ala) die Gruppe darstellt, die aus Ala, Gly, Thr und Pro besteht,
X(Val) die Gruppe darstellt, die aus Val, Met, Tyr, Phe, Ile und Leu besteht,
X(Gly) die Gruppe darstellt, die aus Gly, Ala, Thr, Pro und Ser besteht,
X(Ile) die Gruppe darstellt, die aus Ile, Met, Tyr, Phe, Val und Leu besteht,
X(Phe) die Gruppe darstellt, die aus Phe, Trp, Met, Tyr, Ile, Val und Leu besteht,
X(Tyr) die Gruppe darstellt, die aus Tyr, Trp, Met, Phe, Ile, Val und Leu besteht,
X(His) die Gruppe darstellt, die aus His, Glu, Lys, Gln, Thr und Arg besteht,
X(Gln) die Gruppe darstellt, die aus Gln, Glu, Lys, Asn, His, Thr und Arg besteht,
X(Asn) die Gruppe darstellt, die aus Asn, Glu, Asp, Gln und Ser besteht,
X(Lys) die Gruppe darstellt, die aus Lys, Glu, Gln, His und Arg besteht,
X(Asp) die Gruppe darstellt, die aus Asp, Glu und Asn besteht,
X(Glu) die Gruppe darstellt, die aus Glu, Asp, Lys, Asn, Gln, His und Arg besteht,
X(Cys) die Gruppe darstellt, die aus Cys, Ser und Thr besteht,
X(Trp) Trp ist und
X(Met) die Gruppe darstellt, die aus Met, Phe, Ile, Val, Leu und Tyr besteht,
wobei insbesondere
X(Ser) Ser ist,
X(Arg) die Gruppe darstellt, die aus Arg, His und Lys besteht,
X(Leu) die Gruppe darstellt, die aus Leu, Ile, Phe und Met besteht,
X(Pro) die Gruppe darstellt, die aus Pro und Ala besteht,
X(Thr) Thr ist,
X(Ala) die Gruppe darstellt, die aus Ala und Pro besteht,
X(Val) die Gruppe darstellt, die aus Val, Met und Ile besteht,
X(Gly) Gly ist,
X(Ile) die Gruppe darstellt, die aus Ile, Met, Phe, Val und Leu besteht,
X(His) die Gruppe darstellt, die aus His, Gln und Arg besteht,
X(Gln) die Gruppe darstellt, die aus Gln, Glu und His besteht,
X(Asn) die Gruppe darstellt, die aus Asn und Asp besteht,
X(Lys) die Gruppe darstellt, die aus Lys und Arg besteht,
X(Asp) die Gruppe darstellt, die aus Asp und Asn besteht,
X(Glu) die Gruppe darstellt, die aus Glu und Gln besteht, und
X(Met) die Gruppe darstellt, die aus Met, Phe, Ile, Val und Leu besteht,
wobei das Polypeptid insbesondere innerhalb des Bereichs, der durch die Aminosäurereste 3-29 einschließlich, 35-66 einschließlich, 78-87 einschließlich, 98-99 einschließlich und 105-125 einschließlich definiert ist, einfach insertiert, einfach deletiert oder einfach substituiert ist.

12. Human-Interleukin-4-Mutein Δ¹⁻⁴, Human-Interleukin-4-Mutein IS⁰(Gly-Asn-Phe-Val-His-Gly) und Human-Interleukin-4-Mutein IS⁰(Ala-Glu-Phe).

13. Pharmazeutische Zusammensetzung zur Stimulierung des Immunsystems, die einen therapeutisch verträglichen Träger sowie eine wirksame Menge Human-Interleukin-4 oder eines Polypeptids mit der Wirkung von Human-Interleukin-4 gemäß einem der Ansprüche 1 bis 12 umfaßt.

14. Nucleinsäure, die eine Nucleotidbasensequenz umfaßt, die in der Lage ist, für ein Polypeptid gemäß einem der Ansprüche 1 bis 12 zu codieren, vorzugsweise eine Nucleotidbasensequenz, die zu einer DNA-Sequenz in einem cDNA-Einschub eines Vektors, der aus pcD-46 und pcD-125 ausgewählt ist, effektiv homolog ist, wobei die effektiv homologe Sequenz zu der DNA-Sequenz in dem cDNA-Einschub des Vektors, der aus der Gruppe ausgewählt ist, die aus pcD-46 und pcD-125 besteht, insbesondere zu wenigstens fünfzig Prozent homolog und vorzugsweise zu wenigstens fünfundsiebzig Prozent homolog ist.

15. Verbindung, die aus der Gruppe ausgewählt ist, die aus Nucleinsäuren besteht, die eine Codonsequenz umfassen, die in der Lage ist, für die Polypeptide gemäß einem der Ansprüche 4 bis 6 zu codieren, wobei die Codonsequenz insbesondere durch die Basensequenz von Position 64, vorzugsweise Position 130, bis Position 522 in Figur 1B definiert ist, insbesondere die durch die folgende Formel definierte Codonsequenz:

CAC AAA TGT GAC ATC ACT CTG CAA GAA ATC ATC AAA
ACT CTG AAC TCG TTA ACC GAA CAG AAA ACC CTG TGC
ACC GAG CTC ACT GTT ACT GAT ATC TTC GCT GCT TCC
AAA AAC ACT ACT GAA AAA GAA ACT TTC TGC AGA GCT
GCT ACC GTT CTG CGT CAG TTC TAC TCT CAC CAC GAA
AAA GAC ACG CGT TGT CTC GGC GCC ACT GCG CAG CAG
TTC CAC CGT CAC AAA CAG CTG ATC AGA TTC CTG AAA
CGT CTA GAC CGT AAC CTG TGG GGC CTG GCC GGC CTG
AAC TCT TGT CCG GTT AAA GAA GCT AAC CAG TCG ACT
CTG GAA AAC TTC CTC GAG CGT CTG AAA ACC ATC ATG
CGT GAA AAG TAC TCT AAA TGC TCT TCT.

16. Verfahren zur Herstellung eines Interleukin-4-Proteins, das dadurch gekennzeichnet ist, daß es die Wirkung eines B-Zellen-Wachstumsfaktors (BCGF) und die eines T-Zellen-Wachstumsfaktors (TCGF) auf menschliche Zellen besitzt, wobei das Verfahren das Halten eines Wirtes, in den ein Vektor eingebaut ist, der eine Nucleotidsequenz umfaßt, die für das Polypeptid codiert, unter Bedingungen, die für die Expression der Nucleotidsequenz in Form des Polypeptids geeignet sind, wobei die Nucleotidsequenz von einem Wirt exprimiert werden kann, der den Vektor enthält, und wobei die Nucleotidsequenz zu dem Einschub von pcD-125 zu wenigstens fünfzig Prozent homolog ist, insbesondere ein Verfahren, bei dem die Nucleotidsequenz aus den in Anspruch 15 definierten ausgewählt ist, wobei es sich bei dem Wirt vorzugsweise um eine Säugerzelle handelt.

17. Zelle, die aus Hefezellen, Bakterienzellen und Säugerzellen, vorzugsweise aus *Escherichia-coli*-Zellen, *Saccharomyces-cerevisiae-*Zellen, COS-7-Affenzellen, Eierstockzellen von Chinesischen Hamstern, Maus-L-Zellen und Maus-Myelom-Zellen ausgewählt ist, wobei die Zelle durch einen Vektor, der eine in Anspruch 15 definierte Nucleinsäuresequenz umfaßt, transformiert oder vorübergehend transfiziert ist.

18. Vektor, der ein Wirtsbakterium zu transformieren vermag, wobei der Vektor eine in Anspruch 15 definierte Nucleinsäuresequenz umfaßt, insbesondere ein Vektor, der aus pIN-III-ompA2, TRPC11 und TAC-RBS ausgewählt ist,
oder ein Vektor, der eine Wirtshefe zu transformieren vermag und eine in Anspruch 15 definierte Nucleinsäuresequenz umfaßt, z.B. der Vektor pMF-α8,
oder ein Vektor, der eine Säugerwirtszelle zu transformieren oder vorübergehend zu transfizieren vermag, wobei der Vektor eine in Anspruch 15 definierte Nucleinsäuresequenz umfaßt und vorzugsweise aus einem pcD-Plasmid, z.B. pcD-125 oder pcD-46, besteht.

19. Verwendung von Human-Interleukin-4 gemäß einem der Ansprüche 1 bis 12 zur Herstellung einer pharmazeutischen Zusammensetzung zur Behandlung des Bare-Lymphocyte-Syndroms durch Stimulieren der Expression von HLA-DR-Antigenen der Klasse II auf B-Zellen.

## Patentansprüche (Patentansprüche für folgende(n) Vertragsstaat(en): AT, GR, ES)

1. Verfahren zur Herstellung eines Interleukin-4-Proteins, das dadurch gekennzeichnet ist, daß es sowohl die Wirkung eines B-Zellen-Wachstumsfaktors (BCGF) als auch die eines T-Zellen-Wachstumsfaktors (TCGF) auf menschliche Zellen besitzt, wobei das Verfahren das Kultivieren von Zellen, die Human-Interleukin-4 exprimieren können, sowie das Gewinnen von Human-Interleukin-4 umfaßt.

2. Verfahren gemäß Anspruch 1, wobei das Human-Interleukin-4 zusätzlich mastzellenstimulierende Wirkung zeigt und in einer im wesentlichen reinen Form gewonnen wird.

3. Verfahren gemäß Anspruch 1, wobei das Human-Interleukin-4 ein rekombinantes Polypeptid ist.

4. Verfahren gemäß Anspruch 1, wobei das erzeugte Human-Interleukin-4 eine TCGF-Aktivität (T-Zellen-Wachstumsfaktoraktivität) von wenigstens 9 x 10⁷ Einheiten/mg aufweist und weiterhin wenigstens eine Wirkung zeigt, vorzugsweise wenigstens zwei, die ausgewählt ist aus MHC-Antigen-Induktionswirkung, Fc-ε-Rezeptor-Induktionswirkung, potenzierende Wirkung auf GM-CSF-stimuliertes Granulocytenkoloniewachstum, Interleukin-2-TCGF-potenzierende Wirkung sowie IgG₁- und IgE-Induktionswirkung, wobei die Wirkungen an Human-Zellinien bestimmt werden.

5. Verfahren zur Herstellung eines Proteins mit der Wirkung von Human-Interleukin-4, das ein dreifach substituiertes glycosyliertes oder nicht glycosyliertes Polypeptid umfaßt, welches eine Aminosäuresequenz besitzt, die durch die Formel definiert ist: 
X(His) - X(Lys) - X(Cys) - X(Asp) - X(Ile) - X(Thr) -
X(Leu) - X(Gln) - X(Glu) - X(Ile) - X(Ile) - X(Lys) -
X(Thr) - X(Leu) - X(Asn) - X(Ser) - X(Leu) - X(Thr) -
X(Glu) - X(Gln) - X(Lys) - X(Thr) - X(Leu) - X(Cys) -
X(Thr) - X(Glu) - X(Leu) - X(Thr) - X(Val) - X(Thr) -
X(Asp) - X(Ile) - X(Phe) - X(Ala) - X(Ala) - X(Ser) -
X(Lys) - X(Asn) - X(Thr) - X(Thr) - X(Glu) - X(Lys) -
X(Glu) - X(Thr) - X(Phe) - X(Cys) - X(Arg) - X(Ala) -
X(Ala) - X(Thr) - X(Val) - X(Leu) - X(Arg) - X(Gln) -
X(Phe) - X(Tyr) - X(Ser) - X(His) - X(His) - X(Glu) -
X(Lys) - X(Asp) - X(Thr) - X(Arg) - X(Cys) - X(Leu) -
X(Gly) - X(Ala) - X(Thr) - X(Ala) - X(Gln) - X(Gln) -
X(Phe) - X(His) - X(Arg) - X(His) - X(Lys) - X(Gln) -
X(Leu) - X(Ile) - X(Arg) - X(Phe) - X(Leu) - X(Lys) -
X(Arg) - X(Leu) - X(Asp) - X(Arg) - X(Asn) - X(Leu) -
X(Trp) - X(Gly) - X(Leu) - X(Ala) - X(Gly) - X(Leu) -
X(Asn) - X(Ser) - X(Cys) - X(Pro) - X(Val) - X(Lys) -
X(Glu) - X(Ala) - X(Asn) - X(Gln) - X(Ser) - X(Thr) -
X(Leu) - X(Glu) - X(Asn) - X(Phe) - X(Leu) - X(Glu) -
X(Arg) - X(Leu) - X(Lys) - X(Thr) - X(Ile) - X(Met) -
X(Arg) - X(Glu) - X(Lys) - X(Tyr) - X(Ser) - X(Lys) -
X(Cys) - X(Ser) - X(Ser);
 wobei:
X(Ser) die Gruppe darstellt, die aus Ser, Thr, Gly und Asn besteht,
X(Arg) die Gruppe darstellt, die aus Arg, His, Gln, Lys und Glu besteht,
X(Leu) die Gruppe darstellt, die aus Leu, Ile, Phe, Tyr, Met und Val besteht,
X(Pro) die Gruppe darstellt, die aus Pro, Gly, Ala und Thr besteht,
X(Thr) die Gruppe darstellt, die aus Thr, Pro, Ser, Ala, Gly, His und Gln besteht,
X(Ala) die Gruppe darstellt, die aus Ala, Gly, Thr und Pro besteht,
X(Val) die Gruppe darstellt, die aus Val, Met, Tyr, Phe, Ile und Leu besteht,
X(Gly) die Gruppe darstellt, die aus Gly, Ala, Thr, Pro und Ser besteht,
X(Ile) die Gruppe darstellt, die aus Ile, Met, Tyr, Phe, Val und Leu besteht,
X(Phe) die Gruppe darstellt, die aus Phe, Trp, Met, Tyr, Ile, Val und Leu besteht,
X(Tyr) die Gruppe darstellt, die aus Tyr, Trp, Met, Phe, Ile, Val und Leu besteht,
X(His) die Gruppe darstellt, die aus His, Glu, Lys, Gln, Thr und Arg besteht,
X(Gln) die Gruppe darstellt, die aus Gln, Glu, Lys, Asn, His, Thr und Arg besteht,
X(Asn) die Gruppe darstellt, die aus Asn, Glu, Asp, Gln und Ser besteht,
X(Lys) die Gruppe darstellt, die aus Lys, Glu, Gln, His und Arg besteht,
X(Asp) die Gruppe darstellt, die aus Asp, Glu und Asn besteht,
X(Glu) die Gruppe darstellt, die aus Glu, Asp, Lys, Asn, Gln, His und Arg besteht,
X(Cys) die Gruppe darstellt, die aus Cys, Ser und Thr besteht,
X(Trp) Trp ist und
X(Met) die Gruppe darstellt, die aus Met, Phe, Ile, Val, Leu und Tyr besteht,
umfassend das Kultivieren von Zellen, die das Protein exprimieren können, sowie das Gewinnen des Proteins.

6. Verfahren gemäß Anspruch 5, wobei das glycosylierte oder nicht glycosylierte Polypeptid einfach substituiert ist.

7. Verfahren gemäß Anspruch 5, wobei das glycosylierte oder nicht glycosylierte Polypeptid innerhalb des Bereichs, der durch die Aminosäurereste 3-29 einschließlich, 35-66 einschließlich, 78-87 einschließlich, 98-99 einschließlich und 105-125 einschließlich definiert ist, einfach substituiert ist.

8. Verfahren gemäß einem der Ansprüche 5 bis 7, wobei:
X(Arg) Arg ist,
X(Leu) die Gruppe darstellt, die aus Leu, Ile und Met besteht,
X(Pro) Pro ist,
X(Ala) Ala ist,
X(Val) Val ist,
X(Ile) die Gruppe darstellt, die aus Ile, Met und Leu besteht,
X(Phe) Phe ist,
X(Tyr) Tyr ist,
X(His) His ist,
X(Gln) Gln ist,
X(Asn) Asn ist,
X(Lys) Lys ist,
X(Asp) Asp ist,
X(Glu) Glu ist,
X(Cys) die Gruppe darstellt, die aus Cys und Ser besteht, und
X(Met) die Gruppe darstellt, die aus Met, Ile und Leu besteht,
wobei das Polypeptid insbesondere innerhalb des Bereichs, der durch die Aminosäurereste 3-29 einschließlich, 35-66 einschließlich, 78-87 einschließlich, 98-99 einschließlich und 105-125 einschließlich definiert ist, einfach substituiert ist.

9. Verfahren gemäß einem der vorstehenden Ansprüche, wobei das Human-Interleukin-4 die Formel 
-
-
-
-
-
-
-
-
-
-
-
-

 hat.

10. Verfahren gemäß Anspruch 9, wobei das Human-Interleukin-4 die in Anspruch 9 angegebene Formel hat, zusammen mit der folgenden Leitsequenz:

Met-Gly-Leu-Thr-Ser-Gln-Leu-Leu-Pro
Pro-Leu-Phe-Phe-Leu-Leu-Ala-Cys-Ala-Gly-
Asn-Phe-Val-His-Gly

11. Verfahren gemäß einem der Ansprüche 1 bis 8, wobei das Human-Interleukin-4 ausgewählt ist aus einfach insertierten, einfach deletierten und einfach substituierten Polypeptiden der Formel: 
X(His) - X(Lys) - X(Cys) - X(Asp) - X(Ile) - X(Thr) -
X(Leu) - X(Gln) - X(Glu) - X(Ile) - X(Ile) - X(Lys) -
X(Thr) - X(Leu) - X(Asn) - X(Ser) - X(Leu) - X(Thr) -
X(Glu) - X(Gln) - X(Lys) - X(Thr) - X(Leu) - X(Cys) -
X(Thr) - X(Glu) - X(Leu) - X(Thr) - X(Val) - X(Thr) -
 
X(Asp) - X(Ile) - X(Phe) - X(Ala) - X(Ala) - X(Ser) -
X(Lys) - X(Asn) - X(Thr) - X(Thr) - X(Glu) - X(Lys) -
X(Glu) - X(Thr) - X(Phe) - X(Cys) - X(Arg) - X(Ala) -
X(Ala) - X(Thr) - X(Val) - X(Leu) - X(Arg) - X(Gln) -
X(Phe) - X(Tyr) - X(Ser) - X(His) - X(His) - X(Glu) -
X(Lys) - X(Asp) - X(Thr) - X(Arg) - X(Cys) - X(Leu) -
X(Gly) - X(Ala) - X(Thr) - X(Ala) - X(Gln) - X(Gln) -
X(Phe) - X(His) - X(Arg) - X(His) - X(Lys) - X(Gln) -
X(Leu) - X(Ile) - X(Arg) - X(Phe) - X(Leu) - X(Lys) -
X(Arg) - X(Leu) - X(Asp) - X(Arg) - X(Asn) - X(Leu) -
X(Trp) - X(Gly) - X(Leu) - X(Ala) - X(Gly) - X(Leu) -
X(Asn) - X(Ser) - X(Cys) - X(Pro) - X(Val) - X(Lys) -
X(Glu) - X(Ala) - X(Asn) - X(Gln) - X(Ser) - X(Thr) -
X(Leu) - X(Glu) - X(Asn) - X(Phe) - X(Leu) - X(Glu) -
X(Arg) - X(Leu) - X(Lys) - X(Thr) - X(Ile) - X(Met) -
X(Arg) - X(Glu) - X(Lys) - X(Tyr) - X(Ser) - X(Lys) -
X(Cys) - X(Ser) - X(Ser)
 wobei:
X(Ser) die Gruppe darstellt, die aus Ser, Thr, Gly und Asn besteht,
X(Arg) die Gruppe darstellt, die aus Arg, His, Gln, Lys und Glu besteht,
X(Leu) die Gruppe darstellt, die aus Leu, Ile, Phe, Tyr, Met und Val besteht,
X(Pro) die Gruppe darstellt, die aus Pro, Gly, Ala und Thr besteht,
X(Thr) die Gruppe darstellt, die aus Thr, Pro, Ser, Ala, Gly, His und Gln besteht,
X(Ala) die Gruppe darstellt, die aus Ala, Gly, Thr und Pro besteht,
X(Val) die Gruppe darstellt, die aus Val, Met, Tyr, Phe, Ile und Leu besteht,
X(Gly) die Gruppe darstellt, die aus Gly, Ala, Thr, Pro und Ser besteht,
X(Ile) die Gruppe darstellt, die aus Ile, Met, Tyr, Phe, Val und Leu besteht,
X(Phe) die Gruppe darstellt, die aus Phe, Trp, Met, Tyr, Ile, Val und Leu besteht,
X(Tyr) die Gruppe darstellt, die aus Tyr, Trp, Met, Phe, Ile, Val und Leu besteht,
X(His) die Gruppe darstellt, die aus His, Glu, Lys, Gln, Thr und Arg besteht,
X(Gln) die Gruppe darstellt, die aus Gln, Glu, Lys, Asn, His, Thr und Arg besteht,
X(Asn) die Gruppe darstellt, die aus Asn, Glu, Asp, Gln und Ser besteht,
X(Lys) die Gruppe darstellt, die aus Lys, Glu, Gln, His und Arg besteht,
X(Asp) die Gruppe darstellt, die aus Asp, Glu und Asn besteht,
X(Glu) die Gruppe darstellt, die aus Glu, Asp, Lys, Asn, Gln, His und Arg besteht,
X(Cys) die Gruppe darstellt, die aus Cys, Ser und Thr besteht,
X(Trp) Trp ist und
X(Met) die Gruppe darstellt, die aus Met, Phe, Ile, Val, Leu und Tyr besteht,
wobei insbesondere
X(Ser) Ser ist,
X(Arg) die Gruppe darstellt, die aus Arg, His und Lys besteht,
X(Leu) die Gruppe darstellt, die aus Leu, Ile, Phe und Met besteht,
X(Pro) die Gruppe darstellt, die aus Pro und Ala besteht, X(Thr) Thr ist,
X(Ala) die Gruppe darstellt, die aus Ala und Pro besteht,
X(Val) die Gruppe darstellt, die aus Val, Met und Ile besteht,
X(Gly) Gly ist,
X(Ile) die Gruppe darstellt, die aus Ile, Met, Phe, Val und Leu besteht,
X(His) die Gruppe darstellt, die aus His, Gln und Arg besteht,
X(Gln) die Gruppe darstellt, die aus Gln, Glu und His besteht,
X(Asn) die Gruppe darstellt, die aus Asn und Asp besteht,
X(Lys) die Gruppe darstellt, die aus Lys und Arg besteht,
X(Asp) die Gruppe darstellt, die aus Asp und Asn besteht,
X(Glu) die Gruppe darstellt, die aus Glu und Gln besteht, und
X(Met) die Gruppe darstellt, die aus Met, Phe, Ile, Val und Leu besteht,
wobei das Polypeptid insbesondere innerhalb des Bereichs, der durch die Aminosäurereste 3-29 einschließlich, 35-66 einschließlich, 78-87 einschließlich, 98-99 einschließlich und 105-125 einschließlich definiert ist, einfach insertiert, einfach deletiert oder einfach substituiert ist.

12. Verfahren gemäß Anspruch 11, wobei es sich bei dem erzeugten Polypeptid um Human-Interleukin-4-Mutein Δ¹⁻⁴, Human-Interleukin-4-Mutein IS⁰(Gly-Asn-Phe-Val-His-Gly) oder Human-Interleukin-4-Mutein IS⁰(Ala-Glu-Phe) handelt.

13. Verfahren gemäß einem der vorstehenden Ansprüche, das die Schritte umfaßt:
Aufbauen eines Vektors, der eine Nucleotidsequenz umfaßt, die für das Polypeptid codiert, wobei die Nucleotidsequenz von einem Wirt exprimiert werden kann, der den Vektor enthält, und wobei die Nucleotidsequenz zu dem Einschub von pcD-125 zu wenigstens fünfzig Prozent homolog ist;
Einschleusen des Vektors in den Wirt; und
Halten des vektorhaltigen Wirtes unter Bedingungen, die für die Expression der Nucleotidsequenz in Form des Polypeptids geeignet sind.

14. Verfahren gemäß Anspruch 13, wobei die Nucleotidsequenz zu der DNA-Sequenz in dem cDNA-Einschub des Vektors, der aus der Gruppe ausgewählt ist, die aus pcD-46 und pcD-125 besteht, zu wenigstens neunzig Prozent homolog ist, wobei die Codonsequenz insbesondere durch die Basensequenz von Position 64, vorzugsweise Position 130, bis Position 522 in Figur 1B definiert ist, insbesondere die durch die folgende Formel definierte Codonsequenz:

CAC AAA TGT GAC ATC ACT CTG CAA GAA ATC ATC AAA
ACT CTG AAC TCG TTA ACC GAA CAG AAA ACC CTG TGC
ACC GAG CTC ACT GTT ACT GAT ATC TTC GCT GCT TCC
AAA AAC ACT ACT GAA AAA GAA ACT TTC TGC AGA GCT
GCT ACC GTT CTG CGT CAG TTC TAC TCT CAC CAC GAA
AAA GAC ACG CGT TGT CTC GGC GCC ACT GCG CAG CAG
TTC CAC CGT CAC AAA CAG CTG ATC AGA TTC CTG AAA
CGT CTA GAC CGT AAC CTG TGG GGC CTG GCC GGC CTG
AAC TCT TGT CCG GTT AAA GAA GCT AAC CAG TCG ACT
CTG GAA AAC TTC CTC GAG CGT CTG AAA ACC ATC ATG
CGT GAA AAG TAC TCT AAA TGC TCT TCT.

15. Verfahren gemäß einem der vorstehenden Ansprüche, wobei die kultivierten Zellen Säugerzellen sind.

16. Verfahren zur Herstellung einer pharmazeutischen Zusammensetzung zur Stimulierung des Immunsystems, das das Mischen eines therapeutisch verträglichen Trägers und einer wirksamen Menge eines Human-Interleukin-4 oder eines Polypeptids mit der Wirkung von Human-Interleukin-4, wie es in einem der Ansprüche 1 bis 13 definiert ist, umfaßt.

17. Nucleinsäure, die eine Nucleotidbasensequenz umfaßt, die in der Lage ist, für ein Polypeptid zu codieren, wie es in einem der Ansprüche 1 bis 12 definiert ist, vorzugsweise eine Nucleotidbasensequenz, die zu einer DNA-Sequenz in einem cDNA-Einschub eines Vektors, der aus pcD-46 und pcD-125 ausgewählt ist, effektiv homolog ist, wobei die effektiv homologe Sequenz zu der DNA-Sequenz in dem cDNA-Einschub des Vektors, der aus der Gruppe ausgewählt ist, die aus pcD-46 und pcD-125 besteht, insbesondere zu wenigstens fünfundsiebzig Prozent homolog und vorzugsweise zu wenigstens neunzig Prozent homolog ist.

18. Nucleinsäure, die eine Codonsequenz umfaßt, die in der Lage ist, für die in einem der Ansprüche 1 bis 12 definierten Polypeptide zu codieren, wobei die Codonsequenz insbesondere durch die Basensequenz von Position 64, vorzugsweise Position 130, bis Position 522 in Figur 1B definiert ist, insbesondere die durch die folgende Formel definierte Codonsequenz:

CAC AAA TGT GAC ATC ACT CTG CAA GAA ATC ATC AAA
ACT CTG AAC TCG TTA ACC GAA CAG AAA ACC CTG TGC
ACC GAG CTC ACT GTT ACT GAT ATC TTC GCT GCT TCC
AAA AAC ACT ACT GAA AAA GAA ACT TTC TGC AGA GCT
GCT ACC GTT CTG CGT CAG TTC TAC TCT CAC CAC GAA
AAA GAC ACG CGT TGT CTC GGC GCC ACT GCG CAG CAG
TTC CAC CGT CAC AAA CAG CTG ATC AGA TTC CTG AAA
CGT CTA GAC CGT AAC CTG TGG GGC CTG GCC GGC CTG
AAC TCT TGT CCG GTT AAA GAA GCT AAC CAG TCG ACT
CTG GAA AAC TTC CTC GAG CGT CTG AAA ACC ATC ATG
CGT GAA AAG TAC TCT AAA TGC TCT TCT.

19. Zelle, die aus Hefezellen, Bakterienzellen und Säugerzellen, vorzugsweise aus *Escherichia-coli-*Zellen, *Saccharomyces-cerevisiae*-Zellen, COS-7-Affenzellen, Eierstockzellen von Chinesischen Hamstern, Maus-L-Zellen und Maus-Myelom-Zellen ausgewählt ist, wobei die Zelle durch einen Vektor, der eine in Anspruch 18 definierte Nucleinsäuresequenz umfaßt, transformiert oder vorübergehend transfiziert ist.

20. Vektor, der ein Wirtsbakterium zu transformieren vermag, wobei der Vektor eine in Anspruch 17 definierte Nucleinsäuresequenz umfaßt, insbesondere ein Vektor, der aus pIN-III-ompA2, TRPC11 und TAC-RBS ausgewählt ist,
oder ein Vektor, der eine Wirtshefe zu transformieren vermag und eine in Anspruch 17 definierte Nucleinsäuresequenz umfaßt, z.B. der Vektor pMF-α8,
oder ein Vektor, der eine Säugerwirtszelle zu transformieren oder vorübergehend zu transfizieren vermag, wobei der Vektor eine in Anspruch 17 definierte Nucleinsäuresequenz umfaßt und vorzugsweise aus einem pcD-Plasmid, z.B. pcD-125 oder pcD-46, besteht.

21. Verwendung eines Interleukin-4, wie es in einem der Ansprüche 1 bis 12 definiert ist, zur Herstellung einer pharmazeutischen Zusammensetzung zur Behandlung des Bare-Lymphocyte-Syndroms durch Stimulieren der Expression von HLA-DR-Antigenen der Klasse II auf B-Zellen.

## Revendications (Revendications pour l'(les) Etat(s) contractant(s) suivant(s): BE, CH, LI, DE, FR, GB, IT, LU, NL, SE)

1. Protéine d'interleukine-4 caractérisée en ce qu'elle possède une activité de facteur de croissance des cellules B (BCGF) sur les cellules humaines et une activité de facteur de croissance des cellules T (TCGF) sur les cellules humaines.

2. Interleukine-4 humaine selon la revendication 1 sous une forme sensiblement pure et présentant de plus une activité de stimulation des mastocytes.

3. Interleukine-4 humaine selon la revendication 1 sous la forme d'un polypeptide recombinant.

4. Interleukine-4 humaine selon la revendication 1 et ayant au moins 9 x 10⁷ unités/mg d'activité de TCGF (facteur de croissance des cellules T) et présentant de plus au moins une activité, de préférence au moins deux, sélectionnées parmi l'activité d'induction de l'antigène MHC, l'activité d'induction du récepteur Fc-epsilon, l'activité de potentialisation de la croissance de colonies de granulocytes stimulée par GM-CSF, l'activité de potentialisation de TCGF de l'interleukine-2 et l'activité d'induction de IgG₁ et IgE, lesdites activités étant déterminées sur des lignées de cellules humaines.

5. Protéine ayant une activité d'interleukine-4 humaine et comprenant un polypeptide trois fois substitué, glycosylé ou non glycosylé, ayant une séquence d'acides aminés définie par la formule : 
X(His) - X(Lys) - X(Cys) - X(Asp) - X(Ile) - X(Thr) -
X(Leu) - X(Gln) - X(Glu) - X(Ile) - X(Ile) - X(Lys) -
X(Thr) - X(Leu) - X(Asn) - X(Ser) - X(Leu) - X(Thr) -
X(Glu) - X(Gln) - X(Lys) - X(Thr) - X(Leu) - X(Cys) -
X(Thr) - X(Glu) - X(Leu) - X(Thr) - X(Val) - X(Thr) -
X(Asp) - X(Ile) - X(Phe) - X(Ala) - X(Ala) - X(Ser) -
X(Lys) - X(Asn) - X(Thr) - X(Thr) - X(Glu) - X(Lys) -
 
X(Glu) - X(Thr) - X(Phe) - X(Cys) - X(Arg) - X(Ala) -
X(Ala) - X(Thr) - X(Val) - X(Leu) - X(Arg) - X(Gln) -
X(Phe) - X(Tyr) - X(Ser) - X(His) - X(His) - X(Glu) -
X(Lys) - X(Asp) - X(Thr) - X(Arg) - X(Cys) - X(Leu) -
X(Gly) - X(Ala) - X(Thr) - X(Ala) - X(Gln) - X(Gln) -
X(Phe) - X(His) - X(Arg) - X(His) - X(Lys) - X(Gln) -
X(Leu) - X(Ile) - X(Arg) - X(Phe) - X(Leu) - X(Lys) -
X(Arg) - X(Leu) - X(Asp) - X(Arg) - X(Asn) - X(Leu) -
X(Trp) - X(Gly) - X(Leu) - X(Ala) - X(Gly) - X(Leu) -
X(Asn) - X(Ser) - X(Cys) - X(Pro) - X(Val) - X(Lys) -
X(Glu) - X(Ala) - X(Asn) - X(Gln) - X(Ser) - X(Thr) -
X(Leu) - X(Glu) - X(Asn) - X(Phe) - X(Leu) - X(Glu) -
X(Arg) - X(Leu) - X(Lys) - X(Thr) - X(Ile) - X(Met) -
X(Arg) - X(Glu) - X(Lys) - X(Tyr) - X(Ser) - X(Lys) -
X(Cys) - X(Ser) - X(Ser) ;
 où :
X(Ser) représente le groupe consistant en Ser, Thr, Gly et Asn ;
X(Arg) représente le groupe consistant en Arg, His, Gln, Lys et Glu ;
X(Leu) représente le groupe consistant en Leu, Ile, Phe, Tyr, Met et Val ;
X(Pro) représente le groupe consistant en Pro, Gly, Ala et Thr ;
X(Thr) représente le groupe consistant en Thr, Pro, Ser, Ala, Gly, His et Gln ;
X(Ala) représente le groupe consistant en Ala, Gly, Thr et Pro ;
X(Val) représente le groupe consistant en Val, Met, Tyr, Phe, Ile et Leu ;
X(Gly) représente le groupe consistant en Gly, Ala, Thr, Pro et Ser ;
X(Ile) représente le groupe consistant en Ile, Met, Tyr, Phe, Val et Leu ;
X(Phe) représente le groupe consistant en Phe, Trp, Met, Tyr, Ile, Val et Leu ;
X(Tyr) représente le groupe consistant en Tyr, Trp, Met, Phe, Ile, Val et Leu ;
X(His) représente le groupe consistant en His, Glu, Lys, Gln, Thr et Arg ;
X(Gln) représente le groupe consistant en Gln, Glu, Lys, Asn, His, Thr et Arg ;
X(Asn) représente le groupe consistant en Asn, Glu, Asp, Gln et Ser ;
X(Lys) représente le groupe consistant en Lys, Glu, Gln, His et Arg ;
X(Asp) représente le groupe consistant en Asp, Glu et Asn ;
X(Glu) représente le groupe consistant en Glu, Asp, Lys, Asn, Gln, His et Arg ;
X(Cys) représente le groupe consistant en Cys, Ser et Thr ;
X(Trp) est Trp ; et
X(Met) représente le groupe consistant en Met, Phe, Ile, Val, Leu et Tyr.

6. Polypeptide glycosylé ou non glycosylé selon la revendication 5 qui est substitué une fois.

7. Polypeptide glycosylé ou non glycosylé selon la revendication 5 qui est substitué une fois dans la région définie par les résidus d'acides aminés 3-29 inclus, 35-66 inclus, 78-87 inclus, 98-99 inclus et 105-125 inclus.

8. Protéine selon l'une quelconque des revendications 5 à 7 où :
X(Arg) est Arg ;
X(Leu) représente le groupe consistant en Leu, Ile et Met ;
X(Pro) est Pro ;
X(Ala) est Ala ;
X(Val) est Val ;
X(Ile) représente le groupe consistant en Ile, Met et Leu ;
X(Phe) est Phe ;
X(Tyr) est Tyr ;
X(His) est His ;
X(Gln) est Gln ;
X(Asn) est Asn ;
X(Lys) est Lys ;
X(Asp) est Asp ;
X(Glu) est Glu ;
X(Cys) représente le groupe consistant en Cys et Ser ; et
X(Met) représente le groupe consistant en Met, Ile et Leu ;
en particulier où ledit polypeptide est substitué 1 fois dans la région définie par les résidus d'acides aminés 3-29 inclus, 35-66 inclus, 78-87 inclus, 98-99 inclus et 105-125 inclus.

9. Interleukine-4 humaine mûre ayant la formule

-
-
-

-
-
-

-
-
-
-
.

10. Interleukine-4 humaine ayant la formule donnée à la revendication 9 avec la séquence leader suivante :

Met-Gly-Leu-Thr-Ser-Gln-Leu-Leu-Pro
Pro-Leu-Phe-Phe-Leu-Leu-Ala-Cys-Ala-Gly-
Asn-Phe-Val-His-Gly

11. Polypeptide glycosylé ou non glycosylé ayant une activité d'interleukine-4 humaine et sélectionné parmi des polypeptides insérés 1 fois, délaissés 1 fois et substitués 1 fois de la formule : 
X(His) - X(Lys) - X(Cys) - X(Asp) - X(Ile) - X(Thr) -
X(Leu) - X(Gln) - X(Glu) - X(Ile) - X(Ile) - X(Lys) -
X(Thr) - X(Leu) - X(Asn) - X(Ser) - X(Leu) - X(Thr) -
X(Glu) - X(Gln) - X(Lys) - X(Thr) - X(Leu) - X(Cys) -
X(Thr) - X(Glu) - X(Leu) - X(Thr) - X(Val) - X(Thr) -
X(Asp) - X(Ile) - X(Phe) - X(Ala) - X(Ala) - X(Ser) -
X(Lys) - X(Asn) - X(Thr) - X(Thr) - X(Glu) - X(Lys) -
X(Glu) - X(Thr) - X(Phe) - X(Cys) - X(Arg) - X(Ala) -
X(Ala) - X(Thr) - X(Val) - X(Leu) - X(Arg) - X(Gln) -
X(Phe) - X(Tyr) - X(Ser) - X(His) - X(His) - X(Glu) -
X(Lys) - X(Asp) - X(Thr) - X(Arg) - X(Cys) - X(Leu) -
X(Gly) - X(Ala) - X(Thr) - X(Ala) - X(Gln) - X(Gln) -
X(Phe) - X(His) - X(Arg) - X(His) - X(Lys) - X(Gln) -
X(Leu) - X(Ile) - X(Arg) - X(Phe) - X(Leu) - X(Lys) -
X(Arg) - X(Leu) - X(Asp) - X(Arg) - X(Asn) - X(Leu) -
X(Trp) - X(Gly) - X(Leu) - X(Ala) - X(Gly) - X(Leu) -
X(Asn) - X(Ser) - X(Cys) - X(Pro) - X(Val) - X(Lys) -
X(Glu) - X(Ala) - X(Asn) - X(Gln) - X(Ser) - X(Thr) -
X(Leu) - X(Glu) - X(Asn) - X(Phe) - X(Leu) - X(Glu) -
X(Arg) - X(Leu) - X(Lys) - X(Thr) - X(Ile) - X(Met) -
X(Arg) - X(Glu) - X(Lys) - X(Tyr) - X(Ser) - X(Lys) -
X(Cys) - X(Ser) - X(Ser) ;
 où :
X(Ser) représente le groupe consistant en Ser, Thr, Gly et Asn ;
X(Arg) représente le groupe consistant en Arg, His, Gln, Lys et Glu ;
X(Leu) représente le groupe consistant en Leu, Ile, Phe, Tyr, Met et Val ;
X(Pro) représente le groupe consistant en Pro, Gly, Ala et Thr ;
X(Thr) représente le groupe consistant en Thr, Pro, Ser, Ala, Gly, His et Gln ;
X(Ala) représente le groupe consistant en Ala, Gly, Thr et Pro ;
X(Val) représente le groupe consistant en Val, Met, Tyr, Phe, Ile et Leu ;
X(Gly) représente le groupe consistant en Gly, Ala, Thr, Pro et Ser ;
X(Ile) représente le groupe consistant en Ile, Met, Tyr, Phe, Val et Leu ;
X(Phe) représente le groupe consistant en Phe, Trp, Met, Tyr, Ile, Val et Leu ;
X(Tyr) représente le groupe consistant en Tyr, Trp, Met, Phe, Ile, Val et Leu ;
X(His) représente le groupe consistant en His, Glu, Lys, Gln, Thr et Arg ;
X(Gln) représente le groupe consistant en Gln, Glu, Lys, Asn, His, Thr et Arg ;
X(Asn) représente le groupe consistant en Asn, Glu, Asp, Gln et Ser ;
X(Lys) représente le groupe consistant en Lys, Glu, Gln, His et Arg ;
X(Asp) représente le groupe consistant en Asp, Glu et Asn ;
X(Glu) représente le groupe consistant en Glu, Asp, Lys, Asn, Gln, His et Arg ;
X(Cys) représente le groupe consistant en Cys, Ser et Thr ;
X(Trp) est Trp ; et
X(Met) représente le groupe consistant en Met, Phe, Ile, Val, Leu et Tyr ;
en particulier où
X(Ser) est Ser ;
X(Arg) représente le groupe consistant en Arg, His et Lys ;
X(Leu) représente le groupe consistant en Leu, Ile, Phe et Met ;
X(Pro) représente le groupe consistant en Pro et Ala ;
X(Thr) est Thr ;
X(Ala) représente le groupe consistant en Ala et Pro ;
X(Val) représente le groupe consistant en Val, Met et Ile ;
X(Gly) est Gly ;
X(Ile) représente le groupe consistant en Ile, Met, Phe, Val et Leu ;
X(His) représente le groupe consistant en His, Gln et Arg ;
X(Gln) représente le groupe consistant en Gln, Glu et His ;
X(Asn) représente le groupe consistant en Asn et Asp ;
X(Lys) représente le groupe consistant en Lys et Arg ;
X(Asp) représente le groupe consistant en Asp et Asn ;
X(Glu) représente le groupe consistant en Glu et Gln ; et
X(Met) représente le groupe consistant en Met, Phe, Ile, Val et Leu ;
en particulier où ledit polypeptide est inséré 1 fois, délaissé 1 fois ou substitué 1 fois dans la région définie par les résidus d'acides aminés 3-29 inclus, 35-66 inclus, 78-87 inclus, 98-99 inclus et 105-125 inclus.

12. Interleukine-4 mutéine Δ¹⁻⁴ humaine, interleukine-4 mutéine IS^{O} (Gly-Asn-Phe-Val-His-Gly) humaine et interleukine-4 mutéine IS^{O} (Ala-Glu-Phe) humaine.

13. Composition pharmaceutique pour stimuler le système immunitaire comprenant un support thérapeutiquement compatible et une quantité efficace d'interleukine-4 humaine ou d'un polypeptide ayant une activité d'interleukine-4 humaine selon l'une quelconque des revendications 1 à 12.

14. Acide nucléique comprenant une séquence de bases de nucléotides capables de coder pour un polypeptide tel que défini selon l'une quelconque des revendications 1 à 12, de préférence une séquence de bases de nucléotides qui est effectivement homologue à une séquence d'ADN dans un insert d'ADNc d'un vecteur sélectionné parmi pcD-46 et pcD-125, en particulier où ladite séquence effectivement homologue est homologue au moins à cinquante pour-cent et de préférence homologue au moins à soixante quinze pour-cent avec ladite séquence d'ADN dans ledit insert d'ADNc dudit vecteur sélectionné dans le groupe consistant en pcD-46 et pcD-125.

15. Composé sélectionné dans le groupe consistant en acides nucléiques comprenant une séquence de codons capables de coder pour les polypeptides selon l'une quelconque des revendications 4 à 6, en particulier où ladite séquence de codons est définie par la séquence des bases de la position 64, de préférence la position 130, à la position 522 sur la figure 1B, en particulier la séquence de codons définie par la formule suivante :

CAC AAA TGT GAC ATC ACT CTG CAA GAA ATC ATC AAA
ACT CTG AAC TCG TTA ACC GAA CAG AAA ACC CTG TGC
ACC GAG CTC ACT GTT ACT GAT ATC TTC GCT GCT TCC
AAA AAC ACT ACT GAA AAA GAA ACT TTC TGC AGA GCT
GCT ACC GTT CTG CGT CAG TTC TAC TCT CAC CAC GAA
AAA GAC ACG CGT TGT CTC GGC GCC ACT GCG CAG CAG
TTC CAC CGT CAC AAA CAG CTG ATC AGA TTC CTG AAA
CGT CTA GAC CGT AAC CTG TGG GGC CTG GCC GGC CTG
AAC TCT TGT CCG GTT AAA GAA GCT AAC CAG TCG ACT
CTG GAA AAC TTC CTC GAG CGT CTG AAA ACC ATC ATG
CGT GAA AAG TAC TCT AAA TGC TCT TCT.

16. Méthode de production d'une protéine d'interleukine-4 caractérisée en ce qu'elle possède une activité de facteur de croissance de cellules B (BCGF) sur les cellules humaines et une activité de facteur de croissance de cellules T (TCGF) sur les cellules humaines, la méthode consistant à maintenir, dans des conditions appropriées à l'expression de la séquence de nucléotides dans ledit polypeptide, un hôte où est incorporé un vecteur comprenant une séquence de nucléotides codant pour ledit polypeptide, où ladite séquence de nucléotides peut être exprimée par un hôte contenant le vecteur et où la séquence de nucléotides est homologue au moins à 50 pour-cent avec l'insert de pcD-125 en particulier une méthode où ladite séquence de nucléotides est sélectionnée parmi celles définies par la revendication 15 et où ledit hôte est de préférence une cellule mammalienne.

17. Cellule sélectionnée parmi des cellules de levure, des cellules bactériennes et des cellules mammaliennes, de préférence parmi les cellules d' Escherichia coli, les cellules de Saccharomyces cerevisiae, les cellules de singe COS 7, les cellules d'ovaire de hamster chinois, les cellules L de souris et les cellules de myélome de souris, ladite cellule étant transformée ou transfectée de manière transitoire par un vecteur comprenant une séquence d'acides nucléiques définie par la revendication 15.

18. Vecteur capable de transformer un hôte bactérien, le vecteur comprenant une séquence d'acides nucléiques définie par la revendication 15, en particulier un vecteur sélectionné parmi pIN-III-ompA2, TRPC11 et TAC-RBS,
ou bien un vecteur capable de transformer une levure hôte et comprenant une séquence d'acide nucléique définie par la revendication 15, e.g. le vecteur pMF-alpha8,
ou bien un vecteur capable de transformer ou de transfecter de manière transitoire un hôte mammalien, le vecteur comprenant une séquence d'acide nucléique définie par la revendication 15, consistant de préférence en un plasmide pcD, e.g. pcD-125 ou pcD-46.

19. Utilisation d'interleukine-4 humaine telle que définie selon l'une quelconque des revendications 1 à 12 pour la préparation d'une composition pharmaceutique pour le traitement du syndrome des lymphocytes nus par stimulation de l'expression des antigènes de HLA-DR classe II sur les cellules B.

## Revendications (Revendications pour l'(les) Etat(s) contractant(s) suivant(s): AT, GR, ES)

1. Méthode de production d'une protéine d'interleukine-4 qui est caractérisée en ce qu'elle possède à la fois une activité de facteur de croissance des cellules B (BCGF) sur les cellules humaines et une activité de facteur de croissance des cellules T (TCGF) sur les cellules humaines, lequel procédé comprend la mise en culture de cellules capables d'exprimer l'interleukine-4 humaine et la récupération de l'interleukine-4 humaine.

2. Méthode selon la revendication 1 où l'interleukine-4 humaine présente en plus une activité de stimulation des mastocytes et est récupérée sous une forme sensiblement pure.

3. Méthode selon la revendication 1 où ladite interleukine-4 humaine est un polypeptide recombinant.

4. Méthode selon la revendication 1 où ladite interleukine-4 humaine produite a au moins 9 x 10⁷ unités/mg d'activité de TCGF (facteur de croissance des cellules T) et présente de plus au moins une activité, de préférence au moins deux, sélectionnées parmi l'activité d'induction de l'antigène MHC, l'activité d'induction du récepteur Fc-epsilon, l'activité de potentialisation de la croissance des colonies de granulocytes stimulée par GM-CSF, l'activité de potentialisation de TCGF de l'interleukine-2 et l'activité d'induction de IgG₁ et IgE, lesdites activités étant déterminées sur des lignées de cellules humaines.

5. Méthode de production d'une protéine ayant une activité d'interleukine-4 humaine et comprenant un polypeptide substitué 3 fois, glycosylé ou non glycosylé, ayant une séquence d'acides aminés définie par la formule : 
X(His) - X(Lys) - X(Cys) - X(Asp) - X(Ile) - X(Thr) -
X(Leu) - X(Gln) - X(Glu) - X(Ile) - X(Ile) - X(Lys) -
X(Thr) - X(Leu) - X(Asn) - X(Ser) - X(Leu) - X(Thr) -
X(Glu) - X(Gln) - X(Lys) - X(Thr) - X(Leu) - X(Cys) -
X(Thr) - X(Glu) - X(Leu) - X(Thr) - X(Val) - X(Thr) -
X(Asp) - X(Ile) - X(Phe) - X(Ala) - X(Ala) - X(Ser) -
X(Lys) - X(Asn) - X(Thr) - X(Thr) - X(Glu) - X(Lys) -
X(Glu) - X(Thr) - X(Phe) - X(Cys) - X(Arg) - X(Ala) -
X(Ala) - X(Thr) - X(Val) - X(Leu) - X(Arg) - X(Gln) -
X(Phe) - X(Tyr) - X(Ser) - X(His) - X(His) - X(Glu) -
X(Lys) - X(Asp) - X(Thr) - X(Arg) - X(Cys) - X(Leu) -
X(Gly) - X(Ala) - X(Thr) - X(Ala) - X(Gln) - X(Gln) -
X(Phe) - X(His) - X(Arg) - X(His) - X(Lys) - X(Gln) -
X(Leu) - X(Ile) - X(Arg) - X(Phe) - X(Leu) - X(Lys) -
X(Arg) - X(Leu) - X(Asp) - X(Arg) - X(Asn) - X(Leu) -
X(Trp) - X(Gly) - X(Leu) - X(Ala) - X(Gly) - X(Leu) -
X(Asn) - X(Ser) - X(Cys) - X(Pro) - X(Val) - X(Lys) -
X(Glu) - X(Ala) - X(Asn) - X(Gln) - X(Ser) - X(Thr) -
X(Leu) - X(Glu) - X(Asn) - X(Phe) - X(Leu) - X(Glu) -
X(Arg) - X(Leu) - X(Lys) - X(Thr) - X(Ile) - X(Met) -
X(Arg) - X(Glu) - X(Lys) - X(Tyr) - X(Ser) - X(Lys) -
X(Cys) - X(Ser) - X(Ser) ;
 où :
X(Ser) représente le groupe consistant en Ser, Thr, Gly et Asn ;
X(Arg) représente le groupe consistant en Arg, His, Gln, Lys et Glu ;
X(Leu) représente le groupe consistant en Leu, Ile, Phe, Tyr, Met et Val ;
X(Pro) représente le groupe consistant en Pro, Gly, Ala et Thr ;
X(Thr) représente le groupe consistant en Thr, Pro, Ser, Ala, Gly, His et Gln ;
X(Ala) représente le groupe consistant en Ala, Gly, Thr et Pro ;
X(Val) représente le groupe consistant en Val, Met, Tyr, Phe, Ile et Leu ;
X(Gly) représente le groupe consistant en Gly, Ala, Thr, Pro et Ser ;
X(Ile) représente le groupe consistant en Ile, Met, Tyr, Phe, Val et Leu ;
X(Phe) représente le groupe consistant en Phe, Trp, Met, Tyr, Ile, Val et Leu ;
X(Tyr) représente le groupe consistant en Tyr, Trp, Met, Phe, Ile, Val et Leu ;
X(His) représente le groupe consistant en His, Glu, Lys, Gln, Thr et Arg ;
X(Gln) représente le groupe consistant en Gln, Glu, Lys, Asn, His, Thr et Arg ;
X(Asn) représente le groupe consistant en Asn, Glu, Asp, Gln et Ser ;
X(Lys) représente le groupe consistant en Lys, Glu, Gln, His et Arg ;
X(Asp) représente le groupe consistant en Asp, Glu et Asn ;
X(Glu) représente le groupe consistant en Glu, Asp, Lys, Asn, Gln, His et Arg ;
X(Cys) représente le groupe consistant en Cys, Ser et Thr ;
X(Trp) est Trp ; et
X(Met) représente le groupe consistant en Met, Phe, Ile, Val, Leu et Tyr.
qui comprend la mise en culture de cellules capables d'exprimer ladite protéine et la récupération de ladite protéine.

6. Méthode selon la revendication 5 où ledit polypeptide glycosylé ou non glycosylé est substitué une fois.

7. Méthode selon la revendication 5 où ledit polypeptide glycosylé ou non glycosylé est substitué une fois dans la région définie par les résidus d'acides aminés 3-29 inclus, 35-66 inclus, 78-87 inclus, 98-99 inclus et 105-125 inclus.

8. Méthode selon l'une quelconque des revendications 5 à 7 où :
X(Arg) est Arg ;
X(Leu) représente le groupe consistant en Leu, Ile et Met ;
X(Pro) est Pro ;
X(Ala) est Ala ;
X(Val) est Val ;
X(Ile) représente le groupe consistant en Ile, Met et Leu ;
X(Phe) est Phe ;
X(Tyr) est Tyr ;
X(His) est His ;
X(Gln) est Gln ;
X(Asn) est Asn ;
X(Lys) est Lys ;
X(Asp) est Asp ;
X(Glu) est Glu ;
X(Cys) représente le groupe consistant en Cys et Ser ; et
X(Met) représente le groupe consistant en Met, Ile et Leu ;
en particulier, où ledit polypeptide est substitué 1 fois dans la région définie par les résidus d'acides aminés 3-29 inclus, 35-66 inclus, 78-87 inclus, 98-99 inclus et 105-125 inclus.

9. Méthode selon toute revendication précédente où ladite interleukine-4 humaine a pour formule :

-
-

-

-
-
-

-
-
-
-
.

10. Méthode selon la revendication 9 où ladite interleukine-4 humaine a la formule donnée à la revendication 9 avec la séquence leader suivante :

Met-Gly-Leu-Thr-Ser-Gln-Leu-Leu-Pro
Pro-Leu-Phe-Phe-Leu-Leu-Ala-Cys-Ala-Gly-
Asn-Phe-Val-His-Gly

11. Méthode selon l'une quelconque des revendications 1 à 8 où ladite interleukine-4 humaine est sélectionnée parmi des polypeptides insérés 1 fois, délaissés 1 fois et substitués 1 fois de la formule : 
X(His) - X(Lys) - X(Cys) - X(Asp) - X(Ile) - X(Thr) -
X(Leu) - X(Gln) - X(Glu) - X(Ile) - X(Ile) - X(Lys) -
X(Thr) - X(Leu) - X(Asn) - X(Ser) - X(Leu) - X(Thr) -
X(Glu) - X(Gln) - X(Lys) - X(Thr) - X(Leu) - X(Cys) -
X(Thr) - X(Glu) - X(Leu) - X(Thr) - X(Val) - X(Thr) -
X(Asp) - X(Ile) - X(Phe) - X(Ala) - X(Ala) - X(Ser) -
X(Lys) - X(Asn) - X(Thr) - X(Thr) - X(Glu) - X(Lys) -
X(Glu) - X(Thr) - X(Phe) - X(Cys) - X(Arg) - X(Ala) -
X(Ala) - X(Thr) - X(Val) - X(Leu) - X(Arg) - X(Gln) -
X(Phe) - X(Tyr) - X(Ser) - X(His) - X(His) - X(Glu) -
X(Lys) - X(Asp) - X(Thr) - X(Arg) - X(Cys) - X(Leu) -
X(Gly) - X(Ala) - X(Thr) - X(Ala) - X(Gln) - X(Gln) -
X(Phe) - X(His) - X(Arg) - X(His) - X(Lys) - X(Gln) -
 
X(Leu) - X(Ile) - X(Arg) - X(Phe) - X(Leu) - X(Lys) -
X(Arg) - X(Leu) - X(Asp) - X(Arg) - X(Asn) - X(Leu) -
X(Trp) - X(Gly) - X(Leu) - X(Ala) - X(Gly) - X(Leu) -
X(Asn) - X(Ser) - X(Cys) - X(Pro) - X(Val) - X(Lys) -
X(Glu) - X(Ala) - X(Asn) - X(Gln) - X(Ser) - X(Thr) -
X(Leu) - X(Glu) - X(Asn) - X(Phe) - X(Leu) - X(Glu) -
X(Arg) - X(Leu) - X(Lys) - X(Thr) - X(Ile) - X(Met) -
X(Arg) - X(Glu) - X(Lys) - X(Tyr) - X(Ser) - X(Lys) -
X(Cys) - X(Ser) - X(Ser) ;
 où :
X(Ser) représente le groupe consistant en Ser, Thr, Gly et Asn ;
X(Arg) représente le groupe consistant en Arg, His, Gln, Lys et Glu ;
X(Leu) représente le groupe consistant en Leu, Ile, Phe, Tyr, Met et Val ;
X(Pro) représente le groupe consistant en Pro, Gly, Ala et Thr ;
X(Thr) représente le groupe consistant en Thr, Pro, Ser, Ala, Gly, His et Gln ;
X(Ala) représente le groupe consistant en Ala, Gly, Thr et Pro ;
X(Val) représente le groupe consistant en Val, Met, Tyr, Phe, Ile et Leu ;
X(Gly) représente le groupe consistant en Gly, Ala, Thr, Pro et Ser ;
X(Ile) représente le groupe consistant en Ile, Met, Tyr, Phe, Val et Leu ;
X(Phe) représente le groupe consistant en Phe, Trp, Met, Tyr, Ile, Val et Leu ;
X(Tyr) représente le groupe consistant en Tyr, Trp, Met, Phe, Ile, Val et Leu ;
X(His) représente le groupe consistant en His, Glu, Lys, Gln, Thr et Arg ;
X(Gln) représente le groupe consistant en Gln, Glu, Lys, Asn, His, Thr et Arg ;
X(Asn) représente le groupe consistant en Asn, Glu, Asp, Gln et Ser ;
X(Lys) représente le groupe consistant en Lys, Glu, Gln, His et Arg ;
X(Asp) représente le groupe consistant en Asp, Glu et Asn ;
X(Glu) représente le groupe consistant en Glu, Asp, Lys, Asn, Gln, His et Arg ;
X(Cys) représente le groupe consistant en Cys, Ser et Thr ;
X(Trp) est Trp ; et
X(Met) représente le groupe consistant en Met, Phe, Ile, Val, Leu et Tyr ;
en particulier où
X(Ser) est Ser ;
X(Arg) représente le groupe consistant en Arg, His et Lys ;
X(Leu) représente le groupe consistant en Leu, Ile, Phe et Met ;
X(Pro) représente le groupe consistant en Pro et Ala ;
X(Thr) est Thr ;
X(Ala) représente le groupe consistant en Ala et Pro ;
X(Val) représente le groupe consistant en Val, Met et Ile ;
X(Gly) est Gly ;
X(Ile) représente le groupe consistant en Ile, Met, Phe, Val et Leu ;
X(Phe) représente le groupe consistant en Phe, Met, Tyr, Ile et Leu ;
X(Tyr) représente le groupe consistant en Tyr et Phe ;
X(His) représente le groupe consistant en His, Gln et Arg ;
X(Gln) représente le groupe consistant en Gln, Glu et His ;
X(Asn) représente le groupe consistant en Asn et Asp ;
X(Lys) représente le groupe consistant en Lys et Arg ;
X(Asp) représente le groupe consistant en Asp et Asn ;
X(Glu) représente le groupe consistant en Glu et Gln ; et
X(Met) représente le groupe consistant en Met, Phe, Ile, Val et Leu ;
en particulier où ledit polypeptide est inséré 1 fois, délaissé 1 fois ou substitué 1 fois dans la région définie par les résidus d'acides aminés 3-29 inclus, 35-66 inclus, 78-87 inclus, 98-99 inclus et 105-125 inclus.

12. Méthode selon la revendication 11 où ledit polypeptide produit est l'interleukine-4 mutéine 1-4 humaine, l'interleukine-4 mutéine IS^{o} (Gly-Asn-Phe-Val-His-Gly) humaine et l'interleukine-4 mutéine IS^{o} (Ala-Glu-Phe) humaine.

13. Méthode selon toute revendication précédente comprenant les étapes de :
construire un vecteur comprenant une séquence de nucléotides codant pour ledit polypeptide, où la séquence de nucléotides peut être exprimée par un hôte contenant le vecteur et où la séquence de nucléotides est homologue à au moins 50% avec l'insert de pcD-125 ;
incorporer le vecteur dans l'hôte ; et
maintenir l'hôte contenant le vecteur dans des conditions appropriées pour l'expression de la séquence de nucléotides dans ledit polypeptide.

14. Méthode selon la revendication 13 où ladite séquence de nucléotides est homologue à au moins 90% avec la séquence d'ADN dans l'insert d'ADNc du vecteur sélectionné dans le groupe consistant en pcD-46 et pcD-125 ;
en particulier où ladite séquence de codons est définie par la séquence des bases de la position 64, de préférence de la position 130, à la position 522 sur la figure 1B, en particulier la séquence de codons définie par la formule suivante :

CAC AAA TGT GAC ATC ACT CTG CAA GAA ATC ATC AAA
ACT CTG AAC TCG TTA ACC GAA CAG AAA ACC CTG TGC
ACC GAG CTC ACT GTT ACT GAT ATC TTC GCT GCT TCC
AAA AAC ACT ACT GAA AAA GAA ACT TTC TGC AGA GCT
GCT ACC GTT CTG CGT CAG TTC TAC TCT CAC CAC GAA
AAA GAC ACG CGT TGT CTC GGC GCC ACT GCG CAG CAG
TTC CAC CGT CAC AAA CAG CTG ATC AGA TTC CTG AAA
CGT CTA GAC CGT AAC CTG TGG GGC CTG GCC GGC CTG
AAC TCT TGT CCG GTT AAA GAA GCT AAC CAG TCG ACT
CTG GAA AAC TTC CTC GAG CGT CTG AAA ACC ATC ATG
CGT GAA AAG TAC TCT AAA TGC TCT TCT.

15. Méthode selon toute revendication précédente où lesdites cellules cultivées sont des cellules mammaliennes.

16. Méthode pour la préparation d'une composition pharmaceutique pour stimuler le système immunitaire, qui consiste à mélanger un support thérapeutiquement compatible et une quantité efficace d'une interleukine-4 humaine ou d'un polypeptide ayant une activité d'interleukine-4 humaine selon l'une quelconque des revendications 1 à 13.

17. Acide nucléique comprenant une séquence de bases de nucléotides capable de coder pour un polypeptide tel que spécifié selon l'une quelconque des revendications 1 à 12, de préférence une séquence de bases de nucléotides qui est effectivement homologue à une séquence d'ADN dans un insert d'ADNc d'un vecteur sélectionné parmi pcD-46 et pcD-125, en particulier où ladite séquence effectivement homologue est homologue au moins à 75% et de préférence homologue au moins à 90% avec ladite séquence d'ADN dans ledit insert d'ADNc dudit vecteur sélectionné dans le groupe consistant en pcD-46 et pcD-125.

18. Acide nucléique comprenant une séquence de codons capable de coder pour les polypeptides définie selon l'une quelconque des revendications 1 à 12, en particulier où ladite séquence de codons est définie par la séquence des bases de la position 64, de préférence de la position 130, à la position 522 sur la figure 1B, en particulier la séquence de codons définie par la formule suivante :

CAC AAA TGT GAC ATC ACT CTG CAA GAA ATC ATC AAA
ACT CTG AAC TCG TTA ACC GAA CAG AAA ACC CTG TGC
ACC GAG CTC ACT GTT ACT GAT ATC TTC GCT GCT TCC
AAA AAC ACT ACT GAA AAA GAA ACT TTC TGC AGA GCT
GCT ACC GTT CTG CGT CAG TTC TAC TCT CAC CAC GAA
AAA GAC ACG CGT TGT CTC GGC GCC ACT GCG CAG CAG
TTC CAC CGT CAC AAA CAG CTG ATC AGA TTC CTG AAA
CGT CTA GAC CGT AAC CTG TGG GGC CTG GCC GGC CTG
AAC TCT TGT CCG GTT AAA GAA GCT AAC CAG TCG ACT
CTG GAA AAC TTC CTC GAG CGT CTG AAA ACC ATC ATG
CGT GAA AAG TAC TCT AAA TGC TCT TCT.

19. Cellule sélectionnée parmi des cellules de levure, des cellules bactériennes et des cellules mammaliennes, de préférence parmi des cellules d'Escherichia coli, des cellules de Saccharomyces cerevisiae, des cellules de singe COS 7, des cellules d'ovaire de hamster chinois, des cellules L de souris et des cellules de myélome de souris, ladite cellule étant transformée ou transfectée de manière transitoire par un vecteur comprenant une séquence d'acide nucléique définie par la revendication 18.

20. Vecteur capable de transformer un hôte bactérien, le vecteur comprenant une séquence d'acide nucléique définie par la revendication 17, en particulier un vecteur sélectionné parmi pIN-III-ompA2, TRPC11 et TAC-RBS,
ou bien un vecteur capable de transformer un hôte de levure et comprenant une séquence d'acide nucléique définie par la revendication 17, e.g. le vecteur pMF-alpha8,
ou bien un vecteur capable de transformer ou de transfecter de manière transitoire un hôte mammalien, le vecteur comprenant une séquence d'acide nucléique définie par la revendication 17, consistant de préférence en un plasmide pcD, e.g. pcD-125 ou pcD-46.

21. Utilisation d'une interleukine-4 selon l'une quelconque des revendications 1 à 12 pour la préparation d'une composition pharmaceutique pour le traitement du syndrome des lymphocytes nus par stimulation de l'expression des antigènes HLA-DR classe II sur les cellules B.
